(19) Europäisches Patentamt
European Patent Office
Office européen des brevets

(11) Publication number: **0 665 223 A1**

(12) # EUROPEAN PATENT APPLICATION

(21) Application number: **95101077.6**

(22) Date of filing: **26.01.95**

(51) Int. Cl.$^{6}$: **C07D 237/16**, C07D 237/18, A61K 31/50

(30) Priority: **28.01.94 JP 8855/94**

(43) Date of publication of application:
**02.08.95 Bulletin 95/31**

(84) Designated Contracting States:
**AT BE CH DE DK ES FR GB GR IE IT LI LU NL PT SE**

(71) Applicant: **TAKEDA CHEMICAL INDUSTRIES, LTD.**
**1-1, Doshomachi 4-chome**
**Chuo-ku,**
**Osaka 541 (JP)**

(72) Inventor: **Marui, Shogo**
**8-1-405, Owakidai,**
**Kita-ku**
**Kobe,**
**Hyogo 651-11 (JP)**
Inventor: **Aso, Kazuyoshi**
**7-1-523, Kosei 2-chome,**
**Minato-ku**
**Osaka 552 (JP)**
Inventor: **Yamaoka, Masuo**
**5-10-202, Shoyamacho 2-chome,**
**Nagata-ku**
**Kobe,**
**Hyogo 653 (JP)**
Inventor: **Ikeyama, Shuichi**
**32-15, Nanpeidai 5-chome,**
**Takatsuki**
**Osaka 569 (JP)**

(74) Representative: **KUHNEN, WACKER & PARTNER**
**Alois-Steinecker-Strasse 22**
**D-85354 Freising (DE)**

(54) **Antitumor agent, novel 3(2H)-pyridazinone derivatives and their preparation.**

(57) Anti-tomor agents comprising novel 3(2H)-pyridazinone derivatives or salts thereof and methods of producing the same are disclosed. Also disclosed is a method for treating or preventing tumors by administering to a mammal a therapeutically effective amount of 4,5-di substituted 3(2H)-pyridazinones and salts thereof.

EP 0 665 223 A1

BACKGROUND OF INVENTION

FIELD OF INVENTION

This invention relates to an antitumor agent comprising a 3(2H)-pyridazinone derivative or a salt thereof, and to the novel 3(2H)-pyridazinone derivative or the salt thereof. More specifically, this invention relates to a prophylactic or therapeutic agent of intractable solid tumors principally exemplified by pancreatic carcinoma, prostatic cancer, lung cancer and breast cancer, and of remote metastatis of them to other organs.

RELATED PRIOR ART

Pancreatic carcinoma, which has been known as one of the intractable solid tumors, is, in many cases, impossible to subject to surgical operation, because of metastatis or peritonisis carcinomatosa found at the time when the carcinomatous peritonitis is detected, and even when the surgical operation is possible, the prognosis is generally not good. Such patients suffering from an advanced cancer or being in the post-operative stage are generally treated with 5-fluorouracil (5-FU) or by combination chemotherapy including 5-FU and mitomycin. The therapeutic effects of such treatment are not always successful. Similarly, against prostatic cancer, which is also known as one of the intractable solid tumors, especially hormone-independent tumors or recurrent tumors or metastatic tumors, chemotherapeutic agents including adriamycin, cisplatin, and 5-FU are employed singly or in combination, but the therapeutic effects are not necessarily successful.

Pyridazinone derivatives have been reported as having insecticidal or miticidal action [e.g.,JPA S58-(1983)-160989, JPA S59(1984)-39874, JPA S60(1985)-4173, JPA S60(1985)-54370, JPA S61(1986)-17570, JPA S63(1988)-215674 and J. Pestic. Sci. Vol. 17, S231 (1992)], analgesic and antiphlogistic action [e.g.,J. Pharm. Soc. Jpn. Vol.107, p.910 (1987) and Pharm. Acta Helv. Vol. 38, p.510 (1963)], antibacterial or an antifungal action [e.g.,J. Pharm. Soc. Jpn., Vol. 113, p.676 (1993) and ibid. Vol. 89, p.1516 (1969)], and herbicidal or plant-growth regulating action [JPB S38(1963)-7998 and JPB S40(1965)-3798]. And, in JPA S52(1977)-91883, there is a report that a sulfonyl pyridazinone derivative represented by the formula:

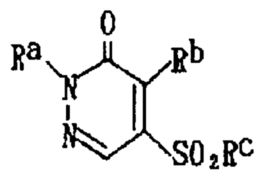

[wherein $R^a$ is an alkyl group or phenyl group, $R^c$ is an alkyl group, phenyl group or benzyl group, and $R^b$ is an amino group, a substituted amino group, an alicyclic amino group, a heterocyclic amino group, hydroxyl group or alkoxy group] having a side chain through $-SO_2-$ at the 5-position of the 3(2H)-pyridazinone ring inhibits the proliferation of leuchemic cells (L-1210) in mice, and in JPA H5(1993)-17357, there is a report that an aminopyridazinone derivative represented by the formula:

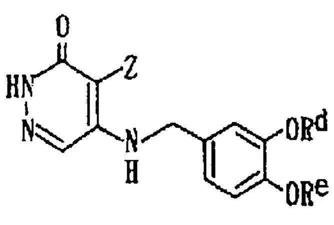

[wherein $R^d$ and $R^e$ independently is a H or a $C_{1-4}$ alkyl group, Z is Cl or Br] having a side chain through -NH- at the 5-position of the 3(2H)-pyridazinone ring is useful for the therapy of chronic myelogenic leukemia. However, no reports have been made yet that a compound having a side chain through -O- or -S- at the 5-position of 3(2H)-pyridazinone ring has an antitumor action, especially against solid tumors, above all intractable solid tumors including pancreatic carcinoma, prostatic cancers, lung cancers and breast

cancers. Circumstances being such as above, there is no question that prophylactic and therapeutic agents having an excellent antitumor action and which are effective against solid tumors, especially intractable solid tumors including pancreatic carcinoma, prostatic cancers, lung cancers and breast cancers are highly desired.

## SUMMARY OF THE INVENTION

The present inventors have made an extensive study of antitumor agents, and, as a result, they found that the 4,5-disubstituted 3(2H)-pyridazinone, the substituent at the 5-position being -X-$Y^0$-$Q^0$ wherein X is O or S, $Y^0$ is an optionally substituted divalent aliphatic hydrocarbon residue and $Q^0$ is an optionally substituted cyclic group, for example, a compound represented by the formula

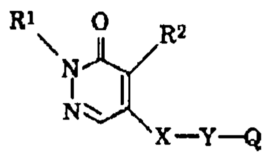

[I]

wherein $R^1$ is a lower alkyl group or an optionally substituted phenyl group, $R^2$ is H, halogen, an optionally substituted lower alkoxy group, an optionally substituted lower alkylthio group or an optionally substituted aliphatic hydrocarbon group, X is O or S, Y is an optionally substituted alkylene group or an optionally substituted alkenylene group, and Q is an optionally substituted aryl group or an optionally substituted aromatic heterocyclic group or a salt thereof, has an unexpectedly excellent antitumor action, an especially excellent ability to suppressing the proliferation of solid tumor cells including pancreatic carcinoma cells and prostatic cancer cells, and is useful for prophylaxis/therapy of intractable solid tumors including pancreatic carcinoma, prostatic cancer, lung cancers and breast cancers. Based on this finding, the present invention was accomplished.

## DETAILED DESCRIPTION OF THE INVENTION

More specifically, the present invention relates to

(1) an antitumor composition which comprises a 4,5-disubsutituted 3(2H)-pyridazinone, the substituent at the 5-position being -X-$Y^0$-$Q^0$ wherein X is O or S, $Y^0$ is an optionally substituted divalent aliphatic hydrocarbon residue and $Q^0$ is an optionally substituted cyclic group, or its salt;

(2) a composition described in (1), wherein the substituent at the 4-position of the 3(2H)-pyridazinone is a group having 1 to 150 atoms;

(3) a composition described in (1), wherein the 3(2H)-pyridazinone may be substituted with an optionally substituted hydrocarbon residue at the 2-position;

(4) a composition described in (1), wherein the divalent aliphatic hydrocarbon residue has 1 to 3 carbon atoms;

(5) a composition described in (4), wherein the substituent of the divalent aliphatic hydrocarbon residue is a hydroxyl group;

(6) a composition described in (1), wherein the cyclic group is an optionally substituted aromatic cyclic group;

(7) a composition described in (1), wherein the cyclic group is an optionally substituted phenyl group;

(8) a composition described in (7), wherein the phenyl group represented by the formula

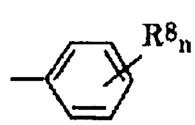

wherein $R^8$ is a halogen, a lower alkyl, a lower cycloalkyl, a lower alkoxy, a halo-lower alkyl, a halo-lower alkoxy, a cyano, a trimethylsilyl, a nitro,

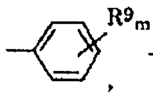, 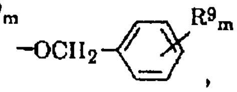, 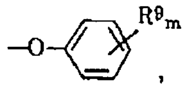, 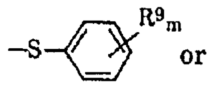 or 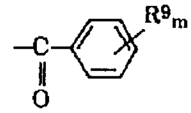

wherein $R^9$ is for a halogen, a lower alkyl, a lower cycloalkyl, a lower alkoxy, a halo-lower alkyl, a halo-lower alkoxy, a cyano or a nitro group, m denotes an integer 0 to 5 and $R^9$ may be different in each of the m repeating units, n denotes an integer of 0 to 5 and $R^8$ may be different in each of the n repeating units;

(9) a composition described in (8), wherein $R^8$ is represented by the formula

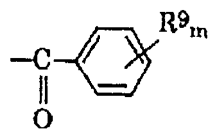

wherein $R^9$ is of the same meaning as defined above;

(10) a composition described in (9), wherein $R^9$ is a halogen atom;

(11) an composition described in (1), wherein the 3(2H)-pyridazinone is a compound of the formula:

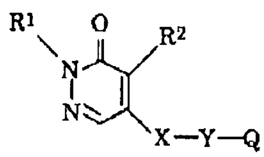

[I]

wherein $R^1$ is a lower alkyl group, a cycloalkyl group or an optinally substituted phenyl group, $R^2$ is a halogen, an optionally substituted lower alkoxy group, an optionally substituted lower alkylthio group or an optionally substituted aliphatic hydrocarbon group, X is O or S, Y is an optionally substituted alkylene group or an optionally substituted alkenylene group and Q is an optionally substituted aryl group or an optionally substituted aromatic heterocyclic group, or its salt;

(12) a composition described in (11), wherein $R^1$ is a branched lower alkyl group;

(13) a composition described in (11), where $R^2$ is a halogen, a lower alkoxy group or a lower alkyl group;

(14) a composition described in (11), wherein Y is represented by the formulae

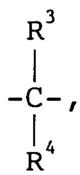, 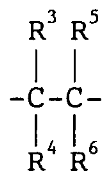 or 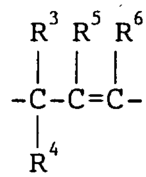

wherein $R^3$, $R^4$, $R^5$ and $R^6$ independently are selected from a hydrogen, a hydroxy group or an optionally substituted lower alkyl group;

(15) a 4,5-disubstituted 3(2H)-pyridazinone, the substituent at the 5-position being

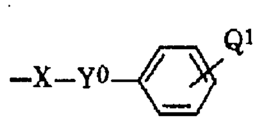

wherein X and Y are of the same meaning as defined above, $Q^1$ is an optionally substituted N-containing

aromatic heterocyclic group, or its salt;

(16) a 4,5-disubstituted 3(2H)-pyridazinone, the substitutent at the 5-position being -X-$Y^1$-$Q^0$ are of the same meaning as defined above, $Y^1$ is a divalent aliphatic hydrocarbon residue substituted with a hydroxy group, or its salt;

(17) the compound described in (15), which is 2-tert-Butyl-4-chloro-5-[4-(1,2,4-thiadiazol-5-yl)benzylthio]-3(2H)-pyridazinone;

(18) a compound of the formula:

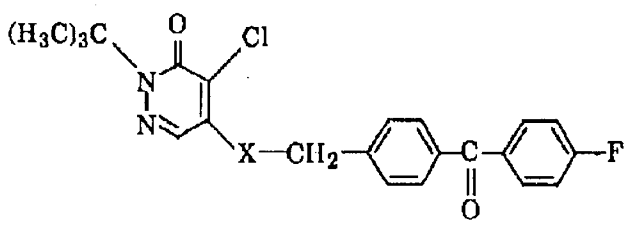

wherein X is of the same meaning as defined above, or its salt;

(19) a compound of the formula:

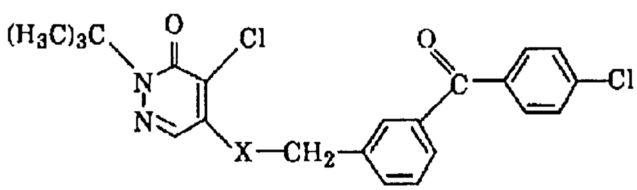

wherein X is of the same meaning as defined above, or its salt;

(20) a process for producing the compound described in (15), which comprises reacting a 4,5-disubstituted 3(2H)-pyridazinone, the substituent at the 5-position being -X-H wherein X is of the same meaning as defined above, with a compound of the formula:

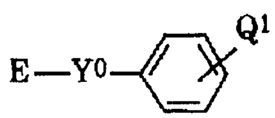

wherein E sands for a leaving group, $Y^0$ and $Q^1$ are of the same meaning as defined above, or its salt;

(21) a process for producing the compound described in (16), which comprises reacting a 4,5-disubstituted 3(2H)-pyridazinone, the substituent at the 5-position being -X-H wherein X is of the same meaning as defined above, with a compound of the formula: E-$Y^1$-Q wherein E, $Y^1$ and Q are of the same meaning as defined above, or its salt;

(22) a method for treating or preventing a tumor which comprises administrating an effective amount of a 4,5-disubstituted 3(2H)-pyridazinone, the substituent at the 5-position being -X-$Y^0$-$Q^0$ wherein X, $Y^0$ and $Q^0$ are of the same meaning as defined above, or its pharmaceutically acceptable salt together with a pharmaceutically acceptable carrier or diluent to mammal;

(23) use of a 4,5-disubstituted 3(2H)-pyridazinone, the substituent at the 5-position being -X-$Y^0$-$Q^0$ wherein X, $Y^0$ and $Q^0$ are of the same meaning as defined above, or a pharmaceutically acceptable salt thereof, as a component in the preparation of an antitumor composition;

(24) the antitumor composition described in (10), wherein $R^2$ is halogen or a lower alkyl group;

(25) the antitumor composition described in (10), wherein Y is

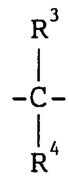 or 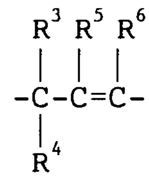

(wherein $R^3$, $R^4$, $R^5$ and $R^6$ are of the same meaning as defined above);
(26) the antitumor composition described in (10), wherein Y is

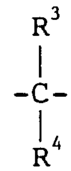

($R^3$ and $R^4$ are of the same meaning as defined above);
(27) the antitumor composition described in (10), wherein Q is an optionally substituted phenyl group, an optionally substituted naphthyl group or an optionally substituted quinolyl group;
(28) the antitumor composition described in (27), wherein the respective substituents of the phenyl group, naphthyl group and quinolyl group are an optionally substituted aryl group or N-containing aromatic heterocyclic group;
(29) a novel compound represented by the formula

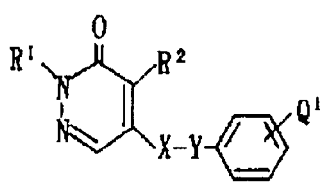

[II]

wherein $Q^1$ is an N-containing heterocyclic group, and other symbols are of the same meaning as defined above or a salt thereof; and
(30) a compound represented by the formula

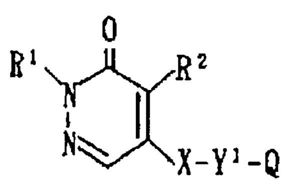

[III]

wherein $Y^1$ is

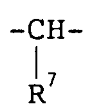

(wherein $R^7$ is a lower alkyl group substituted with a hydroxy group), and other symbols are of the same meanings as defined above, or a salt thereof.

As set forth above, X is O or S, and $Y^0$ is an optionally substituted divalent aliphatic hydrocarbon residue.

As the divalent aliphatic hydrocarbon residue represented by $Y^0$, use is made of, for example of an alkylene group or alkenylene group.

As the alkylene group shown by $Y^0$, use is made of, for example, $C_{1-6}$ alkylene groups such as methylene, ethylene, propylene and butylene, and, among them, for example, methylene and ethylene are preferable.

As the alkenylene groups shown by $Y^0$, use is made of, for example, $C_{1-6}$ alkenylene groups such as vinylene, propenylene and butenylene, and, among them, for example, propenylene is preferable.

The alkylene group or alkenylene group, which are shown by $Y^0$, may optionally be substituted, at any substitutive position, with 1 to 5, preferably 1 to 2, substituents exemplified by a hydroxyl group, a halogen (e.g.,fluorine, chlorine, bromine and iodine), a cyano group, a nitro group, a di-lower alkylamino group (e.g.,di-$C_{1-3}$ alkylamino such as dimethylamino and diethylamino), a lower alkoxy group (e.g.,a $C_{1-4}$ alkoxy group such as methoxy, ethoxy, propoxy, isopropoxy. butoxy and tert-butoxy), a lower alkoxycarbonyl group (e.g.,a (C1-4 alkoxy) carbonyl group such as methoxycarbonyl, ethoxycarbonyl, isopropoxycarbonyl, butoxycarbonyl, tert-butoxycarbonyl), an optionally substituted lower alkyl group (e.g.,substantially the same ones as the lower alkyl groups shown by $R^1$, which may be substituted, at any substitutive position, with one or two of, for example, hydroxyl groups), and, as such substituents, the hydroxyl group, for example, is preferable.

$Q^0$ is an optionally substituted cyclic group.

As the cyclic group represented by $Q^0$, use is made of, for example, a cyclic hydrocarbon residue or an aromatic heterocyclic group.

As the cyclic hydrocarbon residue represented by $Q^0$, use is made of, for example, an aryl group or a cyclohexyl group.

As the aryl group shown by $Q^0$, use is made of, for example, a $C_{6-10}$ aryl group such as phenyl or 1-or 2-naphthyl. Phenyl is preferable.

As the aromatic heterocyclic group shown by $Q^0$, use is made of a 5- or 6-membered aromatic heterocyclic group containing, in addition to carbon atoms, 1 to 4 hetero-atoms selected from oxygen, sulfur, nitrogen and so on, and, further, condensed heterocyclic groups formed by condensation of these 5- or 6-membered aromatic heterocyclic groups with each other or those formed by condensation of the 5- or 6-membered aromatic heterocyclic groups with a phenyl group, for example, thienyl, furyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, benzofuranyl, isobenzofuranyl, benzo[b]-thienyl, indolyl, isoindolyl, 1H-indazolyl, benzimidazolyl, benzoxazolyl, 1,2-benzisoxazolyl, benzothiazolyl, 1,2-isobenzothiazolyl, 1H-benzotriazolyl, quinolyl, isoquinolyl, cinnolyl, quinazolyl, quinoxalyl, phthalazinyl, naphthylizinyl, purinyl, pteridinyl, carbazolyl, $\alpha$-carbolinyl, $\beta$-carbolinyl, $\gamma$-carbolinyl, acridinyl, phenoxazinyl, phenothiazinyl, phenazinyl, phenoxathiinyl, thianthrenyl, phenanthridinyl, phenanthrolinyl, indolizinyl, pyrrolo-[1,2-b]pyridazinyl, pyrrolo[1,2-a]pyrazinyl, pyrazolo[1,5-a]pyridyl, imidazo[1,2-a]pyridyl, imidazo[1,5-a]-pyridyl, imidazo[1,2-b]pyridazinyl, imidazo[1,2-a]pyrimidinyl, 1,2,4-triazolo[4,3-a]pyridyl and 1,2,4-triazolo-[4,3-a]pyridazinyl. Among them, 5- or 6-membered N-containing heterocyclic groups or condensed heterocyclic groups of them such as pyridyl, benzoimidazolyl, isoxazolyl and quinolyl are preferable, and, for example, quinolyl is more preferable.

Such aryl groups, cyclohexyl groups and aromatic heterocyclic groups shown by $Q^0$ as described above may optionally be substituted, at any substitutive position, with 1 to 5, preferably 1 to 3 substituents as exemplified by a hydroxyl group, an amino group, a cyano group, a carboxyl group, a lower alkoxycarbonyl group (e.g.,a $C_{1-6}$ alkoxycarbonyl group such as methoxycarbonyl, ethoxycarbonyl and tert-butoxycarbonyl), a nitro group, a lower alkyl group (e.g.,a $C_{1-6}$ alkyl group such as methyl, ethyl, propyl, isopropyl, butyl, isobutyl, tert-butyl, pentyl and hexyl), a lower alkenyl group (e.g.,a $C_{2-6}$ alkenyl group such as vinyl and 2-propenyl), a lower alkynyl group (e.g.,a $C_{2-6}$ alkynyl group such as ethynyl, 1-propynyl, 2-propynyl and 2-butynyl), a lower alkoxy group (e.g.,a $C_{1-6}$ alkoxy group such as methoxy, ethoxy and propoxy), a lower alkylthio group (e.g.,a $C_{1-6}$ alkylthio group such as methylthio and ethylthio), a halo lower alkyl group (e.g.,a halo- $C_{1-6}$ alkyl group such as fluoromethyl, trifluoromethyl and 2,2-difluoromethyl), a mono- or di- $C_{1-6}$ alkylamino group (e.g.,a mono- or di- $C_{1-6}$ alkylamino group such as methylamino, ethylamino, propylamino, dimethylamino and diethylamino), a halogen (e.g.,fluorine, chlorine, bromine and iodine), a trityl group, a styryl group, a phenyl group (optionally substituted with 1 to 2 of e.g.,cyano groups), a benzoyl group (optionally substituted with 1 to 3 halogens (e.g.,fluorine, chlorine, bromine and iodine)) or a benzyloxy group (optionally substituted with 1 to 3 halogens (e.g.,fluorine, chlorine, bromine

and indine), preferably, for example, the cyano group, a $C_{1-4}$ alkyl group such as tert-butyl or the phenyl group.

When $Q^0$ is a phenyl group, it may be substituted with an N-containing aromatic heterocyclic group having, in addition to the above-mentioned substituents, further substituents. As said N-containing aromatic heterocyclic group, use is made of a 5- or 6-membered aromatic monocyclic heterocyclic group such as pyrrolyl, oxazolyl, isoxazoly, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyi, 1,3,4-thiadiazolyl, 1,2,3-triazolyl, 1,2,4-triazolyl, tetrazolyl, pyridyl, pyridazinyl, pyrimidinyl, pyrazinyl and triazinyl, an aromatic condensed heterocyclic ring such as indolyl, isoindolyl, 1H-indazolyl, benzimidazolyl, benzoxazolyl, 1,2-benzisoxazolyl, benzothiazolyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, $\alpha$-carbolinyl, $\beta$-carbolinyl, $\gamma$-carbolinyl, acridinyl, pyrrolo[1,2-b]pyridazinyl, pyrrolo[1,2-a]pyrazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl, preferably, for example, pyrrolyl, imidazolyl, oxazolyl, isoxazolyl, thiazolyl, thiadiazolyl, pyridyl, pyrimidinyl, pyrazinyl, quinolyl and benzoimidazolyl, more preferably, pyrrolyl, imidazolyl, pyridyl and quinolyl. Such N-containing aromatic heterocyclic group as described above may optionally be substituted, at any substitutive position, with 1 to 3 substituents, independently selected from, for example, a lower alkyl group (e.g.,a $C_{1-3}$ alkyl group such as methyl, ethyl, propyl and isopropyl), a hydroxyl group, an amino group, a mono- or di-lower alkylamino group (e.g.,a mono- or di- $C_{1-3}$alkyl amino group such as methylamino, ethylamino, dimethylamino and diethylamino), a halogen (e.g.,fluorine, chlorine, bromine and iodine) and a trityl group.

Preferable examples of the groups shown by $Q^0$ are

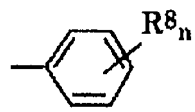

wherein $R^8$ and n are as defined above.

A preferable example of $R^8$ is represented by the formula

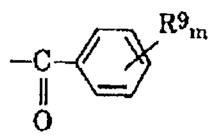

wherein $R^9$ and n are as defined above.

A preferable example of $R^9$ is halogen.

As the halogen shown by $R^8$ and $R^9$, use is made of, for example, fluorine, chlorine, bromine and iodine.

As the lower alkyl group of $R^8$ and $R^9$, use is made of, for example, a $C_{1-6}$ alkyl group such as methyl, ethyl, propyl, isopropyl, butyl, isobutyl, tert-butyl, pentyl and hexyl.

As the lower cycloalkyl group of $R^8$ and $R^9$, use is made of, for example, a $C_{3-6}$ cycloalkyl group such as cyclopropyl, cyclobutyl cyclopentyl and cyclohexyl.

As the lower alkoxy group of $R^8$ and $R^9$, use is made of, for example, a $C_{1-6}$ alkoxy group such as methoxy, ethoxy and propoxy

As the halo-lower alkyl group of $R^8$ and $R^9$, use is made of, for example, a halo-$C_{1-6}$ alkyl group such as fluoromethyl, trifluoromethyl and 2,2-difluoromethyl.

As the halo-lower alkoxy group of $R^8$ and $R^9$, use is made of, for example, a halo-$C_{1-6}$ alkoxy group such as fluoromethoxy, trifluoromethoxy and 2,2-difluoromethoxy.

The substituent at the 4-position of the 3(2H)-pyridazinone is a group having 1 to 150 atoms, preferably 1 to 50 atoms and most preferably 1 to 25 atoms.

The 3(2H)-pyridazinone may be substituted with an optionally subtituted hydrocarbon residue at the 2-position. As the hydrocarbon residue substituent at the 2-position, use is made of, for example, of an optionally substituted lower alkyl, a cycloalkyl group, an optionally substituted phenyl group or aralkyl group.

As the lower alkyl group substituent at the 2-position of the 3(2H)-pyridazinone, use is made of, among others, $C_{1-8}$ straight chain or branched alkyl groups including, for example, methyl, ethyl, propyl, isopropyl, butyl, isobutyl, sec-butyl, tert-butyl, pentyl, neopentyl, tert-pentyl, 1-methylbutyl, 2-methylbutyl, 1,2-dimethylpropyl, 1-ethylpropyl, hexyl, isohexyl, a-methylpentyl, 2-methylpentyl, 3-methylpentyl, 1,1-dimethyl butyl, 2,2-dibutylbutyl, 3,3-dimethylbutyl, 1,3-dibutylbutyl, 2,3-dibutylbutyl, 1-ethylbutyl, 2-ethylbutyl, 1,1,2-trimethylpropyl, 1-ethyl-1-methylpropyland 1-ethyl-2-methylpropyl. Among them, $C_{1-4}$ alkyl groups as exemplified by methyl, ethyl, isopropyl and tert-butyl are preferable.

The cycloalkyl group substituent at the 2-position of the 3(2H)-pyridazinone, may optionally be substituted, at any substitutive position, with 1 to 5, preferably 1 to 3 substituents independently selected from a hydroxyl group, an amino group, a carboxyl group, a nitro group, a lower alkyl group (for example, substantially the same ones as the lower alkyl groups shown by the substituent at the 2-position of the 3-(2H)-pyridazinone, mentioned above, preferably $C_{1-6}$alkyl groups as exemplified by methyl, ethyl, propyl, isopropyl, butyl, isobutyl, tert-butyl, pentyl and hexyl), a lower alkoxy group (for example, substantially the same ones as the lower alkoxy groups shown by the substituent at the 4-position of the 3(2H)-pyridazinone below, preferably $C_{1-4}$ alkoxy groups as exemplified by methoxy, ethoxy and propoxy), a halo lower alkyl group (for example, halo-$C_{1-6}$ alkyl groups as exemplified by fluoromethyl, trifluoromethyl and 2,2-difluoromethyl), a mono- or di- lower alkylamino group (for example, mono- or di- $C_{1-6}$ alkyl amino groups as exemplified by methylamino, ethylamino, propylamino, dimethylamino and diethylamino) and a halogen (for example, fluorine, chlorine and iodine). Preferable examples of such substituents include halo- lower alkyl groups and halogen. Among them, chlorine and trifluoromethyl, for example, are more preferable.

As the aralkyl group substituent at the 2-position, use is made of, among others, $C_{7-16}$ aralkyl groups including, for example, benzyl, 1-phenylethyl, 2-phenylethyl and 1-phenylpropyl. Among them, benzyl is preferable.

The lower alkyl group substituent at the 2-position of the 3(2H)-pyridazinone, may be substituted with 1 to 5, preferably 1 to 3 substituents inedependently selected from for example, a hydroxy group, a $C_{1-6}$ alkoxy group (for example, methoxy and ethoxy), a $C_{1-3}$ alkoxy-$C_{1-6}$ alkoxy group (for example, methoxymethoxy and methoxyethoxy), a $C_{1-6}$ alkanoyloxy group (for example, acetoxy, propionyloxy, butyryloxy and pivaloyloxy), a di($C_{1-6}$ alkyl) amino group (for example, dimethylamino and diethylamino), di(halo-$C_{1-6}$ alkyl) amino group (for example, di(chloroethyl)amino) and cyclic amino (for example, 1-pyrrolidinyl and 1-piperidyl).

As the group having 1 to 150 atoms, (i.e., the substituent at the 4-position, use is made of, for example, a halogen, an optionallly substituted lower alkoxy, an optionally substituted lower alkylthio group or an optionally substituted aliphatic hydrocarbon residue.

As the halogen substituent at the 4-position of the 3(2H)-pyridazinone, use is made of, for example, fluorine, chlorine, bromine and iodine. Among them, chlorine and bromine are preferable, and chlorine is more preferable.

Examples of the lower alkoxy group substituent at 4-position of the 3(2H)-pyridazinone, include $C_{1-8}$ straight chain or branched alkoxy groups as exemplified by methoxy, ethoxy, propoxy, isopropoxy, butoxy, isobutoxy, sec-butoxy, tert-butoxy, pentyloxy, isopentyloxy, neopentyloxy, tert-pentyloxy, 1-methylbutoxy, 2-methylbutoxy, 1,2-dimethylpropoxy, 1-ethylpropoxy, hexyloxy, isohexyloxy, 1-methylpentyloxy, 2-methylpentyloxy, 3-methylpentyloxy, 1,1-dimethylbutyloxy, 2,2-dibutylbutoxy, 3,3-dimethylbutoxy, 1,3-dibutylbutoxy, 2,3-dibutylbutoxy, 1-ethylbutoxy, 2-ethylbutoxy, 1,1,2-trimethylpropoxy, 1-ethyl-1-methylpropoxy and 1-ethyl-2-methylpropoxy, and, among them, $C_{1-4}$ alkoxy groups such as methoxy and ethoxy are preferable.

Examples of the lower alkylthio group substituent at the 4-position of the 3(2H)-pyridazinone include $C_{1-8}$ straight chain or branched alkylthio groups as exemplified by methylthio, ethylthio, propylthio, isopropylthio, butylthio, isobutylthio, sec-butylthio, tert-butylthio, pentylthio, isopentylthio, neopentylthio, tert-pentylthio, 1-methylbutylthio, 2-methylbutylthio, 1,2-dimethylpropylthio, 1-ethylpropylthio, hexylthio, isohexylthio, 1-methylpentylthio, 2-methylpentylthio, 3-methylpentylthio, 1,1-dimethylbutylthio, 2,2-dibutylthio, 3,3-dimethylbutylthio, 1,3-dibutylthio, 2,3-dibutylbutylthio, 1-ethylbutylthio, 2-ethylbutylthio, 1,1,2-trimethylpropylthio, 1-ethyl-1-methylpropylthio and 1-ethyl-2-methylpropylthio, and, among them, $C_{1-4}$ alkyl thio groups such as methylthio and ethylthio are preferable.

As the aliphatic hydrocarbon group substituent at the 4-position of 3(2H)-pyridazinone use is made of, for example, lower alkyl groups, lower alkenyl groups and lower alkynyl groups, and, among them, lower alkyl groups are preferable. As such lower alkyl groups, use is made of, for example, ones which are substantially the same as the lower alkyl groups shown by the above-mentioned $R^1$, and, among them, $C_{1-4}$ alkyl groups such as methyl, ethyl and isopropyl are preferable, and the methyl group, for example, is more preferable. As the lower alkenyl groups, use is made of, for example, $C_{2-8}$ straight chain or branched

alkenyl groups, as exemplified by vinyl, 1-propenyl, 2-propenyl, isopropenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-hexenyl, 2-hexenyl, 3-hexenyl, 4-hexenyl, 5-hexenyl, 1-methyl-1-pentenyl, 2-methyl-1-pentenyl, 4-methyl-3-pentenyl and 2-ethyl-1-butenyl, and, among them, $C_{2-4}$ alkenyl groups such as vinyl and 2-propenyl are preferable. As the lower alkynyl groups, use is made of $C_{2-8}$ straight chain or branched alkynyl groups, as exemplified by ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-3-butynyl, 2-methyl-3-butynyl, 1-hexynyl, 2-hexynyl, 3-hexynyl, 4-hexenyl and 5-hexenyl, and, among them, $C_{2-4}$ alkynyl groups such as 2-propynyl are preferable.

The lower alkoxy groups, lower alkylthio groups and aliphatic hydrocarbon groups, which are the substituent at the 4-position of the 3(2H)-pyridazinone, may optionally be substituted, at any substitutive position, with 1 to 3, more preferably one, of a hydroxyl group, a halogen (e.g.,fluorine, chlorine, bromine and iodine), an amino group, mono- or di-lower alkylamino groups (e.g.,mono- or di-$C_{1-6}$ alkylamino such as methylamino, ethylamino, propylamino and dimethylamino), and, among these substituents, the hydroxyl group and the amino group, for example, are more preferable.

Preferable examples of groups shown by $R^2$ are halogen such as chlorine, $C_{1-4}$ alkoxy groups such as methoxy, and $C_{1-4}$ alkyl groups such as methyl, and, among them, chlorine and methyl are more preferable. The compound of the formula:

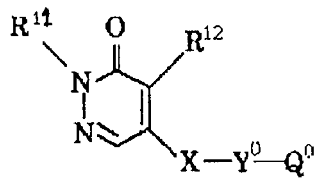

wherein $R^{11}$ is a hydrogen or an optionally substituted hydrocarbon residue, $R^{12}$ is a group having 1 to 150 atoms, X, $Y^0$ and $Q^0$ is of the same meaning as defined above, is a preferable example of the 3(2H)-pyridazinone antitumor compound of this invention. The optionally substituted hydrocarbon residue of $R^{11}$ is the same as described for the substituent at the 2-position of the 3(2H)-pyridazinone mentioned above.
The group having 1 to 150 atoms shown by $R^{12}$ is the same as described for the substituent at the 4-position of the 3(2H)-pyridazinone mentioned above.
The compound represented by formula [I] is a preferable 3(2H)-pyridazinone of this invention.
$R^1$, as set forth previously, is a lower alkyl group, a cycloalkyl group or an optionally substituted phenyl group.

As the lower alkyl group represented by $R^1$, use is made of, among others, $C_{1-8}$ straight chain or branched alkyl groups including, for example, methyl, ethyl, propyl, isopropyl, butyl, isobutyl, sec-butyl, tert-butyl, isopentyl, pentyl, neopentyl, tert-pentyl, 1-methylbutyl, 2-methylbutyl, 1,2-dimethylpropyl, 1-ethylpropyl, hexyl, isohexyl, 1-methylpentyl, 2-methylpentyl, 3-methylpentyl, 1,1-dimethylbutyl, 2,2-dibutylbutyl, 3,3-dimethylbutyl, 1,3-dibutylbutyl, 2,3-dibutylbutyl, 1-ethylbutyl, 2-ethylbutyl, 1,1,2-trimethylpropyl, 1-ethyl-1-methylpropyl and 1-ethyl-2-methylpropyl. Among them, $C_{1-4}$ alkyl groups as exemplified by methyl, ethyl, isopropyl and tert-butyl are preferable.

As the cycloalkyl group represented by $R^1$, use is made of, among others, $C_{3-8}$ cycloalkyl groups including, for example, cyclopropyl, cyclobutyl and cyclohexyl. Among them, for example, cyclopentyl and cyclohexyl are preferable.

The phenyl group represented by $R^1$ may optionally be substituted, at any substitutive position, with 1 to 5, preferably 1 to 3 substituents independently selected from, for example, a hydroxyl group, an amino group, a carboxyl group, a nitro group, a lower alkyl group (for example, substantially the same ones as the lower alkyl groups shown by $R^1$ mentioned above, preferably $C_{1-6}$ alkyl groups as exemplified by methyl, ethyl, propyl, isopropyl, butyl, isobutyl, tert-butyl, pentyl and hexyl), a lower alkoxy group (for example, substantially the same ones as the lower alkoxy groups shown by $R^2$ below, preferably $C_{1-4}$ alkoxy groups as exemplified by methoxy, ethoxy and propoxy), a halo lower alkyl group (for example, halo-$C_{1-6}$ alkyl groups as exemplified by fluoromethyl, trifluoromethyl and 2,2-difluoromethyl), a mono- or di- lower alkylamino group (for example, mono- or di- $C_{1-6}$ alkyl amino groups as exemplified by methylamino, ethylamino, propylamino, dimethylamino and diethylamino) and a halogen (for example, fluorine, chlorine, bromine and iodine). Preferable examples of such substituents include halo-lower alkyl groups and halogen.

Among them, chlorine and trifluoromethyl, for example, are more preferable.

Preferable example of groups shown by $R^1$ are hydrocarbon groups with more than 3 carbons and branched at the $\alpha$-position, and among them, $C_{3-8}$ branched alkyl groups such as tert-butyl are more preferable.

$R^2$, as set forth previously, is H, halogen, an optionally substituted lower alkoxy group, an optionally substituted lower alkylthio group or an optionally substituted aliphatic hydrocarbon group.

As the halogen shown by $R^2$, use is made of, for example, fluorine, chlorine, bromine and iodine. Among them, chlorine and bromine are preferable, and chlorine is more preferable.

Examples of the lower alkoxy group shown by $R^2$ include $C_{1-8}$ straight chain or branched alkoxy groups as exemplified by methoxy, ethoxy, propoxy, isopropoxy, butoxy, isobutoxy, sec-butoxy, tert-butoxy, pentyloxy, isopentyloxy, neopentyloxy, tert-pentyloxy, 1-methylbutoxy, 2-methylbutoxy, 1,2-dimethylpropoxy, 1-ethylpropoxy, hexyloxy, isohexyloxy, 1-methylpentyloxy, 2-methylpentyloxy, 3-methylpentyloxy, 1,1-dimethylbutyloxy, 2,2-dibutylbutoxy, 3,3-dimethylbutoxy, 1,3-dibutylbutoxy, 2,3-dibutylbutoxy, 1-ethylbutoxy, 2-ethylbutoxy, 1,1,2-trimethylpropoxy, 1-ethyl-1-methylpropoxy and 1-ethyl-2-methylpropoxy, and, among them, $C_{1-4}$ alkoxy groups such as methoxy and ethoxy are preferable.

Examples of the lower alkylthio group shown by $R^2$ include $C_{1-8}$ straight chain or branched alkylthio groups as exemplified by methylthio, ethylthio, propylthio, isopropylthio, butylthio, isobutylthio, sec-butylthio, tert-butylthio, pentylthio, isopentylthio, neopentylthio, tert-pentylthio, 1-methylbutylthio, 2-methylbutylthio, 1,2-dimethylpropylthio, 1-ethylpropylthio, hexylthio, isohexylthio, 1-methylpentylthio, 2-methylpentylthio, 3-methylpentylthio, 1,1-dimethylbutylthio, 2,2-dibutylthio, 3,3-dimethylbutylthio, 1,3-dibutylthio, 2,3-dibutylbutylthio, 1-ethylbutylthio, 2-ethylbutylthio, 1,1,2-trimethylpropylthio, 1-ethyl-1-methylpropylthio and 1-ethyl-2-methylpropylthio, and, among them, $C_{1-4}$ alkyl thio groups such as methylthio and ethylthio are preferable.

As the aliphatic hydrocarbon groups shown by $R^2$, use is made of, for example, lower alkyl groups, lower alkenyl groups and lower alkynyl groups, and, among them, lower alkyl groups are preferable. As such lower alkyl groups, use is made of, for example, ones which are substantially the same as the lower alkyl groups shown by the above-mentioned $R^1$, and, among them, $C_{1-4}$ alkyl groups such as methyl, ethyl and isopropyl are preferable, and the methyl group, for example, is more preferable. As the lower alkenyl groups, use is made of, for example, $C_{2-8}$ straight chain or branched alkenyl groups, as exemplified by vinyl, 1-propenyl, 2-propenyl, isopropenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-hexenyl, 2-hexenyl, 3-hexenyl, 4-hexenyl, 5-hexenyl, 1-methyl-1-pentenyl, 2-methyl-1-pentenyl, 4-methyl-3-pentenyl and 2-ethyl-1-butenyl, and, among them, $C_{2-4}$ alkenyl groups such as vinyl and 2-propenyl are preferable. As the lower alkynyl groups, use is made of $C_{2-8}$ straight chain or branched alkynyl groups, as exemplified by ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-3-butynyl, 2-methyl-3-butynyl, 1-hexynyl, 2-hexynyl, 3-hexynyl, 4-hexenyl and 5-hexenyl, and, among them, $C_{2-4}$ alkynyl groups such as 2-propynyl are preferable.

The lower alkoxy groups, lower alkylthio groups and aliphatic hydrocarbon groups, which are shown by $R^2$, may optionally be substituted, at any substitutive position, with 1 to 3, more preferably one, of hydroxyl group, a halogen (e.g.,fluorine, chlorine, bromine and iodine), an amino group, mono- or di-lower alkylamino groups (e.g.,mono- or di-$C_{1-6}$ alkylamino such as methylamino, ethylamino, propylamino and dimethylamino), and, among these substituents, the hydroxyl group and amino group for example are more preferable.

Preferable examples of groups shown by $R^2$ are halogen such as chlorine, $C_{1-4}$ alkoxy groups such as methoxy, and $C_{1-4}$ alkyl groups such as methyl, and, among them, chlorine and methyl are more preferable.

As previously set forth, X is O or S, and the former is preferable.

Y is an optionally substituted alkylene group or an optionally substituted alkenylene group.

As the alkylene group shown by Y, use is made of, for example, $C_{1-6}$ alkylene groups such as methylene, ethylene, propylene and butylene, and, among them, for example, methylene and ethylene are preferable.

As the alkenylene groups shown by Y, use is made of, for example, $C_{1-6}$ alkenylene groups such as vinylene, propenylene and butenylene, and, among them, for example, propenylene is preferable.

The alkylene group or alkenylene group, which are shown by Y, may optionally be substituted, at any substitutive position, with 1 to 5, preferably 1 to 2, substituents independently selected from, for example, a hydroxyl group, a halogen (e.g.,fluorine, chlorine, bromine and iodine), a cyano group, a nitro group, a di-lower alkylamino group (e.g.,di-$C_{1-3}$ alkylamino such as dimethylamino and diethylamino), a lower alkoxy group (e.g.,a $C_{1-4}$ alkoxy group such as methoxy, ethoxy, propoxy, isopropoxy, butoxy and tert-butoxy), a lower alkoxycarbonyl group (e.g.,a ($C_{1-4}$ alkoxy)carbonyl group such as methoxycarbonyl, ethoxycarbonyl, isopropoxycarbonyl, butoxycarbonyl, tert-butoxycarbonyl), an optionally substituted lower alkyl group (e.g.,substantially the same ones as the lower alkyl groups shown by $R^1$, which may be substituted, at any substitutive position, with one or two of, for example, hydroxyl groups), and, as such substituents, the hydroxyl group, for example, is preferable.

As Y, use is often made of, for example, groups represented by the formulae

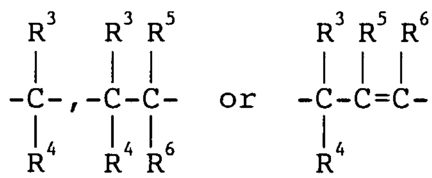

wherein $R^3$, $R^4$, $R^5$ and $R^6$, independently are selected from a hydrogen, a hydroxy group or an optionally substituted lower alkyl group.

As the lower alkyl groups shown by $R^3$, $R^4$, $R^5$ and $R^6$, use is made of, for example, such ones as substantially the same with lower alkyl groups shown by $R^1$ described above, preferably $C_{1-4}$ alkyl groups such as methyl or ethyl. Such lower alkyl groups may have one or two substituents (e.g.,hydroxyl groups) at any substitutive position.

As Y, use is made of, for example,

$-CH_2-$,

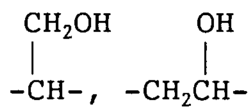

or $-CH_2-CH=CH-$

more preferably, for example,

$-CH_2-$,

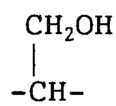

or $-CH_2-CH=CH-$

further more preferably, for example,

$-CH_2-$

Q is an optionally substituted aryl group or an optionally substituted aromatic heterocyclic group.

As the aryl group shown by Q, use is made of, for example, a $C_{6-10}$ aryl group such as phenyl or 1- or 2-naphthyl. Phenyl is preferable.

As the aromatic heterocyclic group shown by Q, use is made of a 5- or 6-membered aromatic heterocyclic group containing, in addition to carbon atoms 1 to 4 hetero-atoms selected from oxygen, sulfur, nitrogen and so on, and, further, condensed heterocyclic groups formed by condensation of these 5- or 6-membered aromatic heterocyclic groups with each other or those formed by condensation of the 5- or 6-membered aromatic heterocyclic groups with a phenyl group, for example, thienyl, furyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, 1,2-isobenzothiazolyl, 1H-benzotriazolyl, quinolyl, isoquinolyl, cinnolyl, quinazolyl, quinoxalyl, phthalazinyl, naphthylizinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrrolo[1,2-a]pyrazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-a]pyridazinyl. Among them, 5- or 6-membered N-containing heterocyclic groups or condensed heterocyclic groups of them such as pyridyl, benzoimidazolyl, isoxazolyl and quinolyl are preferable, and, for example, quinolyl is more preferable.

Such aryl groups and aromatic heterocyclic groups shown by Q as described above may optionally be substituted, at any substitutive position, with 1 to 5, preferably 1 to 3 substituents independently selected from, for example, a hydroxyl group, amino group, cyano group, carboxyl group, a lower alkoxycarbonyl group (e.g.,a $C_{1-6}$ alkoxycarbonyl group such as methoxycarbonyl, ethoxycarbonyl and tert-butoxycarbonyl), a nitro group, a lower alkyl group (e.g.,a $C_{1-6}$ alkyl group such as methyl, ethyl, propyl, isopropyl, butyl, isobutyl, tert-butyl, pentyl and hexyl), a lower alkenyl group (e.g.,a $C_{2-6}$ alkenyl group such as vinyl and 2-propenyl), a lower alkynyl group (e.g.,$C_{2-6}$ alkynyl group such as ethynyl, 1-propynyl, 2-propynyl and 2-butynyl), a lower alkoxy group (e.g.,a $C_{1-6}$ alkoxy group such as methoxy, ethoxy and propoxy), a lower alkylthio group (e.g.,a $C_{1-6}$ alkylthio group such as methylthio and ethylthio), a halo lower alkyl group (e.g.,a halo- $C_{1-6}$ alkyl group such as fluoromethyl, trifluoromethyl and 2,2-difluoromethyl), a mono- or di-$C_{1-6}$ alkylamino group (e.g.,a mono- or di- $C_{1-6}$ alkylamino group such as methylamino, ethylamino, propylamino, dimethylamino and diethylamino), a halogen (e.g.,fluorine, chlorine, bromine and iodine), a trityl group, a styryl group a phenyl group (optionally substituted with 1 to 2 of e.g.,cyano group), a benzoyl group (optionally substituted with 1 to 3 halogen (e.g.,fluorine, chlorine, bromine and iodine)) or a benzyloxy group (optionally substituted with 1 to 3 halogen (e.g.,fluorine, chlorine, bromine and iodine)), preferably, for example, the cyano group, a $C_{1-4}$ alkyl group such as tert-butyl, or the phenyl group.

When Q is a phenyl group, it may be substituted with an N-containing aromatic heterocyclic group having, in addition to the above-mentioned substituents, further substituents. As said N-containing aromatic heterocyclic group, use is made of a 5- or 6-membered aromatic monocyclic heterocyclic groups such as pyrrolyl, oxazolyl, isoxazoly, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl, an aromatic condensed heterocyclic ring such as indolyl, isoindolyl, 1H-indazolyl, benzimidazolyl, benzoxazolyl, 1,2-benzisoxazolyl, benzothiazolyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, pyrrolo[1,2-b]pyridazinyl, pyrrolo[1,2-a]pyrazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl, preferably, for example, pyrrolyl, imidazolyl, oxazolyl, isoxazolyl, thiazolyl, thiadiazolyl, pyridyl, pyrimidinyl, pyrazinyl, quinolyl and benzimidazolyl, more preferably, pyrrolyl, imidazolyl, pyridyl and quinolyl. Such N-containing aromatic heterocyclic groups as described above may optionally be substituted, at any substitutive position, with 1 to 3 substituents, independently selected from, for example, a lower alkyl group (e.g.,a $C_{1-3}$ alkyl group such as methyl, ethyl, propyl and isopropyl), hydroxyl group, an amino group, a mono- or di-lower alkylamino group (e.g.,a mono- or di- $C_{1-3}$ alkyl amino group such as methylamino, ethylamino, dimethylamino and diethylamino), halogen (e.g.,fluorine, chlorine, bromine and iodine) and a trityl group.

Preferable examples of Q include phenyl, (4-tert-butyl)phenyl, biphenyl, (2-pyridyl)phenyl, naphthyl, quinolyl, 4-trifluoromethyl)phenyl, 4-(tert-butylcarbonyl)phenyl, p-chlorobenzoylpenyl, 4-[2-(p-flubrobenzyloxy)]pyridyl.

$Q^1$ is an optionally substitutive N-containing aromatic heterocyclic group.

As the N-containing aromatic heterocyclic group shown by $Q^1$, use is made of a 5- or 6-membered aromatic monocyclic heterocyclic groups such as pyrrolyl, oxazolyl, isoxazoly, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl, an N-containing aromatic condensed heterocyclic ring such as indolyl, isoindolyl, 1H-indazolyl, benzimidazolyl, benzoxazolyl, 1,2-benzisoxazolyl, benzothiazolyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, pyrrolo[1,2-b]pyridazinyl, pyrrolo[1,2-a]pyrazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl, preferably, for example, pyrrolyl, imidazolyl, oxazolyl, isoxazolyl, thiazolyl, thiadiazolyl, pyridyl, pyrimidinyl, pyrazinyl, quinolyl and benzoimidazolyl, more preferably, pyrrolyl, imidazolyl, pyridyl and quinolyl. Such N-containing aromatic

heterocyclic group as described above may optionally be substituted, at any substitutive position, with 1 to 3 substituents, independently, selected from, for example, a lower alkyl group (e.g.,a $C_{1-3}$ alkyl group such as methyl, ethyl, propyl and isopropyl), a hydroxyl group, an amino group, a mono-or di-lower alkylamino group (e.g.,a mono- or di- $C_{1-3}$ alkyl amino group such as methylamino, ethylamino, dimethylamino and diethylamino), a halogen (e.g.,fluorine, chlorine, bromine and iodine) and a trityl group.

$Y^1$ is a group represented by the formula

$$-\underset{R^7}{\underset{|}{CH}}-$$

wherein $R^7$ is a lower alkyl group substituted with a hydroxyl group. As the lower alkyl group shown by $R^7$, use is made of, for example, $C_{1-6}$ straight or branched alkyl groups such as methyl, ethyl, propyl, isopropyl, butyl, isobutyl, pentyl and hexyl, and, among them, for example, methyl and ethyl are preferable. Such lower alkyl groups as mentioned above may optionally be substituted with 1 or two substituents, for example hydroxyl groups.

Among them, for example, a hydroxy- $C_{1-4}$ alkyl group such as hydroxymethyl is more preferable.

Preferable examples of the combination of $R^2$, Y and Q include 1 to 8 set forth in [Table 1]. In these combinations, $R^1$ is a lower alkyl group, and X is O or S.

[Table 1]

| | $R^2$ | Y | Q |
|---|---|---|---|
| 1 | halogen | $C_{1-6}$ alkylene gp. | aryl gp.opt.substed. with a lower alkyl gp. |
| 2 | halogen | $\overset{CH_2OH}{\overset{\vert}{-CH-}}$ | N-containing aromatic heterocyclic gp. |
| 3 | $C_{1-4}$ alkoxy gp. | $C_{1-6}$ alkylene gp. | phenyl gp.substed. with opt. substed. aryl gp. |
| 4 | $C_{1-4}$ alkoxy gp. | $C_{1-6}$ alkylene gp. | quinolyl gp. |
| 5 | $C_{1-4}$ alkoxy gp. | $-CH_2-CH=CH-$ | aryl gp.opt. substed. with a lower alkyl gp. |
| 6 | $C_{1-4}$ alkoxy gp. | $\overset{OH}{\overset{\vert}{-CH_2CH-}}$ | N-containing aromatic heterocyclic gp. |
| 7 | $C_{1-4}$ alkyl gp. | $-CH_2-$ | opt. substed. phenyl gp. |
| 8 | $C_{1-4}$ alkyl gp. | $\overset{CH_2OH}{\overset{\vert}{-CH-}}$ | opt. substed. N-containing aromatic heterocyclic gp. |

When Compound [1], [II] or [III] contains an acidic group such as a carboxyl group or tetrazolyl group, or a basic group such as an amino group, a mono-or di-alkylamino group or pyridyl, Compound [I], [II] or [III] may form a salt with the acidic group or basic group. Such salts include, for example, pharmaceutically acceptable salts such as a salt with an inorganic base, a salt with an organic base, a salt with an organic acid, a salt with a basic or acid amino acid. As the salt with an inorganic base, use is made of, for example, an alkali metal salt (e.g.,a sodium salt or potassium salt), an alkaline earth metal salt (e.g.,a calcium salt or magnesium salt), an aluminum salt or an ammonium salt. As the salt with an organic base, use is made of salts with, for example, trimethylamine, triethylamine, pyridine, picoline, ethanolamine, diethanolamine, triethanolamine, dicyclohexylamine and N,N'-dibenzylethylenediamine. As the salt with an inorganic acid, use is made of salts with, for example, hydrochloric acid, hydrobromic acid, nitric acid sulfuric acid and phosphoric acid. As the salt with an organic acid, use is made of salts with, for example, formic acid, acetic acid, trifluoroacetic acid, fumaric acid, oxalic acid, tartaric acid, maleic acid, citric acid, succinic acid, malic acid, methanesulfonic acid, benzenesulfonic acid and p-toluenesulfonic acid. As the salt with a basic amino acid, use is made of salts with, for example, arginine, lysine and ornithine. As the salt with a basic or acid amino acid, use is made of, for example, aspartic acid and glutamic acid. Preferable examples of such salts include sodium salts, potassium salts, ammonium salts, hydrochlorides, acetates, oxalates and citrates, more preferably, for example, sodium salts, potassium salts and hydrochlorides. When the object compound is in the free state, it may optionally be converted into a corresponding salt in accordance with a conventional method, and, when the object compound is a salt, it may optionally be converted, in accordance with a conventional method, into the free compound or any other salt.

Preferable examples of Compound (I) include
2-t-butyl-4-chloro-5-{4-(2-pyridyl)benzylthio}-3(2H)-pyridazinone,
2-t-butyl-4-chloro-5-{4-(2-pyridyl)benzyloxy}-3(2H)-pyridazinone,
2-t-butyl-4-chloro-5-(2-hydroxy-1-phenyl)ethylthio-3(2H)pyridazinone,
2-t-butyl-4-chloro-5-(4-t-butyl)benzylthio-3(2H)-pyridazinone,
2-t-butyl-4-chloro-5-(4-phenyl)benzylthio-3(2H)-pyridazinone,
2-t-butyl-4-chloro-5-(2-naphthyl)methylthio-3(2H)-pyridazinone,
2-t-butyl-4-chloro-5-(2-quinolyl)methylthio-3(2H)-pyridazinone,
2-t-butyl-4-chloro-5-{4-(2-cyanophenyl)}benzylthio-3(2H)pyridazinone,
2-t-butyl-4-chloro-5-(4-trifluoromethyl)benzylthio-3(2H)pyridazinone,
2-t-butyl-4-chloro-5-(4-phenyl)benzyloxy-3(2H)-pyridazinone,
2-t-butyl-5-(4-t-butyl)benzylthio-4-methyl-3(2H)-pyridazinone,
2-t-butyl-5-(4-t-butyl)benzyloxy-4-methyl-3(2H)-pyridazinone,
2-t-butyl-4-chloro-5-benzylthio-3(2H)-pyridazinone,
2-t-butyl-4-chloro-5-{(3-phenyl)-2-propenylthio}-3(2H)-pyridazinone,
2-t-butyl-4-chloro-5-(4-styryl)benzylthio-3(2H)-pyridazinone,
2-t-butyl-4-chloro-5-(4-cyano)benzylthio-3(2H)-pyridazinone,
2-t-butyl-4-chloro-5-(4-t-butyl)benzyloxy-3(2H)-pyridazinone,
2-t-butyl-4-chloro-5-(2-naphthyl)methyloxy-3(2H)-pyridazinone,
2-t-butyl-5-(4-t-butyl)benzylthio-4-methoxy-3(2H)-pyridazinone,
2-tert-Butyl-4-chloro-5-[4-(1,2,4-thiadiazol-5-yl)benzylthio]-3(2H)-pyridazinone,
2-tert-Butyl-4-chloro-5-[4-(4-fluorobenzoyl)benzylthio]-3(2H)-pyridazinone,
2-tert-Butyl-4-chloro-5-[4-(4-fluorobenzoyl)benzyloxy]-3(2H)-pyridazinone,
2-tert-Butyl-4-chloro-5-[3-(4-chlorobenzoyl)benzylthio]-3(2H)-pyridazinone,
2-tert-Butyl-4-chloro-5-[3-(4-chlorobenzoyl)benzyloxy]-3(2H)-pyridazinone,
2-tert-Butyl-4-chloro-5-[4-(1,2,4-thiadiazol-5-yl)benzyloxy]-3(2H)-pyridazinone,
2-tert-Butyl-4-chloro-5-[4-(1,2,3-thiadiazol-4-yl)benzylthio]-3(2H)-pyridazinone,
2-tert-Butyl-4-chloro-5-[4-(1,2,3-thiadiazol-4-yl)benzyloxy]-3(2H)-pyridazinone,
2-tert-Butyl-4-chloro-5-[4-(1H-pyrazol-3-yl)benzylthio]-3(2H)-pyridazinone,
2-tert-Butyl-4-chloro-5-[4-(1H-pyrazol-3-yl)benzyloxy]-3(2H)-pyridazinone,
2-tert-Butyl-4-chloro-5-[4-(1,2-oxazol-5-yl)benzylthio]-3(2H)-pyridazinone,
2-tert-Butyl-4-chloro-5-[4-(1,2-oxazol-5-yl)benzyloxy]-3(2H)-pyridazinone,
2-tert-Butyl-4-chloro-5-[4-(1,3-oxazol-5-yl)benzylthio]-3(2H)-pyridazinone,
2-tert-Butyl-4-chloro-5-[4-(1,3-oxazol-5-yl)benzyloxy]-3(2H)-pyridazinone,
5-[4-[4-(2-Amino)pyridyl]benzyloxy]-2-tert-butyl-4-chloro-3(2H)-pyridazinone,
5-[4-[4-(2-Amino)pyridyl]benzylthio]-2-tert-butyl-4-chloro-3(2H)-pyridazinone,
2-tert-Butyl-4-chloro-5-[4-(2-pyrimidyl)benzylthio]-3(2H)-pyridazinone,
2-tert-Butyl-4-chloro-5-[4-(2-pyrimidyl)benzyloxy]-3(2H)-pyridazinone,

5-[4-[4-(2-Amino)thiazolyl]benzylthio]-2-tert-butyl-4-chloro-3(2H)-pyridazinone,
5-[4-[4-(2-Amino)thiazolyl]benzyloxy]-2-tert-butyl-4-chloro-3(2H)-pyridazinone,
2-tert-Butyl-4-chloro-5-[4-(2-pyridyl)benzylthio]-3(2H)-pyridazinone• Hydrochloride
and
2-tert-Butyl-4-chloro-5-[4-(2-pyridyl)benzyloxy]-3(2H)-pyridazinone• Hydrochloride

Preferable examples of Compound [II] include
2-t-butyl-4-chloro-5-{4-(2-pyridyl)benzylthio}-3(2H)-pyridazinone,
2-t-butyl-4-chloro-5-{4-(2-pyridyl)benzyloxy}-3(2H)-pyridazinone,
2-tert-Butyl-4-chloro-5-[4-(1,2,4-thiadiazol-5-yl)benzylthio]-3(2H)-pyridazinone,
2-tert-Butyl-4-chloro-5-[4-(1,2,4-thiadiazol-5-yl)benzyloxy]-3(2H)-pyridazinone,
2-tert-Butyl-4-chloro-5-[4-(1,2,3-thiadiazol-4-yl)benzylthio]-3(2H)-pyridazinone,
2-tert-Butyl-4-chloro-5-[4-(1,2,3-thiadiazol-4-yl)benzyloxy]-3(2H)-pyridazinone,
2-tert-Butyl-4-chloro-5-[4-(1H-pyrazol-3-yl)benzylthio]-3(2H)-pyridazinone,
2-tert-Butyl-4-chloro-5-[4-(1H-pyrazol-3-yl)benzyloxy]-3(2H)-pyridazinone,
2-tert-Butyl-4-chloro-5-[4-(1,2-oxazol-5-yl)benzylthio]-3(2H)-pyridazinone,
2-tert-Butyl-4-chloro-5-[4-(1,2-oxazol-5-yl)benzyloxy]-3(2H)-pyridazinone,
2-tert-Butyl-4-chloro-5-[4-(1,3-oxazol-5-yl)benzylthio]-3(2H)-pyridazinone,
2-tert-Butyl-4-chloro-5-[4-(1,3-oxazol-5-yl)benzyloxy]-3(2H)-pyridazinone,
5-[4-[4-(2-Amino)pyridyl]benzyloxy]-2-tert-butyl-4-chloro-3(2H)-pyridazinone,
5-[4-[4-(2-Amino)pyridyl]benzylthio]-2-tert-butyl-4-chloro-3(2H)-pyridazinone,
2-tert-Butyl-4-chloro-5-[4-(2-pyrimidyl)benzylthio]-3(2H)-pyridazinone,
2-tert-Butyl-4-chloro-5-[4-(2-pyrimidyl)benzyloxy]-3(2H)-pyridazinone,
5-[4-[4-(2-Amino)thiazolyl]benzylthio]-2-tert-butyl-4-chloro-3(2H)-pyridazinone,
5-[4-[4-(2-Amino)thiazolyl]benzyloxy]-2-tert-butyl-4-chloro-3(2H)-pyridazinone,
2-tert-Butyl-4-chloro-5-[4-(2-pyridyl)benzylthio]-3(2H)-pyridazinone• Hydrochloride
and
2-tert-Butyl-4-chloro-5-[4-(2-pyridyl)benzyloxy]-3(2H)-pyridazinone• Hydrochloride

Preferable examples of Compound [III] include
2-t-butyl-4-chloro-5-(2-hydroxy-2-phenyl)ethoxy-3(2H)-pyridazinone,
2-t-butyl-4-chloro-5-(2-hydroxy-1-phenyl)ethoxy-3(2H)-pyridazinone and
2-t-butyl-4-chloro-5-(2-hydroxy-1-phenyl)ethylthio-3(2H)pyridazinone.

Compounds [I], [II] and [III], when containing asymmetric carbons in their structures, may be either a (+) isomer or a (-) isomer of the optical isomers, or a mixture of these isomers at any optical ratio.

Known compounds among the 4,5-disubsutituted 3(2H)-pyridazinone, the substituent at the 5-position being $-X-Y^0-Q^0$ wherein X,$Y^0$ and $Q^0$ are of the same meaning as defined above, or its salt, can be produced in accordance with the method disclosed in, for example, JPA S60(1985)-4183, the disclosure of which is incorporated by reference herein, or with methods analogus thereto.

In addition the 4,5-disubsutituted 3(2H)-pyridazinone, the substituent at the 5-position being $-X-Y^0-Q^0$ or its salt, can be produced by reacting a 4,5-disubstituted 3(2H)-pyridazinone, the substituent at the 5-position being -X-H with a compound of the formula:$E-Y^0-Q^0$ wherein X,$Y^0$ and $Q^0$ are of the same meaning as defined above and E is a leaving group, in accordance with the same method for producing compound [I] shown hereinafter.

Similarly, the 4,5-disubsutituted 3(2H)-pyridazinone wherein the substituent at the 5-position is

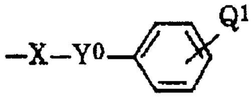

or its salt, can be produced by reacting a 4,5-disubstituted 3(2H)-pyridazinone wherein the substituent at the 5-position is -X-H with a compound of the formula:

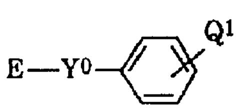

wherein X, $Y^0$, $Q^0$ and E are of the same meaning as defined above and the 4,5-disubsutituted 3(2H)-pyridazinone wherein the substituent at the 5-position is $-X-Y^1-Q^0$

or its salt, can be produced by reacting a 4,5-disubstituted 3(2H)-pyridazinone wherein the substituent at the 5-position is -X-H with a compound of the formula:$E-Y^1-Q^0$

wherein X, $Y^1$, $Q^0$ and E are of the same meaning as defined above.

In addition, Compound [I] including novel Compounds [II] and [III] can be produced by, for example, a method shown by the following reaction formula (1) or (2).

Reaction formula (1)

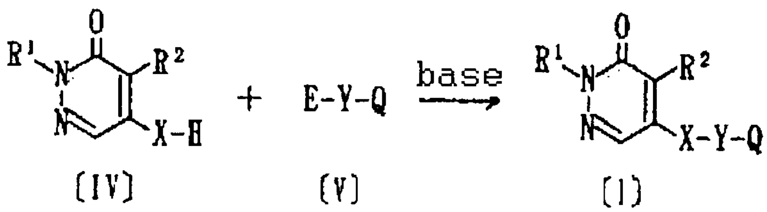


Reaction formula (2)

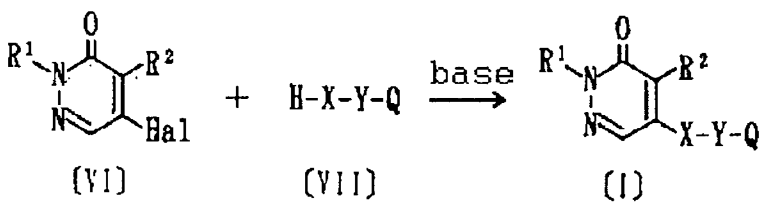


$R^1$, $R^2$, X, Y and Q in the compounds [IV] to [VII] in the above reaction formulae are of the same meaning as defined above, E is a leaving group and Hal is halogen.

As the leaving group shown by E, use is made of

(i) halogen atoms such as chlorine, bromine and fluorine,

(ii) $C_{1-6}$ alkylsulfonyloxy groups, which may optionally be substituted with, for example, 1 to 3 halogen atoms such as chlorine, bromine and fluorine, e.g.,methanesulfonyloxy, ethanesulfonyloxy, butanesulfonyloxy and trifluoromethanesulfonyloxy, and

(iii) $C_{6-10}$ arylsulfonyloxy groups, which may optionally be substituted with 1 to 3 halogen atoms such as chlorine, bromine and fluorine or $C_{1-3}$ alkyl groups such as methyl and ethyl, e.g.,benzenesulfonyloxy, p-toluenesulfonyloxy, p-bromobenzenesulfonyloxy and mesitylenesulfonyloxy.

As the halogen shown by Hal, use is made of, for example, fluorine, chlorine, bromine and iodine.

These reactions are described in more detail. In the reaction (1) or (2), the compound [IV] is allowed to react with [V], or [VI] is allowed to react with [VII] in the presence of a base, respectively to yield the object compound [I].

In these reactions, while relative amounts of the starting compounds [IV] to [VII] vary with the kinds of starting compounds or reaction conditions employed, the reactions are usually conducted by employing, relative to 1 mol. of [IV], 0.5 to 10 mol., preferably 0.75 to 1.5 mol. of [VI], and, relative to 1 mol. of [VI], 0.5 to 10 mol., preferably 0.75 to 1.5 mol. of [VII].

As the base, use is made of an inorganic base or an organic base. As the inorganic base, use is made of, for example, alkali metal hydroxide (e.g.,sodium hydroxide and potassium hydroxide), alkali metal carbonate (e.g.,sodium carbonate, potassium carbonate or cesium carbonate), alkali metal hydride (e.g.,sodium hydride) and alkali metal (e.g., metallic sodium). As the organic base, use is made of alkali metal alkoxide (e.g.,sodium methoxide, sodium ethoxide), trialkylamines (e.g.,triethylamine, tributylamine or diispropylethylamine) and aromatic amines (e.g.,pyridine, lutidine or DBU(1-8-diazabicyclo[5,4,0]-7-undecene). The amount of the base ranges usually from equimol. to about 10 times as much, preferably equimol. to a little excess, relative to the compound [IV] or [VII].

When the reaction system is of two layers, e.g.,solid-liquid or liquid-liquid, the reaction can be accelerated by adding to the reaction system a phase-transfer catalyst such as a quaternary ammonium salt (e.g.,triethylbenzylammonium chloride, trioctylmethylammonium chloride, trimethyldecylammonium chloride or tetramethylammonium bromide).

The reaction (1) and (2) can be allowed to proceed advantageously when conducted in a solvent. Examples of the solvent include lower alcohols (e.g.,methanol, ethanol, propanol and isopropanol), hydrocarbons (e.g.,pentane, hexane, cyclohexane and benzene), ethers (e.g.,diethyl ether, tetrahydrofuran and dioxane), amides (e.g.,N,N-dimethylformamide or hexamethylphosphoric acid triamide), halogenated hydrocarbons (e.g.,dichloromethane or chloroform), sulfoxides (e.g.,dimethylsulfoxide) and ureas (e.g.,1,3-dimethyl-3,4,5,6-tetrahydro-2(1H)-pyrimidinone). These solvents can be used singly or, upon necessity, as a mixture of two or more of them in an appropriate ratio, or as a mixture with water. The volume of these solvents ranges, in the reaction (1), from 0.1 to 100 ml, preferably 5 to 20 ml, relative to 1 g of the compound (IV), and, in the reaction (2), from 0.1 to 100 ml, preferably 5 to 20 ml, relative to 1 g of the compound (VI).

The reaction temperature ranges usually from -20 to 120 °C, preferably from -5 to 100 °C.

The reaction time ranges usually from 10 minutes to 72 hours, preferably from 10 minutes to 24 hours.

Among the starting compounds in the above-mentioned reactions (1) and (2), as the compounds [IV] and [VI], commercially available ones are utilized, or the compounds [IV] and [VI] are produced by methods known to those skilled in the art such as disclosed in JPA S61(1986)-17570, JPA S61(1986)-243078 and Chapters 2 and 3 of "Pyridazines" compiled by Raymondo N Castle, published by John Willey & Sons Inc. in 1973, or analogous methods thereto. As the compounds [V] and [VII], commercially available ones are utilized, or the compounds [V] and [VII] are produced by methods known to one of ordinary skill in the art such as disclosed in Chapters 4, 6, 18 and 21 of "Organic Functional Group Preparations I 2nd. ed." authored by Stanley R Sandler and Wolf Karo, published by Academic Press in 1983 or analogous methods thereto.

And, among the compounds [I], those wherein $R^2$ is a lower alkoxy group or a lower alkylthio group can be produced by employing, as the starting material, the compound [VIII], which is the compound [I] wherein $R^2$ is halogen, in accordance with, the method shown by the following reaction formula (3). Such compounds may also be produced, for examples by methods analogous to the method disclosed in JPA S63(1988)-159372, the disclosure of which is incorporated by reference herein.

Reaction formula (3)

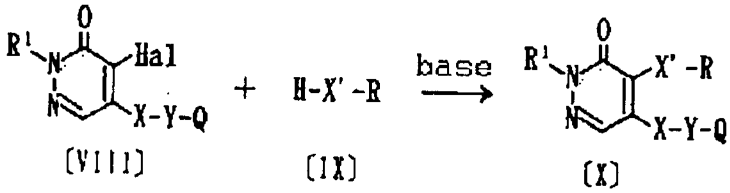


In the compounds [VIII] to [X], $R^1$, X, Y, Q and Hal are of the same meaning as defined above, X' is O or S and R is an optionally substituted lower alkyl group. As the lower alkyl group shown by R, use is made of, for example, $C_{1-6}$ straight chain or branched alkyl groups including methyl, ethyl, propyl, isopropyl, butyl, isobutyl, pentyl and hexyl. These lower alkyl groups may optionally be substituted with 1 to 3, preferably one group, independently selected from hydroxyl group, a halogen (e.g.,fluorine, chlorine, bromine and iodine), an amino group and a mono- or di-lower alkylamino group (e.g.,mono or di- $C_{1-6}$ alkyl amino group such as methylamino, ethylamino, propylamino and dimethylamino), and, as these substituents, the hydyoxyl group and amino group for example are more preferable.

The reaction (3) will be explained in detail. By allowing the compound [VIII] to react with the compound [IX] in the presence of a base, the object compound [X] can be produced. Incidentally, the compound [IX] may optionally be converted into the form of alcoholate or thiolate by treating, in advance, the compound [IX] with a base.

While relative amounts of the starting compounds [VIII] and [IX] employed in these reactions vary with the kinds of starting compounds or reaction conditions, generally 0.5 to 10 mol., preferably 0.5 to 10 mol., of [IX] is allowed to react with 1 mol. of [VIII]. As the base, use is made of inorganic bases or organic bases. Examples of inorganic bases include alkali metal hydroxides (e.g., sodium hydroxide or potassium

hydroxide), alkali metal carbonates (e.g., sodium carbonate, potassium carbonate or cesium hydroxide), alkali metal hydrogencarbonates (e.g., sodium hydrogencarbonate or potassium hydrogencarbonate), alkali metal hydrides (e.g., sodium hydride) and alkali metal (e.g., metallic sodium). As organic bases, use is made of, for example, alkoxides of of an alkali metal (e.g., sodium methoxide and sodium ethoxide), trialkylamines (e.g., triethylamine, tributylamine or diisopropylethylamine) and aromatic amines ( e.g., pyridine, lutidine or DBU(1-8-diazabicyclo[5,4,0]-7-undecene).

The amount of the base ranges usually from equimolar amount to about ten times as much, relative to the compound [VIII], preferably an equimolar amount or a little excess amount (e.g., 1.05 to 1.5 times as much mol.).

When the reaction system is of two layers, e.g.,solid-liquid or liquid-liquid, the reaction can be accelerated by adding to the reaction system a phase-transfer catalyst such as quaternary ammonium salt (e.g.,triethylbenzylammonium chloride, trioctylmethylammonium chloride, trimethyldecylammonium chloride or tetramethylammonium bromide).

The above reaction can be conducted advantageously in a solvent. Examples of the solvent include lower alcohols (e.g.,methanol, ethanol, propanol or isopropanol), hydrocarbons (e.g.,pentane, hexane, cyclohexane and benzene), ethers (e.g.,diethyl ether, tetrahydrofuran or dioxane), amides (e.g.,N,N-dimethylformamide or hexamethyl phosphoric acid triamide), halogenated hydrocarbons (e.g.,dichloromethane or chloroform), sulfoxides (e.g.,dimethylsulfoxide), ureas (e.g.,1,3-dimethyl-3,4,5,6-tetrahydro-2(1H)-pyrimidinone). These solvents can be used singly, and, upon necessity, as a mixture of two or more of them at an appropriate ratio, or as a mixture with water. The volume of these solvents to be used ranges usually from 0.1 to 100 ml, preferably from 5 to 20 ml, relative to 1 g of the compound [VIII]. The reaction temperature ranges usually from -20 °C to 120 °C, preferably from -5 °C to 100 °C.

The reaction time varies with volume and kinds of the starting compound, the base and the solvent employed and the reaction temperature. Usually, it ranges from 10 minutes to 72 hours, preferably from 10 minutes to 24 hours.

Among the starting compounds in the above reaction (3), the compound [VIII] can be produced by, for example, the methods shown by the above-mentioned reaction formulae (1) to (2), and, as the compound [IX], commercially available ones are utilized, or the compound [IX] is produced by methods known to there of ordinary skill in the art such as disclosed in, for example, Chapter 4 and Chapter 18 of "Organic Functional Group Preparations I, 2nd. ed." authored by Stanley R. Sandler and Wolf Karo, published by Academic Press (1983).

The compound [XIII], which is a compound [I] wherein $R^2$ is an optionally substituted aliphatic hydrocarbon group, can also be produced by, for example, the method shown by the reaction formula (4) employing the above-mentioned compound [VIII] as the starting material.

Reaction formula (4)

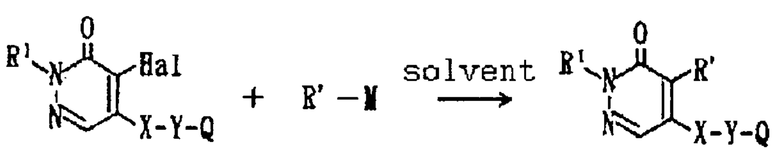


[VIII] [XI] [XII]

In the compounds [VIII], [XI] and [XII] of the above-mentioned reaction formula, $R^1$, X, Y, Q and Hal are of the same meaning as defined above, R' is an optionally substituted aliphatic hydrocarbon group, and M is, for example, magnesium halide, lithium and copper. As the optionally substituted aliphatic hydrocarbon group shown by R', use is made of substantially the same as the above-mentioned optionally substituted aliphatic hydrocarbon shown by $R^2$.

The reaction formula (4) will be explained in detail, i.e., by allowing an organometallic compound [XI] to react with the compound [VIII] in a solvent, the object compound [XII] can be produced.

As the solvent, use is made of, for example, hydrocarbons (e.g., pentane, hexane, cyclohexane or benzene), ethers (e.g., diethyl ether, tetrahydrofuran or dioxane), amides (e.g., N,N-dimethylformamide or hexamethyl phosphoric acid triamide) and ureas (e.g., 1,3-dimethyl-3,4,5,6-tetrahydro-2(1H)-pyrimidinone). These solvents can be used singly, or, upon necessity, as a mixture of two or more of them in an appropriate ratio. The volume of the solvent to be employed ranges usually from 0.1 to 100 ml, preferably

from 5 to 20 ml, relative to 1 g of the compound [VIII].

As the organometallic compound [XI], use is made of, for example, a Grignard reagent, an organolithium reagent or an organocopper reagent, which are produced by the method described in, for example, "Yuki Gosei VI" "Shin Jikken Kagaku Koza 4th ed. Vol.24" published by Maruzen Co., Ltd. (1992), or analogous methods thereto. The amount of the compound [X] to be used ranges usually from an equivalent amount to 10 times the amount, preferably from an equivalent amount to twice as much, relative to 1 mol. of the compound [VIII]. The reaction may advantageously be conducted by adding to the reaction system a metal salt (e.g., zinc chloride, cerium chloride, tin chloride, copper bromide, copper iodide, nickel chloride - phosphor complex or dilithium tetrachlorocuprate), in an amount ranging usually from 0.1 to 10 times mol., preferably 0.1 to twice as much, relative to 1 mol. of [XI].

The reaction temperature ranges usually from -78°C to the boiling point of the solvent employed, preferably from -5°C to 30°C. While the reaction time varies with the amount and kinds of the starting compounds, bases and solvents, and with reaction temperatures, it ranges usually from 10 minutes to 72 hours, preferably from 10 minutes to 24 hours.

The compound [I], wherein $R^2$ is H, can be produced, employing the compound [VIII], by conducting a reduction in accordance with the method described in J. Pharm. Soc. Jpn. Vol. 98, p.1472 (1978), authored by Masahiro TAKATANI, or with methods analogous thereto.

In addition, the above-mentioned compounds [IV], [V], [VI], [VII], [VIII], [IX] and [XII], when they contain an acid group or a basic group, can be used as the corresponding salts.

In the case where the object compound is produced by any of the methods shown by the aforementioned reaction formulae (1) to (4), when $R^1$, Y or Q in the compounds [IV] to [XII] contain functional groups such as a hydroxyl group, an amino group, a mono- $C_{1-6}$ alkylamino group, a carboxyl group or a tetrazolyl group, these functional groups may optionally be protected. Selection of the kinds of protective groups, and methods of protecting or deprotecting are conducted in accordance with the methods described in "Protective Groups in Organic Synthesis, 2nd. ed." authored by T. W. Green and P. G. M. Wuts, published by John Wiley & Sons, Inc. (1991), or by methods analogous thereto. While the starting materials and intermediates can be isolated from the reaction mixtures for synthesizing them, they may optionally be fed to the subsequent reaction as they are.

Thus-produced compound [I] or salts thereof can be isolated and purified by any conventional means (e.g.,phasic transfer, concentration, solvent-extraction, fractional distillation, crystallization, recrystallization or chromatography and combinations thereof).

The antitumor agent of this invention can be obtained by producing an antitumor agent incorporated with the compound [I] or a salt thereof.

While content of the compound [I] or a salt thereof in the pharmaceutical preparation of this invention varies with the form of the preparation, it usually ranges from 0.1 to 100 weight %, preferably 1 to 50 weight %, more preferably 5 to 20 weight %, relative to the total weight of the preparation.

To the pharmaceutical preparation of the present invention can be added additives employed generally in the manufacture of pharmaceutical preparations. More specifically, conventional carriers for pharmaceutical preparations, for example, excipients (e.g., calcium carbonate, kaolin, sodium hydrogencarbonate, lactic acid, starches, crystalline cellulose, talc, fine granulated sugar and porous substances), binders (e.g.,dextrin, rubbers, $\alpha$-starch, gelatin, hydroxypropylcellulose, hydroxypropylmethylcellulose and pulluran), disintegrators (e.g.,carboxymethylcellulose calcium, cross carmellose sodium, cross povidone, low-substituted hydroxypropylcellulose and partial $\alpha$-starch), lubricants (e.g.,magnesium stearate, calcium stearate, talc, starch and sodium benzoate), colorants (e.g.,tar pigment, caramel, iron [III] oxide, titanium oxide and riboflavin), flavoring agents (e.g.,sweeteners and perfume), stabilizers (e.g.,sodium sulfite) and/or preserving agents (e.g, parabens and sorbic acid) can be added in accordance with conventional methods when manufacturing the pharmaceutical preparations.

Carriers for orally administrable preparations are exemplified by starch, mannitol, crystalline cellulose and sodium carboxymethyl cellulose, and, carriers for injectable preparations are exemplified by distilled water, physiological saline, glucose solution and infusion solution.

While the amount of these carriers for pharmaceutical preparations varies with, for example, the effective component and the amount thereof then employed, it usually ranges from 0.01 to 100 weight %, preferably 0.5 to 50 weight %, more preferably 0.1 to 10 weight % relative to 1 weight of the compound [I] or a salt thereof.

The forms of pharmaceutical preparations are exemplified by tablets (including e.g.,sugar-coated tablets and film-coated tablets), pills, capsules, granules, fine granules, powdery products, syrups, emulsions, suspensions and injections. Preferable forms include, for example, injections and tablets.

The antitumor agent of this invention can be prepared by, any of the known methods for manufacturing pharmaceutical preparations (e.g., methods described in the Japanese Pharmacopeia). For example, tablets can be prepared by subjecting the compound [I] or a salt thereof to granulation as it is or after mixing homogeneously with an excipient, a binder, a disintegrant or any other suitable additive, to which are added a lubricant etc., followed by subjecting the mixture to compression molding, or, subjecting the compound [I] or a salt thereof to compression molding directly as it is or after mixing with an excipient, a binder, disintegrant or any other suitable additive, or, subjecting the granules manufactured in advance to compression molding as they are or after mixing homogeneously with a suitable additive. A colorant, a flavoring agent or the like may optionally be added as desired. Further, the tablets thus prepared may optionally be subjected to a suitable coating. Injectable solutions, for example, can be prepared by dissolving, suspending or emulsifying a given amount of the compound [I] or a salt thereof in an aqueous solvent, e.g., injectable water, physiological saline or Ringer's solution, or in a non-aqueous solution, e.g., vegetable oil, or hermetically sealing a given amount of the compound [I] or a salt thereof in a vessel for injection.

The antitumor agent of this invention thus obtained has an excellent antitumor activity, especially an excellent selective growth-inhibiting activity against pancreatic carcinoma, prostatic cancer , lung cancer and breast cancer cells. Since undesirable side effects and toxicity of this agent are very low, no mice were killed by intraperioneal injection of this agent in an amount of, for example, 20 mg/kg. Therefore, the antitumor agent of this invention can be used safely for the therapy of malignant tumors of mammals (e.g., man, mice, rats, hamsters, rabbits, dogs, cats, cows, pigs, horses, sheep and monkeys), especially intractable solid tumors, for example, pancreatic carcinoma or prostatic cancer (especially, e.g., hormone independent tumors or recurrent tumors) and the therapy of metastatic lesions caused by remote metastatis of them to the lungs and liver, or the prophylaxis of remote metastatis of the tumors. The administration route may be either oral or non-oral.

While the administration amount of the antitumor agent of this invention varies with, for example, age, body weight, administration route and administration frequency, it ranges, for the therapy of prostatic cancer in adult humans, usually from 0.1 to 20 mg/kg, preferably 1 to 10 mg/kg per day in terms of the compound [I] or a salt thereof, and the amount mentioned above is preferably administered orally in 1 to 3 divided doses.

## Examples

The following reference, working and test examples are further descriptive of the present invention. It should be understood that these are merely illustrative and by no means definitive of the invention and that various changes and modifications can be made within the scope of the invention.

Elution by column chromatography of reference and working examples was conducted under observation with TLC (Thin Layer Chromatography). In the TLC observation, a TLC plate, 60F254 manufactured by Merck, was employed or, the developing solvent was used as the eluting solvent for the column chromatography and a UV detector was employed or a detecting means. A silica gel column, Kieselgel 60 (70 to 230 mesh) manufactured by Merck was employed. The NMR spectrum was measured with a Gemini 200 type spectrometer using tetramethyl silane as an internal or external standard, and all $\delta$ values were shown in ppm. In addition, % in solutions means gram numbers in 100 ml of the corresponding solution. And, "room temperature" in reference and working examples means the range of 20 to 30 °C, and, in the presentation of NMR data, the following abbreviations are used.

| | |
|---|---|
| s | : singlet |
| d | : doublet |
| t | : triplet |
| q | : quartet |
| ABq | : AB type quartet |
| d.d | : double doublet |
| m | : multiplet |
| bs | : broad singlet |
| J | : coupling constant |
| Hz | : Herz |
| DMSO | : dimethyl sulfoxide |

Reference Example 1

Production of 4-(2-pyridyl)benzyl bromide

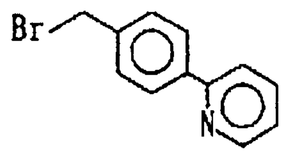

A mixture of p-tolyl pyridine (846 mg), N-bromosuccinimide (979 mg) and benzoyl peroxide (40 mg) was heated for 8.5 hours under reflux in carbon tetrachloride (80 ml). The reaction mixture was cooled to room temperature, then insolubles were filtered off. The filtrate was subjected to distillation under reduced pressure. The residue was purified by means of a silica gel column chromatography (carrier 40 g; developing solvent: hexane-ethyl acetate-9:1) to give 4-(2-pyridyl)benzyl bromide (1.06 g:yield 85%)
NMR($CDCl_3$) $\delta$ : 4.55(2H,s), 7.20-7.29(1H,m), 7.50(2H,d,J=8.2Hz), 7.65-7.79(2H,m), 7.98(2H,dm,J=8.2Hz), 8.65-8.72(1H,m).
IR(KBr): 3050,3000,1580,1540,1460,1400cm$^{-1}$.

Reference Example 2

Production of 5-(4-methylphenyl)tetrazole

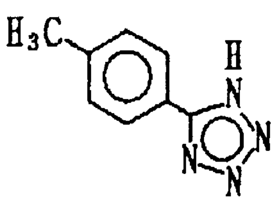

To a solution of p-tolunitrile (1.17 g) and trimethylsilyl azide (2.30 g) in toluene (20 ml) was added dibutyltin oxide (249 mg). The mixture was stirred for 70 hours at 90°C. The reaction mixture was cooled to room temperature, which was concentrated under reduced pressure. The concentrate was dissolved in methanol, which was again concentrated under reduced pressure. To the concentrate were added ethyl acetate and a 10% aqueous solution of sodium hydrogencarbonate, which was made to form two layers. The organic layer was subjected to extraction with a 10% aqueous solution of sodium hydrogen carbonate. To the aqueous layer was added 1N HCl to adjust the pH to 2, followed by extraction with ethyl acetate. The extract solution was dried over anhydrous magnesium sulfate, then the solvent was distilled off under reduced pressure to give 5-(4-methylphenyl)tetrazole (1.60 g:yield 100%).
NMR(DMSO-$d_6$) $\delta$: 2.39(3H,s), 7.41(2H,d,J=8.0Hz), 7.93(2H,d,J=8.0Hz).
MS(m/z): 160(M$^+$)

Reference Example 3

Production of N-(triphenylmethyl)-5-(4-methylphenyl)tetrazole

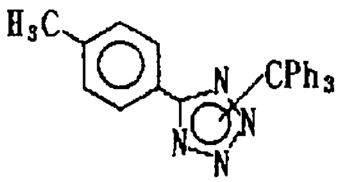

To a solution of 5-(4-methylphenyl)tetrazole (1.60 g) in DMF (20 ml) were added triethylamine (1.53 g) and triphenylmethyl chloride (3.07 g), which was stirred for 3 hours at room temperature. To the reaction mixture was added water, then the resulting precipitate was recovered by filtration. The precipitate was washed with a small volume of methanol and ethanol, successively, followed by drying over phosphorus pentoxide at 60 °C under reduced pressure to give N-(triphenylmethyl)-5-(4-methylphenyl)tetrazole (3.19 g:yield 79%).
NMR(DMSO-$d_6$) $\delta$: 2.37(3H,s), 7.07-7.12(6H,m), 7.33-7.44(1H,m), 7.91(2H,d,J = 8.2Hz).
IR(KBr): 3050,3040,1680,1615,1540cm$^{-1}$.

Reference Example 4

Production of N-(triphenylmethyl)-5-(4-bromomethylphenyl)tetrazole

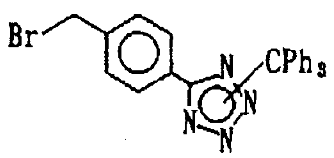

In substantially the same manner as Reference Example 1, from N-(triphenylmethyl)-5-(4-methylphenyl) tetrazole (3.00 g), was obtained N-(triphenylmethyl)-5-(4-bromomethylphenyl) tetrazole (2.62 g:yield 73%).
NMR(DMSO-$d_6$) $\delta$: 4.77(2H,s), 7.07-7.13(6H,m), 7.39-7.48(9H,m), 7.62(2H,d,J = 8.2Hz), 8.02(2H,d,J = 8.2Hz).
IR(KBr): 3050,3020,1590,1540,1490,1460,1440,1420cm$^{-1}$.

Reference Example 5

Production of 2-(t-butyldimethylsilyloxy)-1-phenylethanol

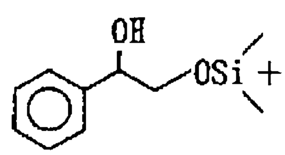

To a solution of 1-phenylethanediol (1.00 g) in dichloromethane (10 ml) were added, under ice-cooling, triethylamine (1.11 ml), t-butyldimethylchlorosilane (1.20 g) and dimethylaminopyridine (35.4 mg), and the mixture was stirred overnight. To the reaction mixture was added water. The resulting product was subjected to extraction with dichloromethane. The extract solution was washed with a saturated aqueous saline solution, which was dried over anhydrous magnesium sulfate, followed by distilling off the solvent under reduced pressure. The residue was purified by means of silica gel column chromatography (carrier

40 g: developing solvent: ether-hexane = 1:4) to give 2-(t-butyldimethylsilyloxy)-1-phenylethanol (1.82 g: yield 93%) as a colorless oily product.
NMR($CDCl_3$) δ: 0.06(3H,s), 0.07(3H,s), 0.91(9H,s), 2.96(1H,d,J = 2.0Hz), 3.54(1H,dd,J = 8.5Hz,11.0Hz), 3.77-(1H,dd,J = 3.5Hz,8.5Hz), 4.70-4.80(1H,m), 7.25(5H,m).

Reference Example 6

Production of 2-t-butyl-4-chloro-5-(4-t-butyl)benzylthio-3(2H)pyridanzine (Compound 1)

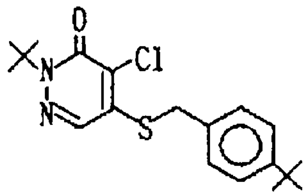

The method described in JPA S61(1986)-17670 or a method analogous thereto was followed. To a solution of 2-t-butyl-4-chloro-5-mercapto-3(2H)pyridazinone (500 mg) in N,N-dimethylformamide (DMF:5ml) were added anhydrous sodium carbonate (242 mg) and 4-t-butylbenzyl chloride (418 mg). The mixture was stirred for 2 hours at 80 °C. The reaction mixture was cooled to room temperature. The resulting precipitate was recovered by filtration, which was washed with water, dried and recrystallized from ethanol to give Compound 1 (667 mg:yield 80%) as colorless flakes.
NMR($CDCl_3$) δ: 1.32(9H,s), 1.63(9H,s), 4.25(2H,s), 7.30-7.45(4H,m), 7.64(1H,s).

Reference Example 7

Production of 2-t-butyl-4-chloro-5-benzylthio-3 (2H)pyridazinone (Compound 2)

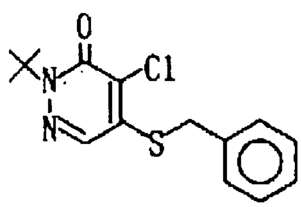

In substantially the same manner as Reference Example 6, Compound 2 (338 mg:yield 80%) was produced as colorless needles from 2-t-butyl-4-chloro-5-mercapto-3(2H)pyridazinone (300 mg), anhydrous sodium carbonate (218 mg) and benzyl bromide (235 mg).
NMR($CDCl_3$) δ: 1.63(9H,s), 4.27(2H,s), 7.25-7.45(5H,m), 7.62(1H,s).

Reference Example 8

Production of 2-t-butyl-4-chloro-5-(4-phenyl)benzylthio-3(2H)pyridazinone (Compound 3)

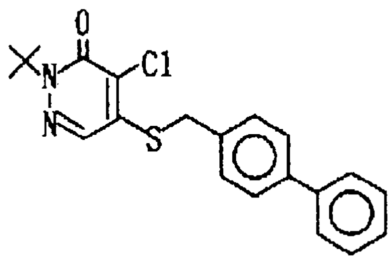

In substantially the same manner as Reference Example 6, Compound 3 (404 mg:yield 76%) was produced as colorless needles from 2-t-butyl-4-chloro-5-mercapto-3(2H) pyridazinone (300 mg), anhydrous sodium carbonate (145 mg) and 4-(chloromethyl)biphenyl (3o6 mg).
NMR($CDCl_3$) $\delta$: 1.63(9H,s), 4.32(2H,s), 7.30-7.65(5H,m), 7.65(1H,s).

Reference Example 9

Production of 2-t-butyl-4-chloro-5-(2-naphthyl)methylthio-3(2H)pyridazinone (Compound 4)

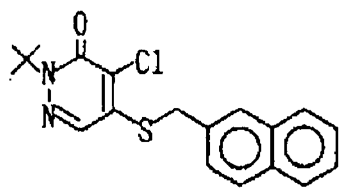

In substantially the same manner as Reference Example 6, Compound 4 (775 mg:yield 94%) was produced as colorless needles from 2-t-butyl-4-chloro-5-mercapto-3(2H) pyridazinone (500 mg), anhydrous sodium carbonate (242 mg) and 2-bromomethylnaphthalene (531 mg).
NMR($CDCl_3$) $\delta$: 1.61(9H,s), 4.43(2H,s), 7.45-7.55(3H,m), 7.64(1H,s), 7.80-7.90(4H,m)

Reference Example 10

Production of 2-t-butyl-4-chloro-5-(2-phenyl)ethylthio-3(2H)pyridazinone (Compound 5)

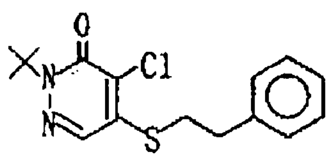

In substantially the same manner as Reference Example 6, 2-t-butyl-4-chloro-5-mercapto-3(2H)-pyridazinone (500 mg) was reacted with anhydrous sodium carbonate (242 mg) and 2-phenyl bromoethane (444 mg). The reaction mixture was purified by means of silica gel column chromatography (carrier 25 g; developing solvent: ether-hexane = 1:4) to give Compound 5 (737 mg:yield 99%) as a colorless oily product.
NMR($CDCl_3$) $\delta$: 1.64(9H,s), 4.32(2H,s), 7.30-7.65(5H,m), 7.65(1H,s)

Reference Example 11

Production of 2-t-butyl-4-chloro-5-{(3-phenyl)-2-propenylthio}-3(2H)pyridazinone (Compound 6)

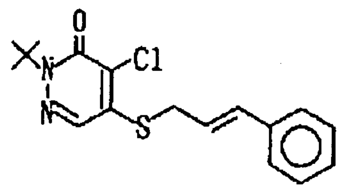

In substantially the same manner as Reference Example 6, from 2-t-butyl-4-chloro-5-mercapto-3(2H) pyridazinone (500 mg), anhydrous sodium carbonate (242 mg) and 3-chloro-1-phenylpropene (349 mg), Compound 6 (622 mg: yield 81%) was produced as colorless needles.
NMR($CDCl_3$) δ: 1.63(9H,s), 3.87(1H,dd,J=1.5Hz,7.0Hz),6.25(1H,dt,J=7.0Hz,16Hz), 6.64-(1H,dt,J=1.5Hz,16Hz), 7.25-7.40(5H,m), 7.68(1H,s).

Reference Example 12

Production of 2-t-Butyl-4-chloro-5-(1-naphthyl)methylthio-3(2H)pyridazinone (Compound 7)

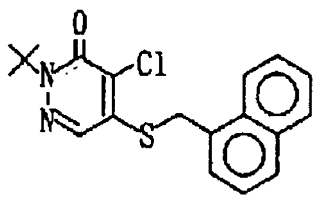

In substantially the same manner as Reference Example 6, from 2-t-butyl-4-chloro-5-mercapto-3(2H) pyridazinone (150 mg), anhydrous sodium carbonate (72 mg) and 1-chloromethyl naphthalene (121 mg), Compound 7 (169 mg:yield 69%) was produced as colorless needles.
NMR($CDCl_3$) δ: 1.65(9H,s), 4.73(2H,s), 7.40-7.55(4H,m), 7.71(1H,s), 7.80-7.95(2H,m), 8.07(1H,m)

Reference Example 13

Production of 2-t-butyl-4-chloro-5-(2-pyridyl)methylthio-3 (2H)pyridazinone (Compound 8)

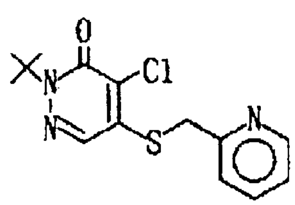

In substantially the same manner as Reference Example 6, 2-t-butyl-4-chloro-5-mercapto-3(2H)-pyridazinone (100 mg) was reacted with anhydrous sodium carbonate (97 mg) and 2-chloromethylpyridine.hydrochloride (108 mg). The reaction mixture was purified by means of silica gel chromatography (carrier 25 g; developing solvent: ether-hexane=1:1) to give Compound 8 (138 mg:yield 97%) as a colorless oily product.
NMR($CDCl_3$) δ: 1.62(9H,s), 4.39(2H,s), 7.24(1H,ddd,J=1.0Hz,5.0Hz,7.5Hz)), 7.46(1H,br.d,J=7.0Hz), 7.72-(1H,dt,J=2.0Hz,7.5Hz), 7.85(1H,s), 8.59(1H,m).

Reference Example 14

Production of 2-t-butyl-4-chloro-5-(3-pyridyl)methylthio-3(2H)pyridazinone (Compound 9)

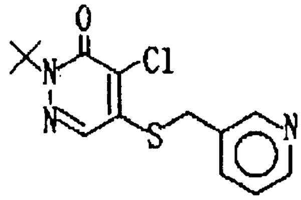

In substantially the same manner as Reference Example 6, 2-t-butyl-4-chloro-5-mercapto-3(2H)-pyridazinone (100 mg) was reacted with anhydrous sodium carbonate (97 mg) and 3-chloromethyl pyridine.hydrochloride (108 mg). The reaction mixture was purified by means of silica gel chromatography (carrier 25 g; developing solvent:ethyl acetate - hexane =2:1) to give Compound 9 (122 mg:yield 86%) as white powder.
NMR($CDCl_3$) δ: 1.63(9H,s), 4.28(2H,s), 7.33(1H,s), 7.77(1H,m), 7.58(1H,m), 7.66(1H,m)

Reference Example 15

Production of 2-t-butyl-4-chloro-5-(4-pyridyl)methylthio-3(2H)pyridazinone (Compound 10)

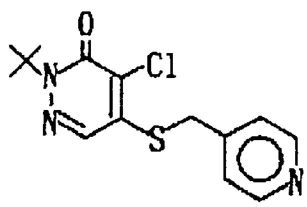

In substantially the same manner as Reference Example 6, 2-t-butyl-4-chloro-5-mercapto-3(2H)-pyridazinone (100 mg) was reacted with anhydrous sodium carbonate (97 mg) and 4-chloromethyl pyridine.hydrochloride (75 mg). The reaction mixture was purified by means of a silica gel column chromatography (carrier 25 g; developing solvent: ethyl acetate - hexane =3:2) to give Compound 10 (64 mg:yield 45%) as a white powdery product.
NMR($CDCl_3$) δ: 1.62(9H,s), 4.24(2H,s), 7.37(2H,br.d,J=6.0Hz), 7.49(1H,s), 8.63(1H,br.d,J=6.0Hz).

Reference Example 16

Production of 2-t-butyl-4-chloro-5-(benzimidazol-2-yl) methylthio-3(2H)pyridazinone (Compound 11)

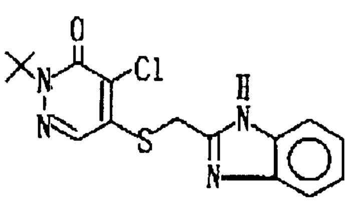

In substantially the same manner as Reference Example 6, from 2-t-butyl-4-chloro-5-mercapto-3(2H) pyridazinone (100 mg), anhydrous sodium carbonate (97 mg) and 2-chloromethyl benzimidazole (114 mg), Compound 11 (61 mg:yield 38%) was produced as colorless needles.
NMR(DMSO-$d_6$) δ: 1.58(9H,s), 4.78(2H,s), 7.10-7.25(2H,m), 7.45-7.65(2H,m), 8.30(1H,s).

Reference Example 17

Production of 2-t-butyl-4-chloro-5-(2-quinolyl)methylthio-3(2H)pyridazinone (Compound 12)

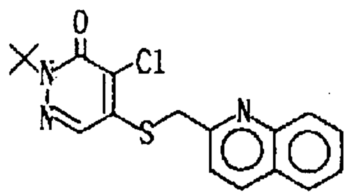

In substantially the same manner as Reference Example 6, from 2-t-butyl-4-chloro-5-mercapto-3(2H) pyridazinone (200 mg), anhydrous sodium carbonate (193 mg) and 2-chloromethylquinoline.hydrochloride (294 mg), Compound 12 (262 mg:yield 80%) was produced as pale yellow needles.
NMR($CDCl_3$) δ: 1.60(9H,s), 4.55(2H,s), 7.50-7.65(2H,m), 7.70-7.85(2H,m), 8.01(1H,s), 8.08(1H,br.d,J = 8.5Hz).

Reference Example 18

Production of 2-t-butyl-4-chloro-5-{4-(2-cyanophenyl)}benzylthio-3(2H)pyridazinone (Compound 13)

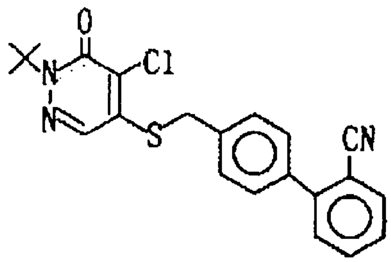

In substantially the same manner as Reference Example 6, from 2-t-butyl-4-chloro-5-mercapto-3(2H) pyridazinone (500 mg), anhydrous sodium carbonate (242 mg) and 2-(4-bromomethylphenyl)benzonitrile (622 mg), Compound 13 (710 mg:yield 63%) was produced as colorless needles.
NMR($CDCl_3$) δ: 1.64(9H,s), 4.34(2H,s), 7.45-7.85(8H,m), 7.66(1H,s).

Reference Example 19

Production of 2-t-butyl-4-chloro-5-(4-styryl)benzylthio-3 (2H)pyridazinone (Compound 14)

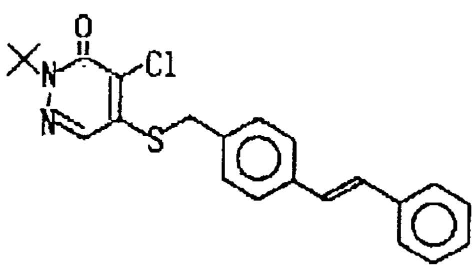

In substantially the same manner as Reference Example 6, from 2-t-butyl-4-chloro-5-mercapto-3(2H) pyridazinone (200 mg), anhydrous sodium carbonate (97 mg) and 4-chloromethylstilbene (209 mg), Compound 13 (232 mg:yield 62%) as colorless needles.
NMR($CDCl_3$) δ: 1.63(9H,s), 4.28(2H,s), 7.08(1H,d,J = 16Hz), 7.14(1H,d,J = 16Hz), 7.20-7.55(9H,m), 7.62(1H,s).

Reference Example 20

Production of 2-t-butyl-4-chloro-5-(3,5-dimethylisoxazol-4-yl)methylthio-3(2H)pyridazinone (Compound 15)

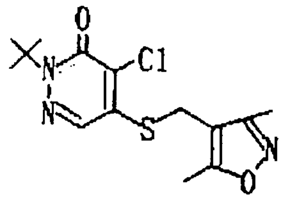

In substantially the same manner as Reference Example 6, from 2-t-butyl-4-chloro-5-mercapto-3(2H) pyridazinone (299 mg), anhydrous sodium carbonate (97 mg), and 4-chloromethyl-3,5-dimethylisoxazole (133 mg), Compound 15 (280 mg:yield 93%) was produced as colorless needles.
NMR($CDCl_3$) δ: 1.65(9H,s), 2.32(3H,s), 2.41(3H,s), 4.01(2H,s), 7.65(1H,s).

Reference Example 21

Production of 2-t-butyl-4-chloro-5-(4-cyano)benzylthio-3(2H)pyridazinone (Compound 16)

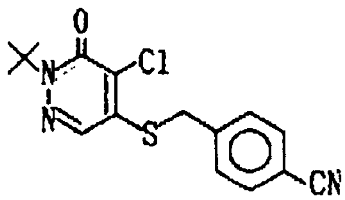

In substantially the same manner as Reference Example 6, from 2-t-butyl-4-chloro-5-mercapto-3(2H) pyridazinone (1.50 g), anhydrous sodium carbonate (0.73 g) and 4-bromomethylbenzonitrile (1.34 g), Compound 16 (2.14 g:yield 95%) was produced as colorless needles.
NMR($CDCl_3$) δ: 1.62(9H,s), 4.30(2H,s), 7.51(1H,s), 7.50-7.60(2H,m), 7.65-7.75(2H,m).

Reference Example 22

Production of 2-t-butyl-4-chloro-5-(4-trifluoromethyl) benzylthio-3(2H)pyridazinone (Compound 17)

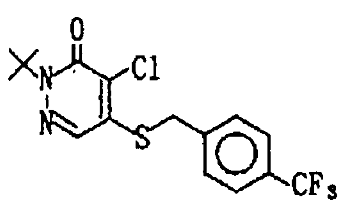

In substantially the same manner as Reference Example 6, from 2-t-butyl-4-chloro-5-mercapto-3(2H) pyridazinone (500 mg), anhydrous potassium carbonate (682 mg) and 4-trifluoromethylbenzyl bromide (656 mg), Compound 17 (547 g:yield 64%) was produced as pale yellow prisms.
NMR(DMSO-$d_6$) δ: 1.57(9H,s), 4.66(2H,s), 7.71(1H,s), 7.65(2H,m), 7.75-7.85(2H,m), 8.05(1H,s).

Reference Example 23

Production of 2-t-butyl-4-chloro-5-(4-phenyl)benzyloxy-3(2H)pyridazinone (Compound 18)

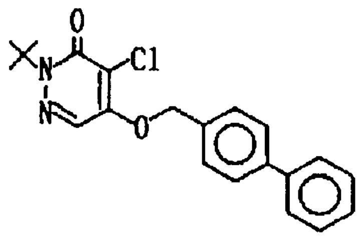

The method described in JPA 59(1984)-98064 or an analogous method thereto was followed. To a solution of 2-t-butyl-4-chloro-5-hydroxy-3(2H)pyridazinone (2.03 g) in N,N-dimethylformamide (DMF:20 ml) were added anhydrous potassium carbonate (2.10 g) and 4-(chloromethyl) biphenyl (2.03 g). The mixture was stirred for 1.5 hour at 100 °C. The reaction mixture was cooled to room temperature, to which was added water. The resulting precipitate was recovered by filtration, washed with water and dried, followed by recrystallization from ethanol to give Compound 18 (2.32 g:yield 63%) as colorless needles.
NMR(DMSO-$d_6$) $\delta$: 1.58(9H,s), 5.51(2H,s), 7.35-7.60(5H,m), 7.65-7.80(4H,m), 8.30(1H,s).

Reference Example 24

Production of 2-t-butyl-4-chloro-5-(4-t-butyl)benzyloxy-3(2H)pyridazinone (Compound 19)

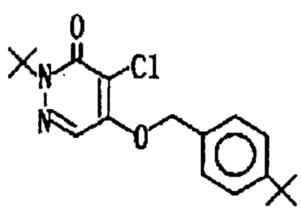

In substantially the same manner as Reference Example 23, from 2-t-butyl-4-chloro-5-hydroxy-3(2H)-pyridazinone (2.22 g), anhydrous sodium carbonate (1.74 g) and 4-t-butylbenzyl bromide (2.0 g), Compound 19 (2.94 g:yield 77%) was produced as colorless needles.
NMR($CDCl_3$) $\delta$: 1.33(9H,s), 1.64(9H,s), 5.28(2H,s), 7.30-7.40(2H,m), 7.40-7.50(2H,m), 7.74(1H,s).

Reference Example 25

Production of 2-t-butyl-4-chloro-5-(2-naphthyl)methyloxy-3(2H)pyridazinone (Compound 20)

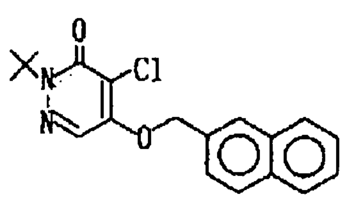

In substantially the same manner as Reference Example 23, from 2-t-butyl-4-chloro-5-hydroxy-3(2H) pyridazinone (1.00 g), anhydrous potassium carbonate (1.36 g) and 2-bromomethyl naphthalene (1.31 g), Compound 20 (1.08 g:yield 64%) was produced as colorless needles.
NMR(DMSO-$d_6$) $\delta$: 1.57(9H,s), 5.63(2H,s), 7.51(3H,m), 7.88-8.04(4H,m), 8.31(1H,s).

Reference Example 26

Production of 2-t-butyl-4-chloro-5-(1-naphthyl)methyloxy-3(2H)pyridazinone (Compound 21)

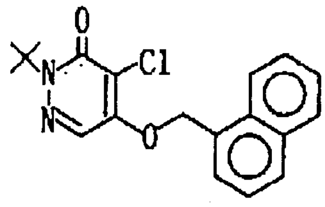

In substantially the same manner as Reference Example 23, from 2-t-butyl-4-chloro-5-hydroxy-3(2H) pyridazinone (500 mg), anhydrous potassium carbonate (682 mg) and 1-chloromethyl naphthalene (523 mg), Compound 21 (493 mg:yield 58%) was produced as colorless needles.
NMR($CDCl_3$) δ: 1.59(9H,s), 5.92(2H,s), 7.50-7.67(3H,m), 7.73(1H,d,J=7.0Hz), 7.95-8.04(2H,m), 8.47(2H,s), 8.47(1H,s).

Reference Example 27

Production of 2-t-butyl-4-chloro-5-benzyloxy-3(2H) pyridazinone (Compound 22)

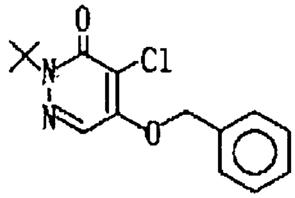

In substantially the same manner as Reference Example 23, from 2-t-butyl-4-chloro-5-hydroxy-3(2H) pyridazinone (500 mg), anhydrous potassium carbonate (682 mg) and benzylbromide (422 mg), Compound 22 (365 mg:yield 51%) was produced as colorless needles.
NMR($CDCl_3$) δ: 1.63(9H,s), 5.32(2H,s), 7.35-7.45(3H,m), 7.73(1H,s).

Reference Example 28

Production of 2-t-butyl-4-chloro-5-(4-trifluoromethyl) benzyloxy-3(2H)pyridazinone (Compound 23)

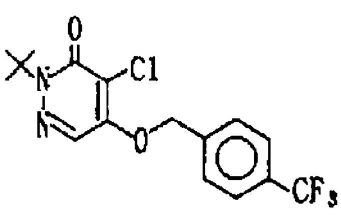

In substantially the same manner as Reference Example 23, from 2-t-butyl-4-chloro-5-mercapto-3(2H) pyridazinone (800 mg), anhydrous potassium carbonate (1.09 g) and 5-trifluoromethyl benzyl bromide (1.11 g), Compound 23 (726 mg:yield 52%) was produced as colorless prisms.
NMR($CDCl_3$) δ: 1.62(9H,s), 4.30(2H,s), 7.5(1H,s), 7.50-7.60(2H,m), 7.65-7.75(2H,m).

Reference Example 29

Production of 2-t-butyl-5-(4-t-butyl)benzylthio-3(2H) pyridazinone (Compound 24)

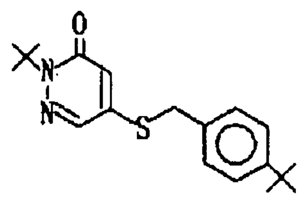

To a solution of 2-t-butyl-4-5-(4-t-butyl)benzylthio-3(2H)pyridazinone (Compound 1:500 mg) in methanol (100 ml) was added 10% palladium-carbon (500 mg). The mixture was stirred overnight under hydrogen atmosphere. From the reaction mixture, the catalyst was filtered off. From the filtrate, the solvent was distilled off under reduced pressure. The residue was purified by means of silica gel column chromatography (carrier: 50 g; developing solvent: ether-hexane = 1:4) to give Compound 24 (5.1 mg: yield 1%) as a white powdery product.
NMR($CDCl_3$) δ: 1.31(9H,s), 1.62(9H,s), 4.10(2H,s), 6.52(1H,,d,J = 2.5Hz), 7.25-7.40(4H,m), 7.49(1H,s).

Reference Example 30

Production of 2-t-butyl-5-(4-t-butyl)benzylthio-4-mercapto-3(2H)pyridazinone (Compound 25)

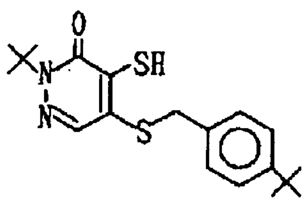

To a solution of 2-t-butyl-4-chloro-5-(4-t-butyl) benzylthio-3(2H)pyridazinone (Compound 1: 3.8 g) in ethanol (70 ml) were added water (2 ml) and 70% sodium hydrosulfide hydrate (2.8 g). The mixture was stirred for 1.5 hour under heating. The reaction mixture was cooled to room temperature, which was concentrated under reduced pressure. To the concentrate were added ethyl acetate and 1N HCl. The organic layer was separated, and the aqueous layer was further subjected to extraction with ethyl acetate. The organic layer and the extract solution were combined and washed with water, which was dried over anhydrous magnesium sulfate, followed by distilling off the solvent under reduced pressure. The residue was recrystallized from ethanol to give Compound 25 (3.31 g: yield 88%) as colorless needles.
NMR($CDCl_3$) δ: 1.31(9H,s), 1.63(9H,s), 4.22(2H,s), 7.25-7.40(4H,m), 7.56(1H,s).

Reference Example 31

Production of 2-t-butyl-5-(4-t-butyl)benzylthio-4-methylthio-3(2H)pyridazinone (Compound 26)

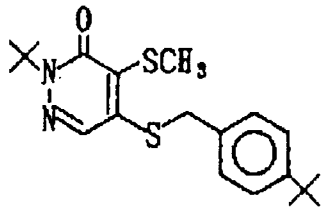

To a solution of 2-t-butyl-5-(4-t-butyl)benzylthio-4-mercapto-3(2H)pyridazinone (Compound 25: 300 mg) in DMF (3 ml), was added sodium hydride (60% oil: 36 mg) under ice-cooling. The mixture was stirred for 30 minutes, to which was further added methyl iodide (0.1 ml). The mixture was stirred for one hour, to which was added water to suspend the reaction. The reaction mixture was subjected to extraction with ether. The extract solution was washed with water, dried over anhydrous magnesium sulfate, then the solvent was distilled off under reduced pressure. The residue was purified by means of silica gel column chromatography (carrier: 30 g; developing solvent: ethyl acetate-hexane = 5:95), followed by recrystallization from ethanol to give Compound 26 (267 mg:yield 86%) as colorless needles.
NMR($CDCl_3$) $\delta$: 1.31(9H,s), 1.63(9H,s), 2.56(3H,s), 4.20(2H,s), 7.30-7.45(4H,m), 7.62(1H,s).

Reference Example 32

Production of 2-t-butyl-5-(4-t-butyl)benzylthio-4-methyl-3(2H)pyridazinone (Compound 27)

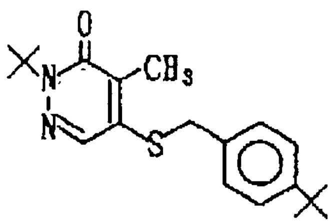

To a solution of 2-t-butyl-5-mercapto-4-methyl-3(2H)pyridazinone (JPA S61(1986)-243078: 108 mg) in DMF (5 ml), anhydrous sodium carbonate (434 mg) and 4-t-butyl benzyl chloride (748 mg) were added, and the mixture was stirred for 30 minutes. To the reaction mixture was added water to suspend the reaction. The reaction mixture was subjected to extraction with ether. The extract solution was washed with water, then dried over anhydrous magnesium sulfate, followed by distilling off the solvent under reduced pressure. The residue was purified by means of a silica gel column chromatography (carrier: 20 g; developing solvent: acetone-hexane = 1:20), followed by recrystallization from hexane to afford Compound 27 (70 mg:yield 37%) as colorless needles.
NMR($CDCl_3$) $\delta$: 1.31(9H,s), 1.63(9H,s), 2.12(3H,s), 4.18(2H,s), 7.25-7.40(4H,m), 7.65(1H,s).

Reference Example 33

Production of 2-t-butyl-5-(4-t-butyl)benzylthio-4-ethyl-3(2H)pyridazinone (Compound 28)

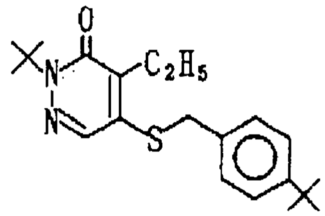

To a solution of 2-t-butyl-4-chloro-5-(4-t-butyl) benzylthio-3(2H)pyridazinone (Compound 1: 500 mg) in tetrahydrofuran (THF: 5 ml) was added dropwise, under ice-cooling, ethyl magnesium chloride (2M THF solution: 0.69 ml). The mixture was stirred for 30 minutes, to which was added water to suspend the reaction. The reaction mixture was subjected to extraction with ether. The extract solution was washed with water, dried and purified by silica gel column chromatography (carrier: 50 g; developing solvent: ether-hexane = 1:9˜ to afford Compound 28 (84 mg:yield 17%) as a white powdery product.
NMR($CDCl_3$) δ: 1.07(3H,t,J = 7.5Hz), 1.31(9H,s), 1.63(9H,s), 2.66(2H,d,J = 7.5Hz), 4.17(2H,s), 7.20-7.40-(4H,m), 7.65(1H,s).

Reference Example 34

Production of 2-t-butyl-5-(4-t-butyl)benzylthio-4-vinyl-3(2H)pyridazinone (Compound 29)

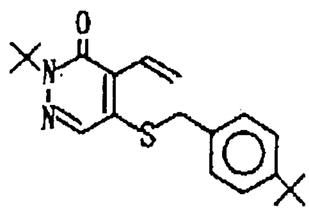

In substantially the same manner as Reference Example 33, from 2-t-butyl-4-chloro-5-(4-t-butyl) benzylthio-3(2H)pyridazinone (Compound 1: 500 mg) and vinyl magnesium bromide (1M THF solution: 2.0 ml), Compound 29 (132 mg:yield 27%) was produced as a white powdery product.
NMR($CDCl_3$) δ: 1.31(9H,s), 1.64(9H,s), 4.20(2H,s), 5.74(1H,dd,J = 2.0Hz,11.5Hz), 6.47-(1H,dd,J = 2.0Hz,17.5Hz), 6.81(1H,dd,J = 11.5Hz), 7.25-7.40(4H,m), 7.71(1H,s).

Reference Example 35

Production of 2-t-butyl-5-(4-t-butyl)benzylthio-4-(2-propenyl)-3(2H)pyridazinone (Compound 30)

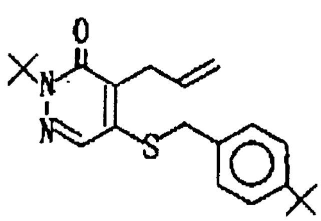

In substantially the same manner as Reference Example 33, from 2-t-butyl-4-chloro-5-(4-t-butyl) benzylthio-3(2H)pyridazinone (Compound 1: 500 mg) and allyl magnesium bromide (1M ether solution: 3.0 ml), Compound 30 (297 mg:yield 59%) was produced as a white powdery product.

NMR($CDCl_3$) δ: 1.31(9H,s), 1.62(9H,s), 3.41(2H,dt, J=1.5Hz,6.5Hz), 4.18(2H,s), 4.95-5.15(2H,m), 5.70-5.95 (1H,m), 7.25-7.40(4H,m), 7.67(1H,s).

Reference Example 36

Production of 2-t-butyl-5-(4-t-butyl)benzyloxy-4-methyl-3(2H)pyridazinone (Compound 31)

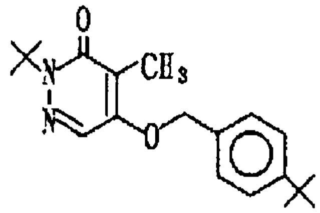

Sodium hydride (60% oil: 163 mg) was suspended in DMF (5 ml), to which was added 4-t-butyl benzyl alcohol (0.58 ml), and the mixture was stirred for 30 minutes. To the mixture was added a solution of 2-t-butyl-5-chloro-4-methyl-3(2H)pyridazinone [JPA S61(1986)-243078: 150 mg] in DMF (5 ml), followed by stirring for further 2 hours. To the reaction mixture was added water to suspend the reaction, and the reaction mixture was subjected to extraction with ether. The extract solution was washed with water, dried over anhydrous magnesium sulfate and, then, the solvent was distilled off under reduced pressure. The residue was purified by means of silica gel column chromatography (carrier: 20 g; developing solvent: acetone-hexane=1:15), followed by recrystallization from isopropyl ether to give Compound 31 (88 mg:yield 36%) as colorless needles.
NMR($CDCl_3$) δ: 1.33(9H,s), 1.84(9H,s), 2.06(3H,s), 5.16(2H,s), 7.25-7.45(4H,m), 7.73(1H,s).

Reference Example 37

Production of 2-t-butyl-5-(4-t-butyl)benzyloxy-4-vinyl-3(2H)pyridazinone (Compound 32)

(1) Production of 2-t-butyl-5-chloro-4-vinyl-3(2H) pyridazinone

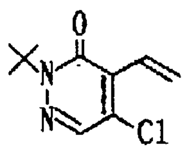

To a solution of 2-t-butyl-4,5-dichloro-3(2H)pyridazinone (1.00 g) in tetrahydrofuran (THF: 5ml) was added dropwise, under ice-cooling, vinyl magnesium bromide (1M THF solution: 13.5 ml). The mixture was stirred for 1 hour. To the reaction mixture was added water to suspend the reaction. The reaction mixture was subjected to extraction with ether. The extract solution was washed with water, dried over anhydrous magnesium sulfate, then the solvent was distilled off under reduced pressure. The residue was purified by means of silica gel column chromatography (carrier: 50 g; developing solvent: ether-hexane=1:9) to afford 2-t-butyl-5-chloro-4-vinyl-3(2H)pyridazinone (334 mg: yield 35%) as a white powdery product.
NMR($CDCl_3$) δ: 1.65(9H,s), 5.83(1H,dd,J=6.0Hz,8.0Hz),6.83(1H,d,J=8.0Hz), 6.84(1H,d,J=6.0Hz), 7.70-(1H,s).

(2) Production of 2-t-butyl-5-(4-t-butyl)benzyloxy-4-vinyl-(2H)pyridazinone (Compound 32)

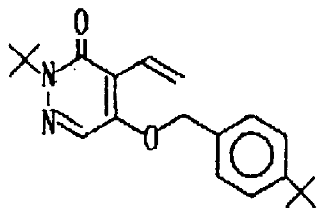

In substantially the same manner as Reference Example 35, from sodium hydride (60% oil: 85 mg), 4-t-butyl benzyl alcohol (0.30 ml) and 2-t-butyl-5-chloro-4-vinyl-3(2H)pyridazinone (300 mg), Compound 32 (91 mg: yield 19%) was produced as a white powdery product.
NMR($CDCl_3$) δ: 1.33(9H,s), 1.64(9H,s), 5.22(2H,s), 5.62(1H,dd,J = 3.0Hz,12.0Hz), 6.57-(1H,dd,J = 3.0Hz,18.0Hz), 6.94(1H,dd,J = 12.0Hz,18.0Hz), 7.30-7.50(4H,m), 7.78(1H,s).

Reference Example 38

Production of 2-t-butyl-4-chloro-5-{4-(t-butoxycarbonyl)benzylthio}-3(2H)-pyridazinone (Compound 33)

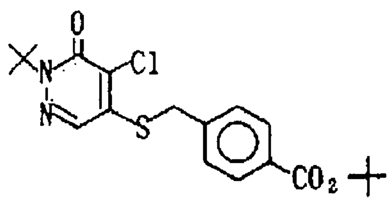

In substantially the same manner as Reference Example 6, from 2-t-butyl-4-chloro-5-mercapto-3(2H)-pyridazinone (1.39 g), anhydrous potassium carbonate (1.90 g) and butyl 4-bromomethyl benzoate (1.90 g), Compound 33 (1.54:yield 59%) was produced as pale yellow prisms.
NMR(DMSO-$d_6$) δ: 1.54(9H,s), 1.56(9H,s), 4.62(2H,s), 7.59(2H,d,J = 8.4Hz), 7.89(2H,d,J = 8.4Hz), 8.01(1H,s).
IR(KBr): 2960, 2940, 1700, 1650, 1605, 1560, 1500 $cm^{-1}$.
m.p.: 101-102 ° C.

| Elemental Analysis for $C_{20}H_{25}ClN_2O_3S$: | | | |
|---|---|---|---|
| Calcd.: | C:58.74%; | H:6.16%; | N:6.85% |
| Found : | C:58.83%; | H:6.27%; | N:6.88% |

Reference Example 39

Production of 2-t-butyl-4-chloro-5-{4-(t-butoxycarbonyl)benzyloxy}-3(2H)-pyridazinone (Compound 34)

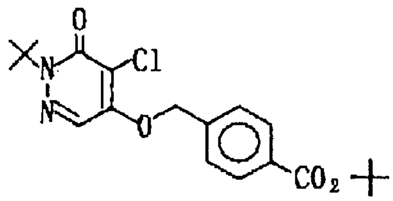

In substantially the same manner as Reference Example 23, from 2-t-butyl-4-chloro-5-hydroxy-3(2H)-pyridazinone (1.24 g), anhydrous potassium carbonate (1.69 g) and butyl 4-bromomethyl benzoate (1.82 g), Compound 34 (1.27 g:yield 53%) was produced as colorless needles.
NMR(DMSO-$d_6$) $\delta$: 1.55(9H,s), 1.57(9H,s), 5.55(2H,s), 7.57(2H,d,J = 8.0Hz), 7.96(2H,d,J = 8.0Hz), 8.23(1H,s).
IR(KBr): 2970, 2930, 1705, 1650, 1605, 1500, 1475, 1445, 1400 $cm^{-1}$.
m.p.: 178-179 ° C.

| Elemental Analysis for $C_{20}H_{25}ClN_2O_4$: | | | |
|---|---|---|---|
| Calcd.: | C:61.14%; | H:6.41%; | N:7.13%. |
| Found : | C:61.18%; | H:6.39%; | N:7.13%. |

Reference Example 40

Production of 2-t-butyl-4-chloro-5-{4-(carboxyl) benzylthio}-3(2H)-pyridazinone (Compound 35)

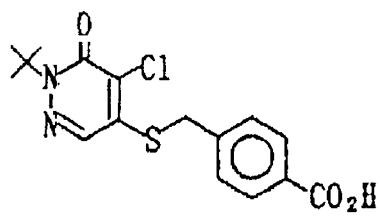

In a 4N HCl ethyl acetate solution (8 ml) was dissolved 2-t-butyl-4-chloro-5-{4-(t-butoxycarbonyl) benzylthio}-3(2H)-pyridazinone (500 mg). The solution was stirred for 3.5 hours at room temperature. The solvent was distilled off under reduced pressure. To the residue was added ether, which was subjected to washing with a supersonic washing machine. The resulting powdery product was recovered by filtration, washed with ether, and then dried under reduced pressure to give Compound 35 (415 mg:yield 96%) as a pale green powdery product.
NMR(DMSO-$d_6$) $\delta$: 1.56(9H,s), 4.63(2H,s), 7.59(2H,d,J = 8.0Hz), 7.94(2H,d,J = 8.0Hz), 8.03(1H,s).
IR(KBr): 2960, 2900, 1710, 1600, 1500 $cm^{-1}$
m.p.: 215-217 ° C

| Elemental Analysis for $C_{16}H_{17}ClN_2O_3S$: | | | |
|---|---|---|---|
| Calcd.: | C: 54.47%; | H: 4.86%; | N: 7.94% |
| Found : | C: 54.26%; | H: 4.93%; | N: 7.82% |

Reference Example 41

Production of 2-t-butyl-4-chloro-5-{4-(carboxyl)benzyloxy}-3(2H)-pyridazinone (Compound 36)

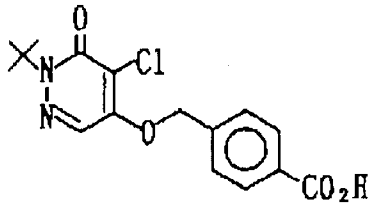

In substantially the same manner as Reference Example 40, from 2-t-butyl-4-chloro-5-{4-(t-butoxycarbonyl)benzyloxy}-3(2H)-pyridazinone (500 mg), Compound 36 (390 mg: yield 93%) was produced as a white powdery compound.
NMR(DMSO-$d_6$) δ: 1.58(9H,s), 5.55(2H,s), 7.57(2H,d,J = 8.4Hz), 8.00(2H,d,J = 8.4Hz), 8.24(1H,s).
IR(KBr): 3080, 2960, 2930, 1690, 1660, 1570, 1500 $cm^{-1}$.
m.p.: 208-209 °C

| Elemental Analysis for $C_{16}H_{17}ClN_2O_4$: | | | |
|---|---|---|---|
| Calcd.: | C:57.06%; | H:5.09%; | N:8.32% |
| Found : | C:56.94%; | H:4.99%; | N:8.65% |

Reference Example 42

Production of 2-t-butyl-5-(4-t-butyl)benzylthio-4-methoxy-3(2H)pyridazinone (Compound 37)

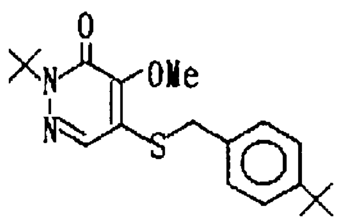

To methanol (2 ml) was added gradually sodium hydride (274 mg). After bubbling ceased, a solution of 2-t-butyl-4-chloro-5-(4-t-butyl)benzylthio-3(2H) pyridazinone (Compound 1: 500 mg) in methanol (2 ml) was added, and the mixture was stirred for 3 hours. To the reaction mixture was added water to suspend the reaction. The reaction product was subjected to extraction with ether. The extract solution was washed with water, dried over anhydrous magnesium sulfate, and the solvent was distilled off under reduced pressure. The residue was purified by means of silica gel column chromatography (carrier: 50 g; developing solvent: ether-hexane = 1:4) to give Compound 37 (67 mg: yield 14%) as a white powdery product.
NMR($CDCl_3$) δ: 1.31(9H,s), 1.63(9H,s), 4.10(3H,s), 4.15(2H,s), 7.25-7.40(4H,m), 7.59(1H,s).

Reference Example 43

Production of 5-(4-t-butyl)benzylthio-4-chloro-2-phenyl-3(2H)pyridazinone (Compound 38)

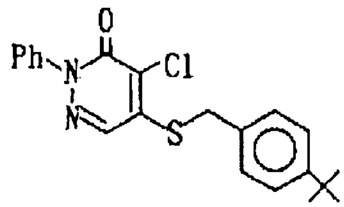

In substantially the same manner as Reference Example 6, from 4-chloro-5-mercapto-2-phenyl-3(2H) pyridazinone (300 mg), anhydrous sodium carbonate (133 mg) and 4-t-butylbenzylchloride (230 mg), Compound 38 (276 mg: yield 57%) was produced as colorless needles.
NMR($CDCl_3$) δ: 1.32(9H,s), 4.32(2H,s), 7.30-7.60(9H,m), 7.82(1H,s).

Reference Example 44

Production of 2-t-butyl-4-chloro-5-[6-(4-fluorobenzyloxy)-3-pyridyl]methoxy-3(2H)-pyridazinone (Compound 49)

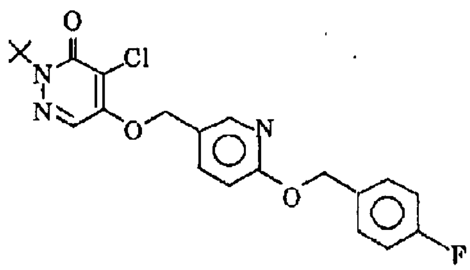

2-t-Butyl-4,5-dichloro-3(2H)-pyridazinone (663 mg) was dissolved in DMF (10 ml). To the solution were added 6-(4-fluorobenzyloxy)-3-pyridine methanol (840 mg) and anhydrous cesium carbonate (977 mg). The mixture was stirred for 17 hours at 80 °C. To the reaction mixture was added water, which was subjected to extraction with ethyl acetate. The organic layer was washed with water and a saturated aqueous solution of sodium chloride, successively, followed by drying over anhydrous magnesium sulfate, which was concentrated under reduced pressure. The concentrate was refined by means of silica gel column chromatography (developing solvent; hexane - ethyl acetate = 2:1), followed by recrystallization from ether to give Compound 49 (552 mg) as a white powdery product.
NMR($CDCl_3$) δ: 1.64(9H,s), 5.23(2H,s), 5.36(2H,s), 6.85(1H,d,J = 8Hz), 7.06(2H,dd,J = 9Hz,9Hz), 7.43-(2H,dd,J = 5Hz,9Hz), 7.68(1H,dd,J = 3Hz,8Hz), 7.76(1H,s), 8.21(1H,d,J = 3Hz).

Reference Example 45

Production of 2-t-butyl-4-chloro-5-[6-(4-fluorobenzyloxy)-3-pyridyl]methylthio-3(2H)-pyridazinone (Compound 50)

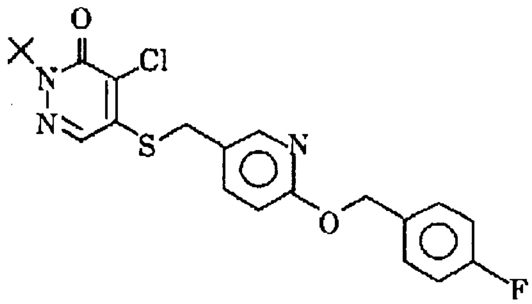

2-t-butyl-4-chloro-5-mercapto-3(2H)-pyridazinone (875 mg) was dissolved in DMF (10 ml). To the solution were added 6-(4-fluorobenzyloxy)-3-chloromethylpyridine (977 mg) and potassium carbonate (829 mg). The mixture was stirred for 17 hours at room temperature. To the reaction mixture was added water, followed by extraction with ethyl acetate. The organic layer was washed with water and a saturated aqueous solution of spdium chloride, successively, followed by drying over anhydrous magnesium sulfate, which was concentrated under reduced pressure. The concentrate was refined by means of silica gel column chromatography (developing solvent: hexane - ethyl acetate = 4:1), followed by recrystallization from hexane-ether to give Compound 50 (600 mg) as a colorless crystalline product.
NMR($CDCl_3$) δ: 1.63(9H,s), 4.21(2H,s), 5.34(2H,s), 6.81(1H,d,J=8Hz), 7.06(2H,dd,J=9Hz,9Hz), 7.43-(2H,dd,J=5Hz,9Hz), 7.63(1H,s), 7.65(1H,dd,J=3Hz,8Hz), 8.17(1H,d,J=3Hz).

| Elemental Analysis for $C_{21}H_{21}N_3O_2SC1F$: | | | |
|---|---|---|---|
| Calcd.: | C:58.13%; | H:4.88%; | N:9.68% |
| Found : | C:57.84%; | H:4.80%; | N:9.72% |

Reference Example 46

Production of 2-t-butyl-5-(4-t-butylcyclohexyl)methoxy-4-chloro-3(2H)-pyridazinone (Compound 51)

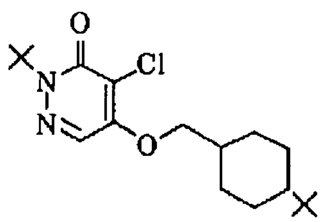

2-t-Butyl-4-chloro-5-hydroxy-3(2H)-pyridazinone (203 mg) was dissolved in DMF (5 ml). To the solution were added 4-t-butyl-1-bromomethylcyclohexane (280 mg) and potassium carbonate (207 mg). The mixture was stirred for 17 hours at 80°C. To the reaction mixture was added water, which was subjected to extraction with eather. The organic layer was washed with water and a saturated aqueous solution of sodium chloride, successively, followed by drying over anhydrous magnesium sulfate, which was concentrated under reduced pressure. The concentrate was refined by means of silica gel column chromatography (developing solvent: hexane - ethyl acetate = 4:1) to give Compound 51 (312 mg) as a white powdery product.
NMR($CDCl_3$) δ: 0.86(9H,s), 1.20-0.90(5H,m), 1.65(9H,s), 1.70-2.00(5H,m), 4.00(2H,d,J=6Hz), 7.72(1H,s).

| Elemental Analysis for $C_{19}H_{31}N_2O_2C1$: | | | |
|---|---|---|---|
| Calcd.: | C:64.30%; | H:8.80%; | N:7.89% |
| Found : | C:64.31%; | H:8.69%; | N:8.03% |

Reference Example 47

Production of 2-t-butyl-5-(4-t-butylcyclohexyl)methylthio-4-chloro-3(2H)-pyridazinone (Compound 52)

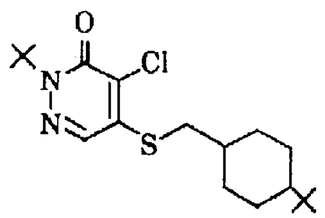

2-t-Butyl-4-chloro-5-mercapto-3(2H)-pyridazinone (131 mg) was dissolved in DMF (5 ml). To the solution were added 4-t-butyl-1-bromomethylcyclohexane (168 mg) and potassium carbonate (124 mg). The mixture was stirred for 17 hours at room temperature. To the reaction mixture was added water, which was subjected to extraction with ether. The organic layer was washed with water and a saturated aqueous solution of sodium chloride, successively, followed by drying over anhydrous magnesium sulfate, which was concentrated under reduced pressure. The concentrate was refined by means of silica gel column chromatography (developing solvent: hexane - ethyl acetate = 4:1), followed by recrystallization from hexane to give Compound 52 (98 mg) as a colorless crystalline product.
NMR($CDCl_3$) δ: 0.85(9H,s), 0.90-2.10(10H,m), 1.65(9H,s), 2.88(2H,d,J=7Hz), 7.59(1H,s).

| Elemental Analysis for $C_{19}H_{31}N_2OSC1$: | | | |
|---|---|---|---|
| Calcd.: | C:61.51%; | H:8.42%; | N:7.55% |
| Found : | C:61.40%; | H:8.28%; | N:7.27% |

Reference Example 48

Production of 2-t-butyl-5-(4-t-butyl-1-hydroxycyclohexyl)methylthio-4-chloro-3(2H)-pyridazinone (Compound 53)

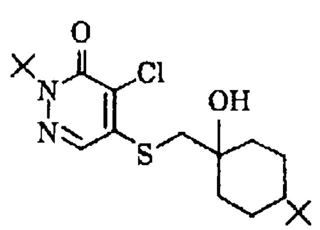

Sodium hydride (60 % in oil: 84 mg) was dissolved in methanol (10 ml). To the solution was added, after the bubbling was over, 2-t-butyl-4-chloro-5-mercapto-3(2H)-pyridazinone (459 mg), and the mixture was stirred for 20 minutes at room temperature. To the reaction mixture was then added 4-t-butylmethylenecyclohexane oxide (353 mg), which was stirred for 17 hours at room temperature. To the reaction mixture was added water, which was subjected to extraction with ethyl acetate - THF. The organic layer was washed with a saturated aqueous solution of sodium chloride, which was dried over anhydrous magnesium

sulfate, followed by concentration under reduced pressure. The concentrate was refined by means of silica gel column chromatography (developing solvent: hexane - ethyl acetate = 4:1), followed by recrystallization from hexane to give Compound 53 (600 mg) as a white powdery product.
NMR($CDCl_3$) δ: 0.87(9H,s), 0.90-2.00(9H,m), 1.64(9H,s) 3.12(2H,s), 7.78(1H,s).

| Elemental Analysis for $C_{19}H_{31}N_2O_2SC1$: | | | |
|---|---|---|---|
| Calcd.:<br>Found : | C:58.97%;<br>C:58.98%; | H:8.07%;<br>H:8.05%; | N:7.24%<br>N:7.10% |

Reference Example 49

Production of 2-t-butyl-5-(1-chloroacetylcarbamoyloxy-4-t-butylcyclohexyl)methylthio-4-chloro-3(2H)-pyridazinone (Compound 54)

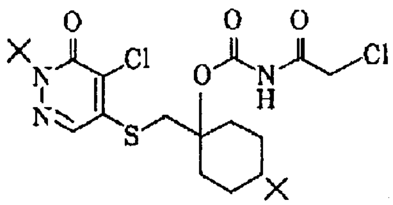

Compound 53 (271 mg) was dissolved in dichloromethane (5 ml), to which was added, under ice-cooling, chloroacetyl isocyanate (100 mg), and the mixture was stirred for 17 hours at room temperature. To the reaction mixture was added water, which was subjected to extraction with dichloromethane. The organic layer was washed with a saturated aqueous solution of sodium chloride, followed by drying over anhydrous magnesium sulfate, followed by concentration under reduced pressure. The concentrate was refined by means of silica gel column chromatography (developing solvent: hexane - ethyl acetate = 4:1) to give Compound 54 (322 mg) as a white powdery product.
NMR($CDCl_3$) δ: 0.87(9H,s), 0.90-1.80(7H,m), 1.64(9H,s), 2.40-2.60(2H,m), 3.63(2H,s), 4.42(2H,s), 7.67(1H,s), 7.87(1H,brs).

| Elemental Analysis for $C_{22}H_{33}N_3O_4SC1_2$: | | | |
|---|---|---|---|
| Calcd.:<br>Found : | C:52.17%;<br>C:52.12%; | H:6.57%;<br>H:6.31%; | N:8.30%<br>N:8.17% |

Reference Example 50

Production of 2-t-butyl-5-(4-t-butyl-1-carbamoyloxycyclohexyl)methylthio-4-chloro-3(2H)-pyridazinone (Compound 55)

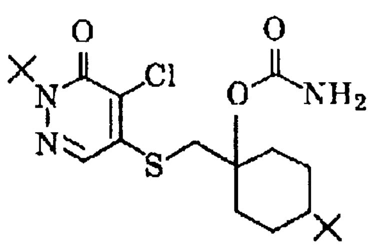

Compound 54 (101 mg) was dissolved in a mixed solvent of methanol and THF (1:1) (5 ml). To the solution was added, under ice-cooling, 1N aqueous solution of sodium hydroxide (0.4 ml). The mixture was stirred for one hour at room temperature. To the reaction mixture was added water, which was subjected to extraction with ethyl acetate. The organic layer was washed with a saturated aqueous solution of sodium chloride, which was dried over anhydrous magnesium sulfate, followed by concentration under reduced pressure. The concentrate was refined by means of silica gel column chromatography (developing solvent: hexane - ethyl acetate = 2:1) to give Compound 55 (61 mg) as a white powdery product.
NMR($CDCl_3$) δ: 0.87(9H,s), 0.90-1.90(7H,m), 1.64(9H,s), 2.45-2.60(2H,m), 3.65(2H,s), 4.56(2H,brs), 7.73-(1H,s).

| Elemental Analysis for $C_{20}H_{32}N_3O_3SC1$: | | | |
|---|---|---|---|
| Calcd.: | C:55.86%; | H:7.50%; | N:9.77% |
| Found : | C:55.82%; | H:7.49%; | N:9.74% |

Reference Example 51

Production of 4,5-dichloro-2-pivaloyloxymethyl-3(2H)-pyridazinone

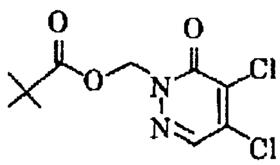

To a solution of 4,5-dichloro-3(2H)-pyridazinone (2.48 g) in DMF (25 ml) were added anhydrous potassium carbonate (1.10 g) and pyvaloyloxymethyl chloride (3.39 g). The mixture was stirred for two hours at 55 °C. The solvent was distilled off under reduced pressure. From the residue was removed ether-soluble substances to give a crude product. This crude product was refined by means of flash column chromatography (developing solvent: hexane - ethyl acetate = 9:1) to give the above-titled compound (1.67 g).
NMR($CDCl_3$) δ: 1.21(9H,s), 6.05(2H,s), 7.81(1H,s).
IR(KBr): 3420, 3330, 3100, 3070, 2980, 2970, 2930, 2870, 1720, 1675, 1580, 1480, 1460, 1440, 1360, 1320, 1270, 1220, 1150, 1130, 1030, 1005 $cm^{-1}$.

Reference Example 52

Production of 4-chloro-5-mercapto-2-pivaloyloxymethyl-3(2H)-pyridazinone

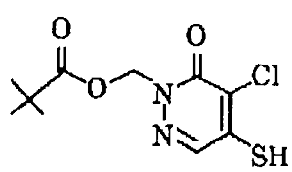

In water (20 ml) was dissolved 70% sodium hydrosulfide hydride. To the solution was added 4,5-dichloro-2-pivaloyloxymethyl-3(2H)-pyridazinone (2.8 g). The mixture was stirred for 3 hours at 60 °C. Insolubles were filtered off, and washed with water. The filtrate and the washing were combined, which was made acidic (pH 2) with conc. hydrochloric acid. The resulting precipitates were collected by filtration and dried to afford the above-titled compound (2.39 g).
NMR($CDCl_3$) δ: 1.21(9H,s), 4.17(1H,s), 6.02(2H,s), 7.65(1H,s).

Reference Example 53

Production of 5-(4-t-butylbenzyloxy)-4-chloro-2-pivaloyloxymethyl-3(2H)-pyridazinone (Compound 56)

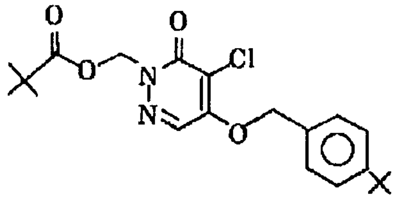

To a solution of 4,5-dichloro-2-pivaloyloxymethyl-3(2H)-pyridazinone (4.51 g) in DMF (85 ml) were added anhydrous cesium carbonate (3.16 g) and 4-t-butylbenzyl alcohol (3.98 g). The mixture was stirred for 12 hours at 80 °C. Insolubles were filtered off, and the solvent was distilled off under reduced pressure. The residue was dissolved in ethyl acetate, which was washed with a saturated aqueous solution of sodium chloride, followed by drying over anhydrous magnesium sulfate. The solvent was distilled off under reduced pressure. The residue was refined by means of flash column chromatography (developing solvent: hexane - ethyl acetate = 9:1 ~ 4:1) to give Compound 56 (4.54 g).

NMR($CDCl_3$) $\delta$: 1.19(9H,s), 1.33(9H,s), 5.33(2H,s), 6.05(2H,s), 7.33(2H,d,J=8Hz), 7.44(2H,d,J=8Hz), 7.84-(1H,s).

IR(KBr): 3430, 2960, 2900, 2870, 1745, 1725, 1650, 1600, 1510, 1475, 1455, 1440, 1400, 1395, 1380, 1375, 1365, 1310, 1280, 1205, 1190, 1135, 1110, 1080, 1030cm $^{-1}$.

Reference Example 54

Production of 5-(4-t-butylbenzylthio)-4-chloro-2-pivaloyloxymethyl-3(2H)-pyridazinone (Compound 57)

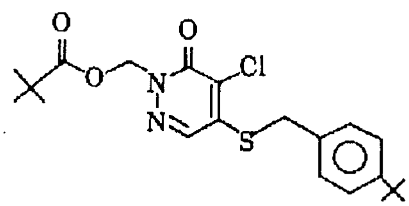

To a solution of 4-chloro-5-mercapto-2-pivaloyloxymethyl-3(2H)-pyridazinone (2.07 g) in DMF (39.5 ml) were added anhydrous potassium carbonate (0.777 g) and 4-t-butylbenzyl chloride (1.64 g). The mixture was stirred for 15 hours at room temperature. Insolubles were filtered off, and the solvent was distilled off under reduced pressure. The residue was dissolved in ethyl acetate. The solution was washed with a saturated aqueous solution of sodium chloride, followed by drying over anhydrous magnesium sulfate. The solvent was distilled off under reduced pressure. The residue was refined by means of flash column chromatography (developing solvent: hexane - ethyl acetate = 9:1 ~ 4:1) to give Compound 57 (0.56 g).

NMR($CDCl_3$) $\delta$: 1.19(9H,s), 1.31(9H,s), 4.28(2H,s), 6.03(2H,s), 7.32(2H,d,J=9Hz), 7.39(2H,d,J=9Hz), 7.72-(1H,s).

IR(KBr): 3430, 3100, 2960, 2860, 1725, 1650, 1570, 1510, 1480, 1460, 1440, 1395, 1360, 1280, 1230, 1210, 1190, 1150, 1120, 1030, 1015 cm$^{-1}$.

Reference Example 55

Production of 5-(4-t-butylbenzyloxy)-4-chloro-3(2H)-pyridazinone (Compound 58)

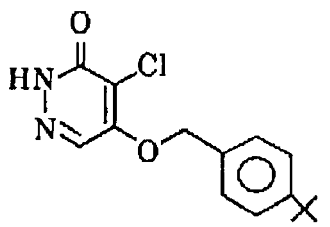

5-(4-t-Butylbenzyloxy)-4-chloro-2-pivaloyloxymethyl-3(2H)-pyridazinone (Compound 56) (2.78 g) was dissolved in a mixture solvent of methanol-THF (2:1) (120 ml). To the solution was added a 0.5N aqueous solution of sodium hydroxide (27.2 ml). The mixture was stirred for 15 hours at room temperature, to which was added 1N HCl (13.6 ml), followed by distilling off the solvent under reduced pressure. The resulting crystalline precipitate was collected by filtration, which was washed with water and ether, followed by drying to give Compound 58 (1.53 g).
NMR($CDCl_3$) δ: 1.33(9H,s), 5.33(2H,s), 7.33(2H,d,J = 8Hz), 7.44(2H,d,J = 8Hz), 7.85(1H,s).
IR(KBr): 3430, 3200, 3120, 3020, 2960, 2870, 1645, 1600, 1515, 1460, 1400, 1320, 1280, 1140, 1100, 1020 $cm^{-1}$.

| Elemental Analysis for $C_{15}H_{17}N_2O_2C1 \cdot 0.3H_2O$: | | | |
|---|---|---|---|
| Calcd.: | C:60.42%; | H:5.95%; | N:9.40% |
| Found : | C:60.35%; | H:5.70%; | N:9.38% |

Reference Example 56

Production of 5-(4-t-butylbenzylthio)-4-chloro-3(2H)-pyridazinone (Compound 59)

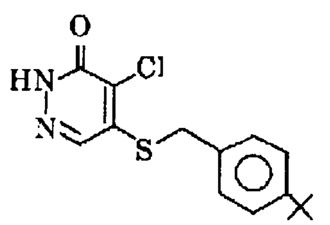

In substantially the same manner as Reference Example 55, Compound 59 (0.518 g) was obtained from 5-(4-t-butylbenzylthio)-4-chloro-2-pivaloyloxymethyl-3(2H)-pyridazinone (Compound 57) (1.01 g), a 0.5N aqueous solution of sodium hydroxide (9.52 ml) and 1N hydrochloric acid (4.76 ml).
NMR($CDCl_3$) δ: 1.32(9H,s), 4.29(2H,s), 7.32(2H,d,J = 9Hz), 7.40(2H,d,J = 9Hz), 7.74(1H,s).
IR(KBr): 3440, 3280, 3220, 3170, 3100, 3030, 2960, 2900, 2850, 1650, 1565, 1515, 1470, 1460, 1440, 1410, 1360, 1300, 1260, 1230, 1175, 1140, 1100, 1070, 1020 $cm^{-1}$.

| Elemental Analysis for $C_{15}H_{17}N_2OSC1 \cdot 0.5H_2O$: | | | |
|---|---|---|---|
| Calcd.: | C:56.68%; | H:5.71%; | N:8.81% |
| Found : | C:56.89%; | H:5.23%; | N:8.73% |

Reference Example 57

Production of 5-(4-t-butylbenzyloxy)-4-chloro-2-piperidinomethyl-3(2H)-pyridazinone (Compound 60)

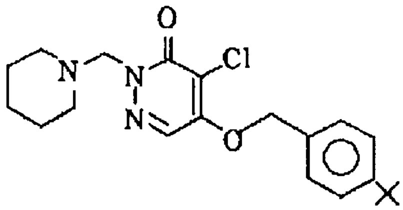

5-(4-t-Butylbenzyloxy)-4-chloro-3(2H)-pyridazinone (Compound 58) (59 mg) was suspended in ethanol (0.4 ml). To the suspension were added piperidine (18.8 mg) and a 37% aqueous solution of formaldehyde (0.02 ml), and the mixture was warmed for 3 minutes at 55 °C. The reaction mixture was cooled with ice. Resulting colorless needles were collected by filtration. The crystals were washed with ethanol and ether, followed by drying to give Compound 60 (49 mg).

NMR($CDCl_3$) δ: 1.33(9H,s), 1.34-1.68(6H,m), 2.66(4H,t,J = 5Hz), 5.04(2H,s), 5.30(2H,s), 7.34(2H,d,J = 8Hz), 7.44(2H,d,J = 8Hz), 7.78(1H,s).

IR(KBr): 3450, 3100, 3060, 2940, 2860, 2810, 1650, 1630, 1600, 1520, 1460, 1405, 1380, 1360, 1310, 1280, 1180, 1150, 1110, 1095, 1020 $cm^{-1}$.

Reference Example 58

Production of 5-(4-t-butylbenzyloxy)-4-chloro-2-dimethylaminomethyl-3(2H)-pyridazinone (Compound 61)

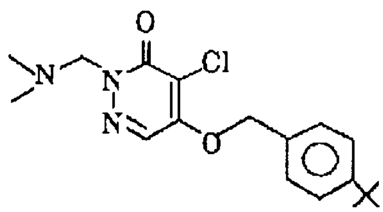

By substantially the same procedure as in Reference Example 57, Compound 61 (204 mg) was produced from 5-(4-t-butylbenzyloxy)-4-chloro-3(2H)-pyridazinone (Compound 58) (293 mg), ethanol (2 ml), a 50% aqueous solution of dimethylamine (99 mg) and a 37% aqueous solution of formaldehyde.

NMR($CDCl_3$) δ: 1.33(9H,s), 2.42(6H,s), 4.99(2H,s), 5.32(2H,s), 7.35(2H,d,J = 9Hz), 7.45(2H,d,J = 9Hz), 7.80-(1H,s).

IR(KBr): 3450, 3100, 3060, 2960, 2860, 2800, 1640, 1600, 1520, 1470, 1460, 1440, 1400, 1380, 1360, 1315, 1290, 1270, 1160, 1140, 1095, 1060, 1020 $cm^{-1}$.

Reference Example 59

Production of 2-bis(2-chloroethyl)aminomethyl-5-(4-butylbenzyloxy)-4-chloro-3(2H)-pyridazinone (Compound 62)

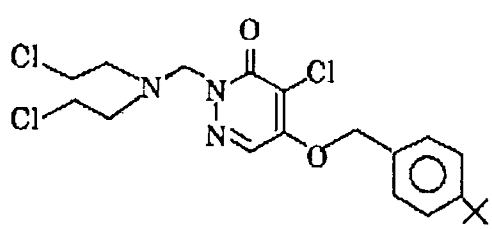

By substantially the same procedure as in Reference Example 57, Compound 62 (86 mg) was obtained from 5-(4-t-butylbenzyloxy)-4-chloro-3(2H)-pyridazinone (Compound 58) (147 mg), ethanol (1.5 ml), bis(2-chloroethyl)amine (106.5 mg) and a 37% aqueous solution of formaldehyde (0.06 ml).
NMR($CDCl_3$) δ: 1.33(9H,s), 3.18(4H,t,J = 7Hz), 3.59(4H,t,J = 7Hz), 5.15(2H,s), 5.31(2H,s), 7.34(2H,d,J = 8Hz), 7.44(2H,d,J = 8Hz), 7.80(1H,s).
IR(KBr): 3430, 3100, 3060, 2960, 2860, 1650, 1630, 1595, 1515, 1450, 1410, 1385, 1365, 1310, 1270, 1230, 1150, 1120, 1110, 1090, 1035, 1015 $cm^{-1}$.

Reference Example 60

Production of 5-(4-t-butylbenzyloxy)-4-chloro-2-hydroxymethyl-3(2H)-pyridazinone (Compound 63)

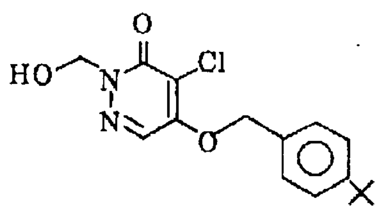

By substantially the same procedure as in Reference Example 57, Compound 63 (72 mg) was obtained from 5-(4-butylbenzyloxy)-4-chloro-3(2H)-pyridazinone (Compound 58) (147 mg), ethanol (1.5 ml) and a 37% aqueous solution of formaldehyde (0.15 ml).
NMR($CDCl_3$) δ: 1.33(9H,s), 4.12(1H,t,J = 7Hz), 5.34(2H,s), 5.54(2H,d,J = 7Hz), 7.33(2H,d,J = 8Hz), 7.45-(2H,d,J = 8Hz), 7.82(1H,s).
IR(KBr): 3440, 3280, 2960, 1625, 1595, 1510, 1460, 1420, 1400, 1360, 1320, 1310, 1270, 1240, 1170, 1095, 1070 $cm^{-1}$.

Reference Example 61

Production of 2-t-butyl-5-hydroxy-3(2H)-pyridazinone

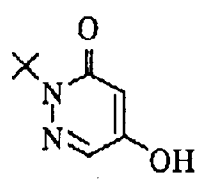

To a solution of 2-t-butyl-4-chloro-5-hydroxy-3(2H)-pyridazinone (608 mg) in methanol (60 ml) was added 10% Pd-C (304 mg). The mixture was stirred vigorously for 20 hours under hydrogen atmosphere. The catalyst was filtered off. The filtrate was washed with methanol, to which were added sodium hydrogencarbonate (252 mg) and water (20 ml), and the mixture was stirred. Methanol was distilled off under reduced pressure to cause precipitation of colorless needles. The crystalline product was collected by filtration, washed with water, methanol and ether, and then dried to give the above-titled compound (349 mg).
NMR($CDCl_3$) δ: 1.64(9H,s), 6.05(1H,d,J = 3Hz), 7.58(1H,d,J = 3Hz).
IR(KBr): 3440, 3060, 3020, 2980, 2930, 1645, 1600, 1565, 1540, 1470, 1395, 1365, 1325, 1270, 1230, 1190, 1150, 1130, 1020, 1005 $cm^{-1}$.

Reference Example 62

Production of 5-(4-t-butylbenzyloxy)-2-t-butyl-3(2H)-pyridazinone (Compound 64)

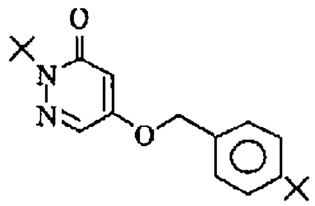

To a solution of 2-t-butyl-5-hydroxy-3(2H)-pyridazinone (169 mg) in DMF (7.5 ml) were added sodium carbonate (106 mg) and 4-t-butylbenzyl chloride (219 mg). The mixture was stirred for 7 hours at 70 °C. Insolubles were filtered off, then the solvent was distilled off under reduced pressure. The residue was refined by means of flash column chromatography (developing solvent: hexane - ethyl acetate = 19:1 ~ 13:1) to give Compound 64 (287 mg).
NMR($CDCl_3$) δ: 1.33(9H,s), 1.62(9H,s), 4.95(2H,s), 6.12(1H,d,J = 3Hz), 7.32(2H,d,J = 8Hz), 7.43(2H,d,J = 8Hz), 7.54(1H,d,J = 3Hz).
IR(KBr): 3430, 3060, 2970, 2930, 2860, 1655, 1600, 1540, 1515, 1450, 1405, 1390, 1360, 1340, 1310, 1265, 1240, 1195, 1145, 1105, 1085, 1020, 1010 $cm^{-1}$.

Reference Example 63

Production of 2-t-butyl-4,5-difluoro-3(2H)-pyridazinone

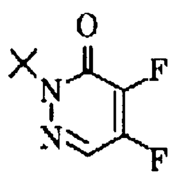

Under argon atmosphere, 2-t-butyl-4,5-dichloro-3(2H)-pyridazinone(6.91 g) and spray-dry potassium fluoride (7.26 g) were suspended in anhydrous dimethyl sulfoxide (300 ml). The suspension was stirred for 25 hours at 160 °C. The reaction mixture was poured into water (1.5 liter) to thereby extract the product with ether (1 liter). The extract solution was washed with a saturated aqueous soluion of sodium chloride and, then, dried over anhydrous sodium sulfate, followed by distilling off the solvent under reduced pressure. The residue was refined by means of a flash column chromatography (developing solvent: hexane - ethyl acetate = 99:1) to give the above-titled compound (1.13 g)
NMR($CDCl_3$) δ: 1.65(9H,s), 7.80(1H,d,J = 8Hz).
IR(KBr): 3430, 3000, 2980, 2940, 2870, 1680, 1665, 1530, 1505, 1490, 1460, 1400, 1365, 1340, 1320, 1260, 1225, 1190, 1140, 1090 $cm^{-1}$.

Reference Example 64

Production of 5-(4-t-butylbenzyloxy)-2-t-butyl-4-fluoro-3(2H)-pyridazinone (Compound 65)

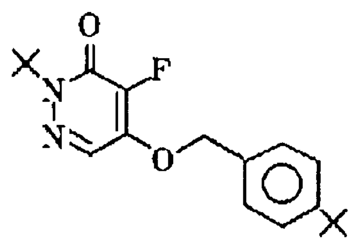

By substantially the same procedure as in Reference Example 53, Compound 65 (347 mg) was obtained from 2-t-butyl-4,5-difluoro-3(2H)-pyridazinone (303 mg), anhydrous cesium carbonate (265 mg) and 4-t-butylbenzyl alcohol (365 mg) in DMF (7.4 ml).
NMR($CDCl_3$) δ: 1.33(9H,s), 1.63(9H,s), 5.30(2H,d,J = 2Hz), 7.33(2H,d,J = 8Hz), 7.43(2H,d,J = 8Hz), 7.61-(1H,d,J = 8Hz).
IR(KBr): 3430, 2990, 2950, 2900, 2870, 1655, 1650, 1640, 1510, 1460, 1405, 1380, 1365, 1340, 1315, 1280, 1270, 1230, 1190, 1150, 1115, 1020 $cm^{-1}$.

| Elemental Analysis for $C_{19}H_{25}N_2O_2F$: | | | | |
|---|---|---|---|---|
| Calcd.: | C:68.65%; | H:7.58%; | N:8.43%; | F:5.72% |
| Found : | C:68.51%; | H:7.34%; | N:8.46%; | F:5.55% |

Reference Example 65

Production of 5-(4-t-butylbenzylthio)-2-t-butyl-4-fluoro-3(2H)-pyridazinone (Compound 66)

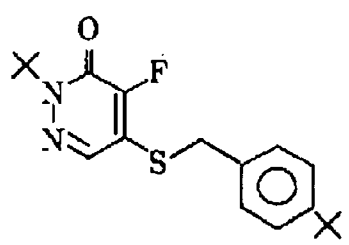

By substantially the same procedure as in Reference Example 53, Compound 66 was obtained from 2-t-butyl-4,5-difluoro-3(2H)-pyridazinone (377 mg), anhydrous cesium carbonate (652 mg) and 4-t-butylbenzylthiol (577 mg) in DMF (10 ml).
NMR($CDCl_3$) δ: 1.31(9H,s), 1.62(9H,s), 4.23(2Hs), 7.27(2H,d,J = 8Hz), 7.36(2H,d,J = 8Hz), 7.56(2H,d,J = 8Hz).
IR(KBr): 3450, 2970, 2940, 2870, 1645, 1600, 1560, 1540, 1520, 1510, 1480, 1460, 1420, 1395, 1365, 1325, 1270, 1225, 1180, 1140, 1105, 1020 $cm^{-1}$.

| Elemental Analysis for $C_{19}H_{25}N_2OSF$: | | | | |
|---|---|---|---|---|
| Calcd.: | C:65.49%; | H:7.23%; | N:8.04%; | F:5.45% |
| Found : | C:65.17%; | H:7.04%; | N:7.99%; | F:5.42% |

Reference Example 66

Production of 4-bromo-2-t-butyl-5-(4-t-butyl)benzyloxy-3(2H)pyridazinone (Compound 67)

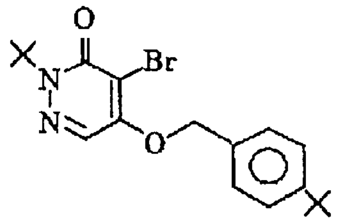

To a solution of 4,5-dibromo-2-t-butyl-3(2H)-pyridazinone (500 mg) in DMF (5 ml) were added anhydrous cesium carbonate (613 mg) and 4-t-butylbenzyl alcohol (0.33 ml). The mixture was stirred for 3 hours at 100 ° C, which was cooled to room temperature, followed by addition of water. The product was extracted with ether. The extract solution was washed with a saturated aqueous solution of sodium chloride and then dried over anhydrous sodium sulfate, followed by distilling off the solvent under reduced pressure. The residue was refined by means of silica gel chromatography (developing solvent: ether-hexane = 1:4), which was recrystallized from ether-hexane to give Compound 67 (188 mg; yield 25%) as colorless needles.
NMR($CDCl_3$) δ: 1.33(9H,s), 1.64(9H,s), 5.29(2H,s), 7.30-7.50(4H,m), 7.66(1H,s).

Reference Example 67

Production of 2-benzyl-5-(4-t-butyl)benzylthio-4-chloro-3(2H)pyridazinone (Compound 68)

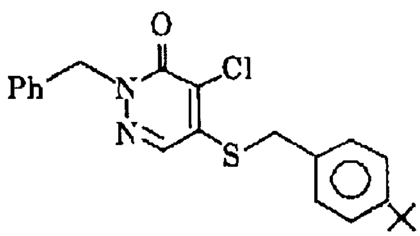

In substantially the same manner as Reference Example 6, Compound 68 (518 mg; yield 65%) was obtained as colorless needles from 2-benzyl-4-chloro-5-mercapto-3(2H)pyridazinone (505 mg), anhydrous sodium carbonate (212 mg) and 4-t-butylbenzyl chloride (0.39 ml).
NMR($CDCl_3$) δ: 1.30(9H,s), 4.23(2H,s), 5.30(2H,s), 7.25-7.45(9H,m), 7.67(1H,s).

Reference Example 68

Production of 2-benzyl-5-(4-t-butyl)benzyloxy-4-chloro-3(2H)pyridazinone (Compound 69)

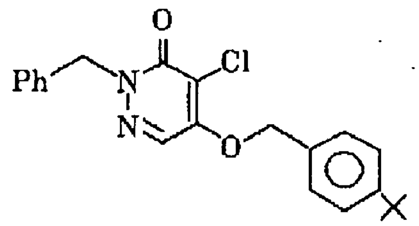

In substantially the same manner as Reference Example 66, Compound 69 (518 mg; yield 65%) was obtained as colorless needles from 2-benzyl-4,5-dichloro-3(2H)pyridazinone (510 mg), anhydrous cesium carbonate (651 mg) and 4-butylbenzyl alcohol (0.53 mg).
NMR($CDCl_3$) δ: 1.32(9H,s), 5.27(2H,s), 5.32(2H,s), 7.25-7.45(9H,m), 7.79(1H,s).

Reference Example 69

Production of 4-chloro-2-cyclohexyl-5-(4-phenyl)benzylthio-3(2H)pyridazinone (Compound 70)

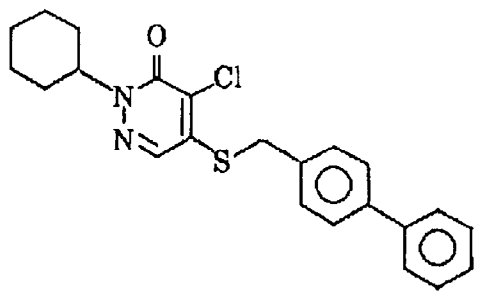

In substantially the same manner as Reference Example 6, Compound 70 (18 mg; yield 54%) was obtained as colorless needles from 4-chloro-2-cyclohexyl-5-mercapto-3(2H)pyridazinone (20 mg), anhydrous sodium carbonate (17 mg) and 4-(chloromethyl) biphenyl (17 mg).
NMR($CDCl_3$) δ: 1.10-1.19(10H,m), 4.32(2H,s), 4.80-4.95(1H,m), 7.30-7.65(9H,m), 7.72(1H,s).

Reference Example 70

Production of 2-t-butyl-4-chloro-5-(3,4-methylenedioxyphenyl)methoxy-3(2H)-pyridazinone (Compound 71)

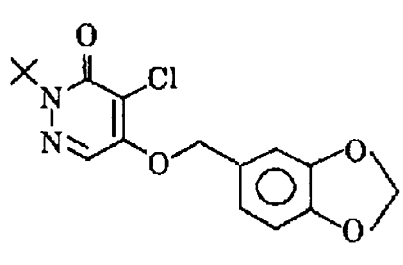

To a solution of 2-t-butyl-4,5-dichloro-3(2H)pyridazinone (220 mg) in DMF (5 ml) were added anhydrous cesium carbonate (485 mg) and piperonyl alcohol (152 mg). The mixture was stirred for two days at room temperature. The reaction mixture was diluted with ethyl acetate, which was washed with a saturated

aqueous solution of sodium chloride, and then dried over anhydrous sodium sulfate. The solvent was distilled off under reduced pressure. The residue was refined by means of silica gel column chromatography (developing solvent: ethyl acetate - hexane = 1:5) to give Compound 71 (225 mg; yield 67%) as colorless needles.
NMR($CDCl_3$) δ: 1.63(9H,s), 5.21(2H,s), 5.90(2H,s), 6.80-6.90(3H,m), 7.22(1H,s).

Reference Example 71

Production of 1-(4-biphenyl)-1,2-ethanediol

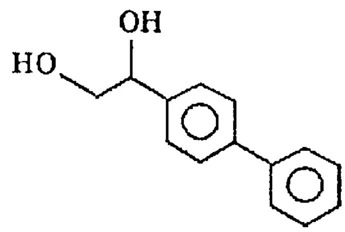

4-Methylmorpholine-N-oxide (1.29 g) was added a mixed solvent of water (25 ml) - acetone (20 ml) - THF (20 ml). To the solution was added osmium tetroxide (1% t-butanol solution; 7 ml), to which was then added 4-vinylphenyl (1.8 g). The mixture was stirred for 8 hours at room temperature, which was then processed with $NaSO_3$. Then, the insolubles were distilled off, and the filtrate was concentrated under reduced pressure. The residue was dissolved in ethyl acetate. The solution was washed with a saturated aqueous solution of sodium chloride, which was dried over anhydrous sodium sulfate. The solvent was distilled off under reduced pressure. The residue was refined by means of silica gel column chromatography (developing solvent: ethyl acetate -hexane = 1:3) to give the above-titled compound (1.89 g; yield 88%).
NMR($CDCl_3$-$CD_3OD$) δ: 3.65(1H,dd,J = 11Hz,8Hz), 3.79(1H,dd,J = 11Hz,3Hz), 4.83(1H,dd,J = 8Hz,3Hz), 7.29-7.64(9H,m).

Reference Example 72

Production of 1-(4-biphenyl)-2-(t-butyldimethylsilyloxy)-ethanol

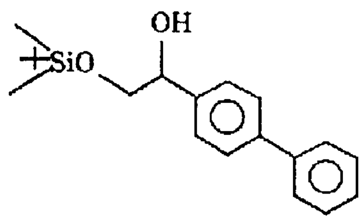

1-(4-Biphenyl)-1,2-ethanediol was dissolved in a mixed solvent of dichloromethane (30 ml) - THF (30 ml). To the solution were added, under ice-cooling, triethylamine (520 mg), t-butyldimethylchlorosilane (775 mg) and dimethylaminopyridine (23 mg). The mixture was warmed up to room temperature, which was stirred overnight. The reaction mixture was diluted with ethyl acetate, which was washed with 1N HCl and a saturated aqueous solution of sodium chloride, followed by drying over anhydrous sodium sulfate. The solvent was then distilled off under reduced pressure. The residue was refined by means of silica gel column chromatography (developing solvent: ethyl acetate - hexane = 1:9) to give the above-titled compound (1.25 g; yield 96%).
NMR($CDCl_3$) δ: 0.01(6H,s), 0.82(9H,s), 2.90(1H,d,J = 2Hz), 3.48(1H,dd,J = 10Hz,9Hz), 3.71-(1H,dd,J = 10Hz,4Hz), 4.70(1H,ddd,J = 9Hz,4Hz,2Hz), 7.25-7.60(9H,m).

Reference Example 73

Production of 3-t-butoxycarbonylamino-6-(4-methylphenyl)pyridine

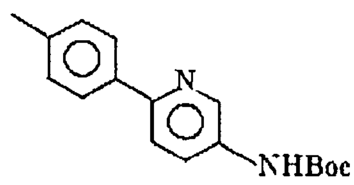

In dichloromethane (10 ml) were dissolved 3-amino-6-(4-methylphenyl) pyridine (350 mg) and dimethylaminopyridine (278 mg). To the solution was added, under ice-cooling, di-t-butyl dicarbonate (497 mg). The mixture was warmed up to room temperature, which was stirred for 5 hours. The reaction mixture was diluted with ethyl acetate, which was washed with a saturated aqueous solution of sodium chloride, followed by drying over anhydrous sodium sulfate. The solvent was distilled off under reduced pressure. The residue was refined by means of silica gel column chromatography (developing solvent: ethyl acetate - hexane = 1:8) to give the above-titled compound (186 mg; yield 34%).
NMR($CDCl_3$) δ: 1.47(9H,s), 2.41(3H,s), 7.28(1H,d,J = 9Hz), 7.64-7.78(2H,m), 7.90(2H,d,J = 9Hz), 8.63(1H,m).

Reference Example 74

Production of 3-t-butoxycarbonylamino-6-(4-bromomethylphenyl)pyridine

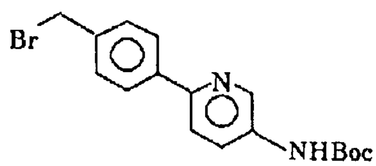

In substantially the same manner as Reference Example 1, the above-titled compound was produced from 3-t-butoxycarbonylamino-6-(4-methylphenyl)pyridine (175 mg), N-bromosuccinimide (121 mg) and benzoyl peroxide (15 mg).
NMR($CDCl_3$) δ: 1.47(9H,s), 4.55(2H,s), 7.51(2H,d,J = 8Hz), 7.66-7.78(2H,m), 8.00(2H,d,J = 8Hz), 8.66(1H,m).

Reference Example 75

Production of 2-t-butoxycarbonylamino-4-(4-methylphenyl)pyridine

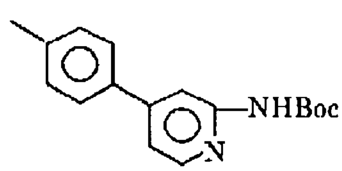

2-Amino-4-(4-methylphenyl)pyridine (1.87 g) was dissolved in anhydrous THF (70 ml). To the solution was added di-t-butyl dicarbonate (2.32 g), and the mixture was refluxed for 3 hours under heating. The reaction mixture was cooled to room temperature, then the organic layer was washed with a saturated aqueous solution of sodium hydrogencarbonate and a saturated aqueous solution of sodium chloride, followed by drying over anhydrous magnesium sulfate. The solvent was distilled off under reduced pressure. The residue was refined by means of silica gel column chromatography (carrier 50 g; developing

solvent; dichloromethane) to give the above-titled compound (1.01 g).
NMR($CDCl_3$) δ: 1.55(9H,s), 2.40(3H,s), 7.18(1H,dd,J = 5Hz,2Hz), 7.26(2H,d,J = 8Hz), 7.59(2H,d,J = 8Hz), 7.84-(1H,brs), 8.21(1H,s), 8.26(1H,d,J = 5Hz).

Reference Example 76

Production of 2-t-butoxycarbonylamino-4-(4-bromomethylphenyl)pyridine

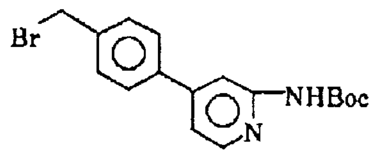

In substantially the same manner as Reference Example 1, the above-titled compound (1.23 g) was produced from 2-t-butoxycarbonylamino-4-(4-bromomethylphenyl)pyridine (971 mg), N-bromosuccinimide (704 mg) and benzoyl peroxide (26 mg).
NMR($CDCl_3$) δ: 1.55(9H,s), 4.54(2H,s), 7.18(1H,dd,J = 5Hz,2Hz), 7.47-7.69(4H,m), 8.22(1H,s), 8.28-(1H,d,J = 5Hz).

Reference Example 77

Production 2-(4-bromomethylphenyl)pyrimidine

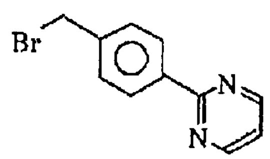

In substantially the same manner as Reference Example 1, the above-titled compound (454 mg) was produced from 2-(4-methylphenyl)pyrimidine (300 mg), N-bromosuccinimide (345 mg) and benzoyl peroxide (12 mg).
NMR($CDCl_3$) δ: 4.56(3H,s), 7.20(1H,t,J = 5Hz), 7.52(2H,d,J = 8Hz), 8.43(2H,d,J = 8Hz), 8.81(2H,d,J = 5Hz).

Reference Example 78

Production of 2-(4-bromomethylphenyl)-4,6-dichloropyrimidine

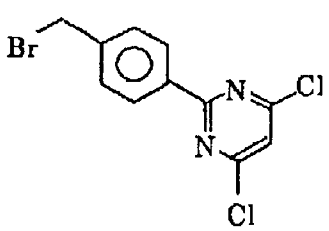

In substantially the same manner as Reference Example 1, the above-titled compound (515 mg; yield 77%) was produced from 4,6-dichloro-2-(4-methylphenyl)pyrimidine (500 mg), N-bromosuccinimide (400 mg) and benzoyl peroxide (15 mg).
NMR($CDCl_3$) δ: 4.54(3H,s), 7.29(1H,s), 7.52(2H,d,J = 8Hz), 8.42(2H,d,J = 8Hz).

Reference Example 79

Production of 2-bromomethylphenyl)-4,6-dimethoxypyrimidine

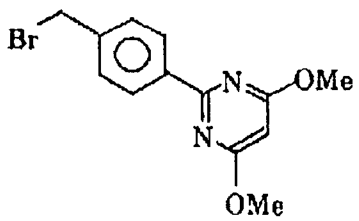

In substantially the same manner as Reference Example 1, the above-titled compound (425 mg; yield 63%) was produced from 4,6-dimethoxy-2-(4-methylphenyl)pyrimidine (500 mg), N-bromosuccimide (425 mg) and benzoyl peroxide (16 mg).
NMR($CDCl_3$) δ: 4.05(6H,s), 4.55(3H,s), 5.98(1H,s), 7.48(2H,d,J = 8Hz), 8.43(2H,d,J = 8Hz).

Reference Example 80

Production of 2-bromo-4'-bromomethylacetophenone

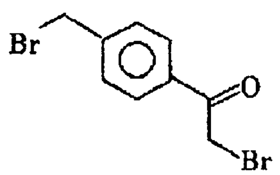

In substantially the same manner as Reference Example 1, the above-titled compound (281 mg; yield 85%) was produced from 2-bromo-4'-methylacetophenone (200 mg), N-bromosuccimide (184 mg) and benzoyl peroxide (7 mg).
NMR($CDCl_3$) δ: 4.44(3H,s), 4.51(3H,s), 7.52(2H,d,J = 9Hz), 7.97(2H,d,J = 9Hz).

Reference Example 81

Production of 2-amino-4-(4-bromomethylphenyl)thiazole • hydrobromide

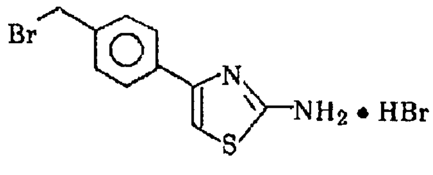

In ethanol (4 ml) were dissolved 2-bromo-4'-bromomethylacetophenone (200 mg) and thiourea (52 mg). The solution was refluxed for 30 minutes under heating. The reaction mixture was cooled to room temperature, then the solvent was distilled off under reduced pressure to give a crude product of the above-titled compound. This crude product was used for the subsequent reaction without purification.
NMR($CDCl_3$) δ: 4.52(2H,s), 7.19(1H,s), 7.50(2H,d,J = 8Hz), 7.76(2H,d,J = 8Hz), 8.95-9.25(3H,m).

Reference Example 82

Production of N-(triphenylmethyl)-5-(4-methylphenyl)-1,2,4-triazole

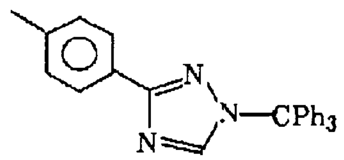

To a solution of 5-(4-methylphenyl)-1,2,4-triazole (1.60 g) in DMF (20 ml) were added triethylamine (1.54 g) and triphenylmethyl chloride (3.08 g). The mixture was stirred for 4 hours at room temperature, to which was added water. The resulting precipitate was collected by filtration, which was washed with water, a small quantity of methanol and ether, successively, followed by drying over phosphorus pentaoxide at 60 °C to give the above-titled compound (3.28 g; yield 81%).
NMR(DMSO-$d_6$) δ: 2.34(3H,s), 7.10-7.16(6H,m), 7.25(2H,d,J=8Hz), 7.36-7.44(9H,m), 7.85(2H,d,J=8Hz), 8.15(1H,s).
IR(KBr): 3050, 3010, 1590, 1480, 1440 $cm^{-1}$

Reference Example 83

Production of N-(triphenylmethyl)-5-(4-bromomethylphenyl)-1,2,4-triazole

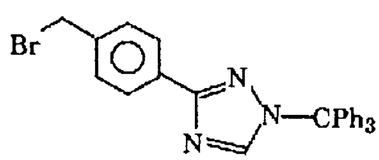

A mixture of N-(triphenylmethyl)-5-(4-methylphenyl)-1,2,4-triazole (3.20 g), N-bromosuccinimide (1.56 g) and benzoyl peroxide (135 mg) was heated under reflux for 4.5 hours in carbon tetrachloride (100 ml). The reaction mixture was cooled to room temperature. The insolubles were then filtered off. The filtrate was subjected to distillation under reduced pressure to leave the above-titled compound (3.83 g; yield 100%).
NMR(DMSO-$d_6$) δ: 4.74(2H,s), 7.10-7.17(6H,m), 7.35-7.46(9H,m), 7.52(2H,d,J=8Hz), 7.95(2H,d,J=8Hz), 8.20(1H,s).

Reference Example 84

Production of N-(triphenylmethyl)-3-(4-methylphenyl)pyrazole

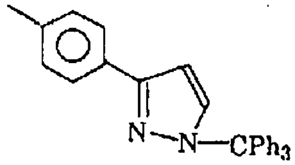

In substantially the same manner as Reference Example 82, the above-titled compound (6.99 g; yield 87%) was produced from 3-(4-methylphenyl)-pyrazole (3.16 g).
NMR(DMSO-$d_6$) δ: 2.30(3H,s), 6.73(1H,d,J=3Hz), 7.09-7.23(6H,m), 7.14(2H,d,J=8Hz), 7.33-7.39(10H,m), 7.63(2H,d,J=8Hz).

Reference Example 85

Production of N-(triphenylmethyl)-3-(4-bromomethylphenyl)pyrazole

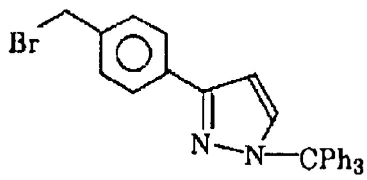

In substantially the same manner as Reference Example 83, the above-titled compound (8.15 g; yield 100%) was produced from N-(triphenylmethyl)-3-(4-methylphenyl)-pyrazole (6.81 g)
NMR(DMSO-$d_6$) δ: 4.71(2H,s), 6.80(1H,d,J = 3Hz), 7.09-7.15(8H,m), 7.33-7.48(10H,m), 7.74(2H,d,J = 8Hz).

Reference Example 86

Production of 5-(4-bromomethylphenyl)-1,2-oxazole

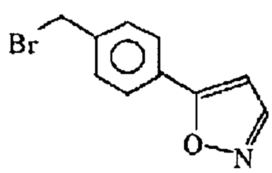

In substantially the same manner as Reference Example 83, the above-titled compound (2.61 g; yield 100%) was produced from 5-(4-methylphenyl)-1,2-oxazole (1.59 g).
NMR($CDCl_3$) δ: 4.52(2H,s), 6.55(1H,d,J = 2Hz), 7.51(2H,d,J = 8Hz), 7.79(2H,d,J = 8Hz),8.31(1H,d,J = 2Hz).

Reference Example 87

Production of 5-(4-bromomethylphenyl)-1,2,4-thiadiazole

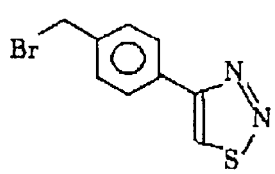

In substantially the same manner as Reference Example 83, the above-titled compound (1.59 g; yield 42%) was produced from 5-(4-methylphenyl)-1,2,4-thiadiazole (2.64 g).
NMR($CDCl_3$) δ: 4.53(2H,s), 7.54(1H,d,J = 8Hz), 7.94(2H,d,J = 8Hz), 8.72(1H,s).

Reference Example 88

Production of 5-(4-bromomethylphenyl)-1,3-oxazole

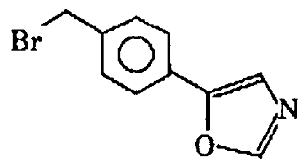

In substantially the same manner as Reference Example 83, the above-titled compound (1.42 g; yield 59%) was produced from 5-(4-methylphenyl)-1,3-oxazole (1.59 g).
NMR($CDCl_3$) δ: 4.52(2H,s), 7.39(1H,s), 7.47(2H,d,J = 8Hz), 7.65(2H,d,J = 8Hz), 7.94(1H,s).

Reference Example 89

Production of 2-t-butyl-4-chloro-5-[4-(4-chlorobenzoyl)benzylthio]-3(2H)-pyridazinone (Compound 72)

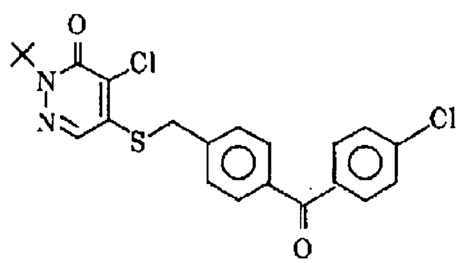

In substantially the same manner as Reference Example 6, Compound 72 (729 mg; yield 65%) was produced as pale yellow crystals by reacting 2-t-butyl-4-chloro-5-mercapto-3(2H)-pyridazinone (547 mg) with 4-(4-chlorobenzoyl)benzyl bromide (851 mg), and then by recrystallizing the product from chloroform-ether.
NMR($CDCl_3$) δ: 1.63(9H,s), 4.34(2H,s), 7.45(2H,d,J = 8Hz), 7.55(2H,d,J = 8Hz), 7.59(1H,s), 7.75(2H,d,J = 8Hz), 7.79(2H,d,J = 8Hz).
IR(KBr): 3100, 2980, 2930, 1640, 1600, 1580, 1560, 1495, 1460 $cm^{-1}$.

| Elemental Analysis for $C_{22}H_{20}Cl_2N_2O_2S$: | | | |
|---|---|---|---|
| Calcd.: | C:59.06%; | H:4.51%; | N:6.26% |
| Found : | C:58.77%; | H:4.51%; | N:6.21% |

Reference Example 90

Production of 2-t-butyl-4-chloro-5-[4-(4-chlorobenzoyl)benzyloxy]-3(2H)-pyridazinone (Compound 73)

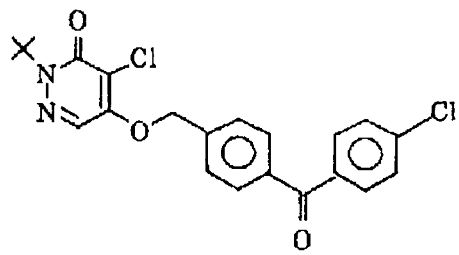

In substantially the same manner as Reference Example 23, 2-t-butyl-4-chloro-5-hydroxy-3(2H)-pyridazinone (608 mg) was reacted with 4-(4-chlorobenzoyl)benzyl bromide (1.02 mg), followed by recrystallization of the reaction product from chloroform-ether to give Compound 73 (791 mg; yield 61%) as colorless crystals.
NMR($CDCl_3$) δ: 1.65(9H,s), 5.40(2H,s), 7.48(2H,d,J = 8Hz), 7.56(2H,d,J = 8Hz), 7.74(1H,s), 7.77(2H,d,J = 8Hz), 7.84(2H,d,J = 8Hz).
IR(KBr): 3050, 2960, 2940, 1660, 1640, 1600, 1580, 1560, 1495, 1460 $cm^{-1}$.

| Elemental Analysis for $C_{22}H_{20}Cl_2N_2O_3$: | | | |
|---|---|---|---|
| Calcd.: | C:61.26%; | H:4.67%; | N:6.49% |
| Found : | C:60.87%; | H:4.56%; | N:6.61% |

Reference Example 91

Production of 5-(4-benzyloxybenzylthio)-2-t-butyl-4-chloro-3(2H)-pyridazinone (Compound 74)

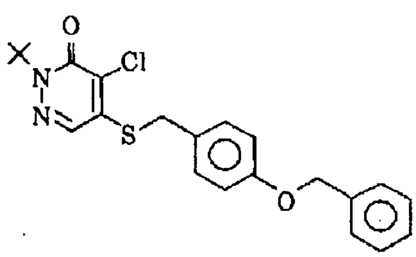

In substantially the same manner as Reference Example 6, 2-t-butyl-4-chloro-5-mercapto-3(2H)-pyridazinone (547 mg) was reacted with 4-benzyloxybenzylchloride (640 mg), followed by triturating the reaction product from hexane to give Compound 74 as a white powdery product (968 mg; yield 93%).
NMR($CDCl_3$) δ: 1.63(9H,s), 4.23(2H,s), 5.07(2H,s), 6.97(2H,d,J = 9Hz), 7.32(2H,d,J = 9Hz), 7.34-7.45(5H,m), 7.62(1H,s).
IR(KBr): 2970, 2925, 2850, 1650, 1645, 1600, 1580, 1560, 1505 $cm^{-1}$.

| Elemental Analysis for $C_{22}H_{23}ClN_2O_2S$: | | | |
|---|---|---|---|
| Calcd.: | C:63.68%; | H:5.59%; | N:6.75% |
| Found : | C:63.69%; | H:5.74%; | N:6.86% |

Reference Example 92

Production of 5-(4-benzyloxybenzyloxy)-2-t-butyl-4-chloro-3(2H)-pyridazinone (Compound 75)

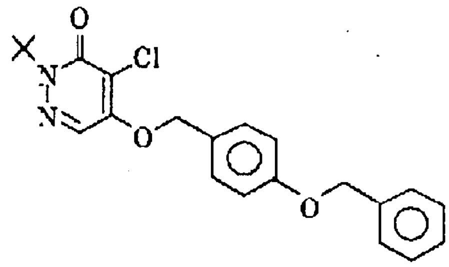

In substantially the same manner as Reference Example 23, 2-t-butyl-4-chloro-5-hydroxy-3(2H)-pyridazinone (608 mg) was reacted with 4-benzyloxybenzyl chloride (768 mg), followed by recrystallization of the reaction product from chloroform-ether to give Compound 75 (473 mg; yield 40%) as a colorless crystalline product.
NMR($CDCl_3$) δ: 1.63(9H,s), 5.08(2H,s), 5.25(2H,s), 7.01(2H,d,J=9Hz), 7.34(2H,d,J=9Hz), 7.30-7.48(5H,m), 7.74(1H,s).
IR(KBr): 3040, 2960, 2940, 1645, 1600, 1585, 1510 $cm^{-1}$.

| Elemental Analysis for $C_{22}H_{23}ClN_2O_3$: | | | |
|---|---|---|---|
| Calcd.: | C:66.24%; | H:5.81%; | N:7.02% |
| Found : | C:65.84%; | H:5.84%; | N:7.30% |

Reference Example 93

Production of 5-(4-t-butylbenzylthio)-4-chloro-2-methyl-3(2H)-pyridazinone (Compound 76)

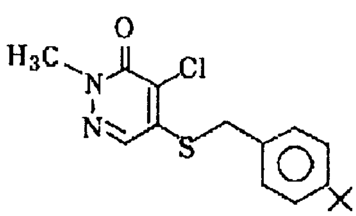

In substantially the same manner as Reference Example 6, 4-chloro-2-methyl-5-mercapto-3(2H)-pyridazinone (361 mg) was reacted with 4-t-butylbenzyl chloride (502 mg), followed by purification of the reaction product by means of silica gel column chromatography (developing solvent: hexane - ethyl acetate = 5:1) to give Compound 76 (498 mg; yield 62%) as a colorless powdery product.
NMR($CDCl_3$) δ: 1.31(9H,s), 3.78(3H,s), 4.26(2H,s), 7.33(2H,d,J=8Hz), 7.39(2H,d,J=8Hz), 7.65(1H,s).
IR(KBr): 3050, 2955, 2900, 2860, 1640, 1560, 1505 $cm^{-1}$.

| Elemental Analysis for $C_{16}H_{19}ClN_2OS$: | | | |
|---|---|---|---|
| Calcd.: | C:59.52%; | H:5.93%; | N:8.63% |
| Found : | C:59.40%; | H:5.70%; | N:8.69% |

Reference Example 94

Production of 5-(4-t-butylbenzyloxy)-4-chloro-2-methyl-3(2H)-pyridazinone (Compound 77)

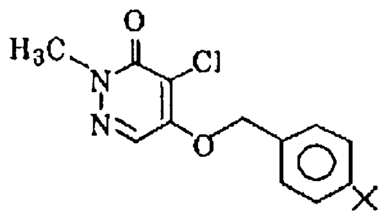

To a solution of 4,5-dichloro-2-methyl-3(2H)-pyridazinone (1.00 g) in DMF (10 ml) were added anhydrous cesium carbonate (1.09 g) and 4-t-butylbenzyl alcohol (1.10 g). The mixture was stirred for 24 hours at 80 °C. The reaction mixture was cooled to room temperature, to which was added water, followed by extraction with ethyl acetate. The extract solution was washed with a saturated aqueous solution of sodium chloride, followed by drying over anhydrous magnesium sulfate. The solvent was distilled off under reduced pressure, and the residue was refined by means of silica gel chromatography (developing solvent: hexane - ethyl acetate = 9:1 ~ 4:1) to afford Compound 77 (580 mg; yield 34%) as a colorless powdery product.
NMR($CDCl_3$) δ: 1.32(9H,s), 3.79(3H,s), 5.30(2H,s), 7.33(2H,d,J = 8Hz), 7.43(2H,d,J = 8Hz), 7.76(1H,s).
IR(KBr): 2960, 2950, 2900, 2860, 1650, 1640, 1600, 1520 $cm^{-1}$.
m.p.: 119-120 °C

| Elemental Analysis for $C_{16}H_{19}ClN_2O_2$: | | | |
|---|---|---|---|
| Calcd.: | C:62.64%; | H:6.24%; | N:9.13% |
| Found : | C:62.66%; | H:6.14%; | N:9.32% |

Reference Example 95

Production of 5-(4-t-butylbenzylthio)-4-chloro-2-(2-propyl)-3(2H)-pyridazinone (Compound 78)

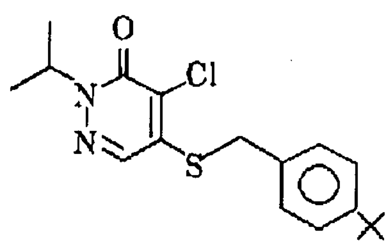

To a solution of 4,5-dichloro-2-(2-propyl)-3(2H)-pyridazinone (621 mg) in DMF (5 ml) were added anhydrous cesium carbonate (977 mg) and 4-t-butylbenzyl thiol (541 mg). The mixture was stirred for one hour at 60 °C. The reaction mixture was cooled to room temperature, to which was added water, followed by extraction with ethyl acetate. The extract was washed with a saturated aqueous solution of sodium chloride, followed by drying over anhydrous magnesium sulfate. The solvent was then distilled off under reduced pressure. The residue was refined by means of silica gel column chromatography (developing solvent: hexane - ethyl acetate = 19:1) to give Compound 78 (582 mg; yield 55%) as a colorless powdery product.
NMR($CDCl_3$) δ: 1.32(9H,s), 1.33(6H,d,J = 6.6Hz), 4.26(2H,s), 5.27(1H,quintet,J = 7Hz), 7.32(2H,d,J = 8Hz), 7.39(2H,d,J = 8Hz), 7.73(1H,s).
IR(KBr): 3050, 2950, 2940, 2860, 1645, 1560, 1505 $cm^{-1}$.

| Elemental Analysis for $C_{18}H_{23}ClN_2OS$: | | | |
|---|---|---|---|
| Calcd.: | C:61.61%; | H:6.61%; | N:7.98% |
| Found : | C:61.71%; | H:6.48%; | N:8.17% |

Reference Example 96

Production of 5-(4-t-butylbenzyloxy)-4-chloro-2-(2-propyl)-3(2H)-pyridazinone (Compound 79)

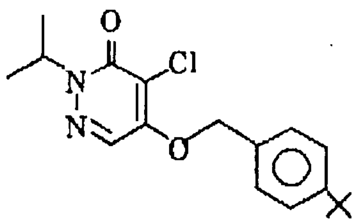

In substantially the same manner as Reference Example 94, Compound 79 (588 mg; yield 50%) was produced as a powdery product from 4,5-dichloro-2-(2-propyl)-3(2H)-pyridazinone (725 mg) and 4-t-butylbenzyl alcohol (690 mg).
NMR($CDCl_3$) δ: 1.33(9H,s), 1.33(6H,d,J=7Hz), 5.29(2H,s), 5.31(1H,quintet,J=7Hz), 7.34(2H,d,J=8Hz), 7.44-(2H,d,J=8Hz), 7.84(1H,s).
IR(KBr): 3050, 2960, 2900, 2860, 1630, 1590, 1510 $cm^{-1}$.

| Elemental Analysis for $C_{18}H_{23}ClN_2O_2$: | | | |
|---|---|---|---|
| Calcd.: | C:64.57%; | H:6.92%; | N:8.37% |
| Found : | C:64.28%; | H:6.81%; | N:8.45% |

Reference Example 97

Production of 4,5-dichloro-2-methoxymethyl-3(2H)-pyridazinone

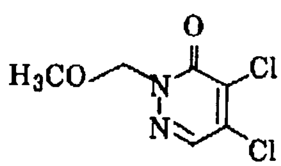

Sodium hydride (60% oil; 880 mg) was washed with hexane, to which was added DMF (10 ml). The mixture was cooled to 0°C, to which was added dropwise a solution of 4,5-dichloro-3-hydroxypyridazine (3.30 g) in DMF (25 ml). The mixture was stirred for 15 minutes at 0°C, to which was added a solution of chloromethyl ether (1.77 g) in DMF (5 ml). The reaction mixture was stirred for 2.5 hours at room temperature, followed by addition of water and extraction with ethyl acetate. The extract solution was washed with a saturated aqueous solution of sodium chloride and, then, dried over anhydrous magnesium sulfate. The solvent was distilled off under reduced pressure. The residue was refined by means of silica gel column chromatography (developing solvent: hexane - ethyl acetate = 5:1) to give the above-titled compound (3.06 g; yield 73%) as a colorless powdery product.
NMR($CDCl_3$) δ: 3.49(3H,s), 5.47(2H,s), 7.82(1H,s).
IR(KBr): 3090, 3050, 3000, 2950, 2820, 1655, 1580, 1505 $cm^{-1}$.

Reference Example 98

Production of 5-(4-t-butylbenzyloxy)-4-chloromethoxymethyl-3(2H)-pyridazinone (Compound 80)

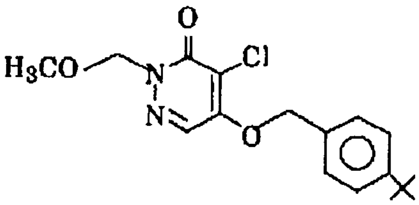

In substantially the same manner as Reference Example 94, Compound 80 (619 mg; yield 55%) was produced as a colorless product from 4,5-dichloro-2-methoxymethyl-3(2H)-pyridazinone (700 mg) and 4-t-butylbenzyl alcohol (660 mg).
NMR($CDCl_3$) $\delta$: 1.33(9H,s), 3.45(3H,s), 5.33(2H,s), 5.45(2H,s), 7.33(2H,d,J=9Hz), 7.44(2H,d,J=9Hz), 7.84-(1H,s),
IR(KBr): 2960, 2940, 1660, 1600 $cm^{-1}$.

| Elemental Analysis for $C_{17}H_{21}ClN_2O_3$: | | | |
|---|---|---|---|
| Calcd.:<br>Found : | C:60.62%;<br>C:60.52%; | H:6.28%;<br>H:6.25%; | N:8.32%<br>N:8.15% |

Reference Example 99

Production of 4,5-dichloro-2-(2-methoxyethoxymethyl)-3(2H)-pyridazinone

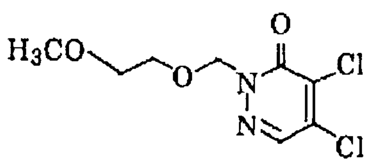

In substantially the same manner as Reference Example 97, the above-titled compound (3.57 g; yield 71%) was produced as a colorless powdery product from 4,5-dichloro-3-hydroxypyridazine (3.30 g) and $\beta$-methoxyethoxymethyl chloride (2.74 g).
NMR($CDCl_3$) $\delta$: 3.36(3H,s), 3.51-3.56(2H,m), 3.81-3.86(2H,m), 5.57(2H,s), 7.81(1H,s).
IR(KBr): 3100, 3050, 2940, 2880, 1670, 1580 $cm^{-1}$.

Reference Example 100

Production of 5-(4-t-butylbenzyloxy)-4-chloro-2-(2-methoxyethoxymethyl)-3(2H)-pyridazinone (Compound 81)

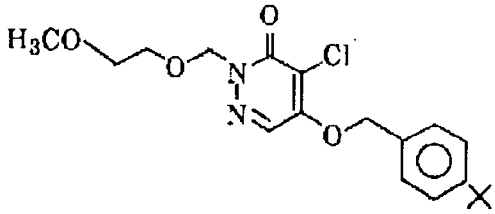

In substantially the same manner as Reference Example 94, Compound 81 (1.06 g; yield 56%) was produced as a colorless powdery product from 4,5-dichlro-2-(2-methoxyethoxymethyl)-3(2H)-pyridazinone (1.27 g) and 4-t-butylbenzyl alcohol (986 mg).
NMR($CDCl_3$) δ: 1.33(9H,s), 3.34(3H,s), 3.49-3.54(2H,m), 3.79-3.84(2H,m), 5.33(2H,s), 5.55(2H,s), 7.33-(2H,d,J = 8Hz), 7.44(2H,d,J = 8Hz), 7.83(1H,s).
$^1$R(KBr): 2955, 2860, 2805, 1650, 1600, 1515 $cm^{-1}$.

| Elemental Analysis for $C_{19}H_{25}ClN_2O_4$: | | | |
|---|---|---|---|
| Calcd.: | C:59.52%; | H:6.62%; | N:7.36%, |
| Found : | C:59.76%; | H:6.59%; | N:7.32%. |

Working Example 1

Production of 2-t-butyl-4-chloro-5-{4-(2-pyridyl) benzylthio}-3(2H)-pyridazinone (Compound 39)

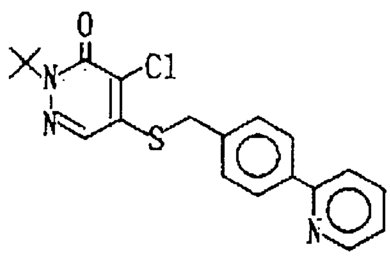

To a solution of 2-t-butyl-4-chloro-5-mercapto-3(2H)-pyridazinone (400 mg) in DMF (5 ml) were added anhydrous potassium carbonate (379 mg) and 4-(2-pyridyl)benzyl bromide (500 mg). The mixture was stirred for 3.5 hours at 100 ° C. The reaction mixture was cooled to room temperature, to which was added water, followed by extraction with ethyl acetate. The extract solution was washed with a saturated aqueous saline solution, dried over anhydrous magnesium sulfate, and then the solvent was distilled off under reduced pressure. The residue was purified by means of silica gel column chromatography (carrier 40 g; developing solvent: ethyl acetate - hexane = 1:5), followed by recrystallization from methanol to give Compound 39 (492 mg: yield 70%) as pale yellow prisms.
NMR(DMSO-$d_6$) δ: 1.56(9H,s), 4.61(2H,s), 7.32-7.38(1H,m), 7.59(2H,d,J = 8.4Hz), 7.84-7.99(2H,m), 8.07-(1H,s), 8.09(2H,d,J = 8.4Hz), 8.65-8.68(1H,m).
IR(KBr): 2980, 2940, 1640, 1590, 1530 $cm^{-1}$.
m.p.: 143-144 ° C

| Elemental Analysis for $C_{20}H_{20}ClN_3O$: | | | |
|---|---|---|---|
| Calcd.: | C:60.35%; | H:5.79%; | N:10.05% |
| Found : | C:60.46%; | H:5.50%; | N:10.29% |

Working Example 2

Production of 2-t-butyl-4-chloro-5-[4-{N-(triphenylmethyl)tetrazol-5-yl}benzylthio]-3(2H)-pyridazinone (Compound 40)

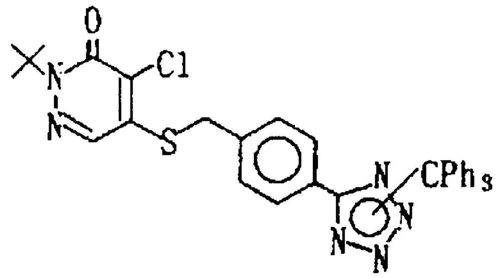

In substantially the same manner as Working Example 1, from 2-t-butyl-4-chloro-5-mercapto-3(2H)-pyridazinone (620 mg), anhydrous potassium carbonate (587 mg) and N-(triphenylmethyl)-5-(4-bromomethylphenyl)tetrazole (1.50 g), Compound 40 (808 mg: yield 47%) was obtained as a white powdery product.
NMR(DMSO-$d_6$) δ: 1.55(9H,s), 4.63(2H,s), 7.05-7.13(6H,m), 7.38-7.46(9H,m), 7.65(2H,d,J=8.0Hz), 8.03-(2H,d,J=8.0Hz), 8.04(1H,s).
IR(KBr): 3050, 2980, 2930, 1735, 1640, 1590, 1560, 1490,1465, 1440, 1420$cm^{-1}$.
m.p.: 188-189 °C

Working Example 3

Production of 2-t-butyl-4-chloro-5-{4-(1H-tetrazol-5-yl)benzylthio}-3(2H)-pyridazinone (Compound 41)

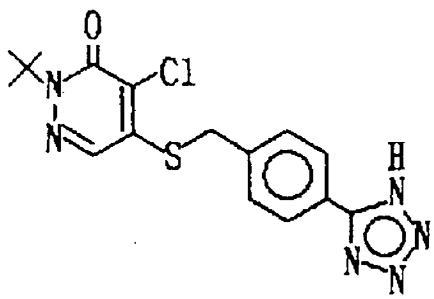

In a mixture of 10% HCl (5 ml) and tetrahydrofuran (10 ml) was dissolved 2-t-butyl-4-chloro-5-{4-[N-(triphenylmethyl)tetrazol-5-yl}benzylthio]-3(2H)-pyridazinone (450 mg). The solution was stirred for 5.5 hours at room temperature. To the reaction mixture was added a 10% aqueous solution of sodium hydroxide (5 ml), followed by distilling off the solvent under reduced pressure. To the residue was added water, then the insolubles were filtered off. To the filtrate was added hydrochloric acid to adjust its pH to 3. The resulting precipitate was recovered by filtration, which was washed with a small volume of water, methanol and ether in sequence, followed by drying over phosphorus pentoxide at 50 °C under reduced pressure to give Compound 41 (79 mg: yield 28%) as a white powdery product.
NMR(DMSO-$d_6$) δ: 1.56(9H,s), 4.65(2H,s), 7.71(2H,d,J=8.0Hz), 8.04(2H,d,J=8.0(1H,s), 8.06(1H,s).
m.p.: 258-259 °C

Working Example 4

Production of 2-t-butyl-4-chloro-5-{4-(2-pyridyl) benzyloxy}-3(2H)-pyridazinone (Compound 42)

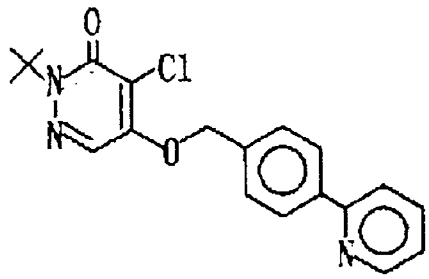

To a solution of 2-t-butyl-4-chloro-5-hydroxy-3(2H)pyridazinone (411 mg) in DMF (5 ml) were added anhydrous potassium carbonate (420 mg) and 4-(2-pyridyl) benzylbromide (554 mg). The mixture was stirred for 3 hours at 120 °C. The reaction mixture was cooled to room temperature, to which was added water, followed by extraction with ethyl acetate. The extract solution was washed with a saturated aqueous saline solution, dried over anhydrous magnesium sulfate, followed by distilling off the solvent under reduced pressure. The residue was purified by means of silica gel column chromatography (carrier 40 g; developing solvent: ethyl acetate - hexane = 1:5), followed by recrystallization from methanol to give Compound 42 (261 mg: yield 35%) as colorless needles.

NMR(DMSO-$d_6$) δ: 1.58(9H,s), 5.53(2H,s), 7.37(1H,ddd,J = 1.4,4.8,7.5Hz), 7.58(2H,d,J = 8.2Hz), 7.89-(1H,dt,J = 1.8,7.5Hz), 8.15(2H,d,J = 8.2Hz), 8.29(1H,s), 8.68(1H,dm,J = 4.8Hz).

IR(KBr): 3100, 3060, 2995, 2940, 1630, 1600, 1580, 1560, 1500 $cm^{-1}$.

m.p.: 164-165 °C

| Elemental Analysis for $C_{20}H_{20}ClN_3O_2$: | | | |
|---|---|---|---|
| Calcd.:<br>Found : | C:64.95%;<br>C:65.07%; | H:5.45%;<br>H:5.51%; | N:11.36%<br>N:11.33% |

Working Example 5

Production of 2-t-butyl-4-chloro-5-[4-{N-(triphenylmethyl)tetrazol-5-yl)benzyloxy]-3(2H)-pyridazinone (Compound 43)

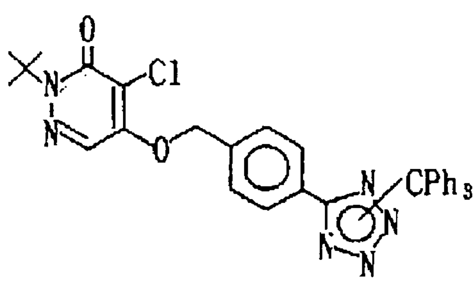

In substantially the same manner as in Working Example 4, from 2-t-butyl-4-chloro-5-hydroxy-3(2H)-pyridazinone (574 mg), anhydrous potassium carbonate (1.27 g) and N-(triphenylmethyl)-5-(4-bromomethylphenyl) tetrazole (1.50 g), Compound 43 (131 mg: yield 8%) was produced as a pale yellow powdery compound.

NMR(DMSO-$d_6$) δ: 1.57(9H,s), 5.55(2H,s), 7.07-7.15(6H,m), 7.38-7.46(9H,m), 7.64(2H,d,J = 8.2Hz), 8.10-(2H,d,J = 8.2Hz), 8.26(1H,s)

IR(KBr): 3050, 2970, 1740, 1635, 1600, 1490, 1470, 1455, 1440, 1405 $cm^{-1}$.

m.p.: 200-201 °C

Working Example 6

Production of 2-t-butyl-4-chloro-5-{4-(1H-tetrazol-5-yl)benzyloxy}-3(2H)-pyridazinone (Compound 44)

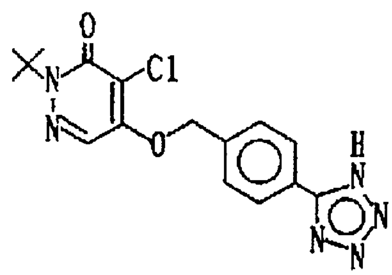

In substantially the same manner as Working Example 3, from 2-t-butyl-4-chloro-5-[4-{N-(triphenylmethyl)tetrazol-5-yl}benzyloxy]-3(2H)-pyridazinone (100 mg), Compound 45 (28 mg: yield 46%) was produced as a white powdery compound.
NMR(DMSO-$d_6$) δ: 1.58(9H,s), 5.60(2H,s), 7.68(2H,d,J = 8.0Hz), 8.11(2H,d,J = 8.0Hz), 8.28(1H,s)
m.p.: 247-249 °C

Working Example 7

Production of 2-t-butyl-4-chloro-5-(2-hydroxy-2-phenyl) ethoxy-3(2H)-pyridazinone (Compound 46) and 2-t-butyl-4-chloro-5-(2-hydroxy-1-phenyl)ethoxy-3(2H)-pyridazinone (Compound 47)

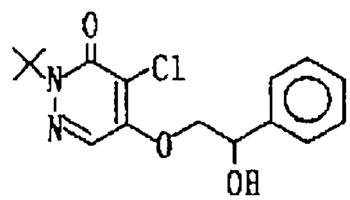

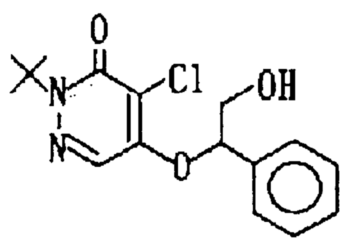

To a solution of 2-t-butyl-4,5-dichloro-3(2H)-pyridazinone (440 mg) and 2-hydroxy-2-phenylethanol (1.38 g) in DMF (100 ml) was added anhydrous cesium carbonate (1.95 mg), then the mixture was stirred overnight. The reaction mixture was diluted with ether, washed with a saturated aqueous saline solution and, then, dried over anhydrous magnesium sulfate. The solvent was distilled off under reduced pressure. The residue was subjected to a silica gel column chromatography (carrier 40 g), eluting with hexane - ethyl acetate = 4:1 to give Compound 46 (210 mg) and, further eluting with hexane - ethyl acetate = 3:1 to give Compound 47 (125 mg), as colorless oily products, respectively.
Compound 46
NMR($CDCl_3$) δ: 1.64(9H,s), 2.72(1H,br.d,J = 3.0Hz), 4.26(1H,dd,J = 8.0Hz,10.0Hz), 4.34-(1H,dd,J = 3.5Hz,10.0Hz), 5.10-5.25(1H,m), 7.35-7.50(5H,m), 7.73(1H,s).
Compound 47
NMR($CDCl_3$) δ: 1.59(9H,s), 2.34(1H,m), 3.80-4.05(2H,m), 5.43(1H,dd,J = 3.5Hz,8.0Hz), 7.30-7.50(5H,m), 7.54-

(1H,s).

Working Example 8

Production of 2-t-butyl-4-chloro-5-(2-hydroxy-1-phenyl) ethylthio-3(2H)-pyridazinone (Compound 48)

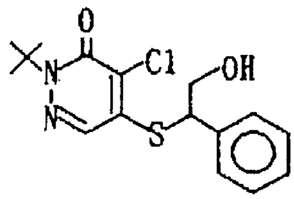

To a solution of 2-(t-butyldimethylsilyloxy)-1-phenylethanol (1.27 g) and triethylamine (0.96 ml) in DMF (5 ml) was added dropwise, under ice-cooling, methanesulfonyl chloride (0.42 ml). The mixture was stirred for 30 minutes, then, the resulting precipitate was filtered off to give a solution of 2-(t-butyldimethylsilyloxy)-1-methanesulfonyloxy-1-phenylethane in DMF.

To a solution of 2-t-butyl-4-chloro-5-mercapto-3(2H)-pyridazinone (1.00 g) in DMF (5 ml) was gradually added, under ice-cooling, sodium hydride (60% in oil: 274 mg), and the mixture was stirred for 10 minutes. To the reaction mixture was added the above mentioned solution of 2-(t-butyldimethylsilyloxy)-1-methansulfonyloxy-1-phenylethane in DMF. The mixture was stirred for 2 hours at room temperature. To the reaction mixture was added water, then the resulting product was extracted with ether. The extract solution was washed with water and dried over anhydrous magnesium sulfate. The solvent was distilled off under reduced pressure to leave a crude product of 2-t-butyl-4-chloro-5-[2-(t-butyldimethylsilyloxy)-1-phenyl]-ethylthio-3(2H)-pyridazinone. This crude product was dissolved in THF (10 ml), to which was added, under ice-cooling, tetrabutyl ammonium fluoride (1M THF solution: 8.5 ml). The mixture was stirred for 15 minutes. To the reaction mixture was added water, then the resulting product was extracted with ether. The extract solution was washed with water and dried over anhydrous magnesium sulfate, then the solvent was distilled off under reduced pressure. The residue was purified by means of silica gel column chromatography (carrier: 50 g; ethyl acetate - hexane = 1:2), followed by recrystallization from ethyl acetate - hexane to give Compound 48 (268 mg) as colorless needles.

NMR($CDCl_3$) δ: 1.60(9H,s), 1.89(1H,br.t,J = 6.5Hz), 3.90-4.15(2H,m), 4.64(1H,t,J = 6.5Hz), 7.25-7.50(5H,m), 7.60(1H,s).

Working Example 9

Production of 2-t-butyl-4-chloro-5-[2-hydroxy-2-(4-biphenyl)]ethoxy-3(2H)-pyridazinone (Compound 82) and 2-t-butyl-4-chloro-5-[2-hydroxy-1-(43-biphenyl)]ethoxy-3(2H)-pyridazinone (Compound 83)

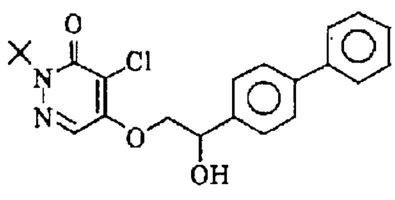

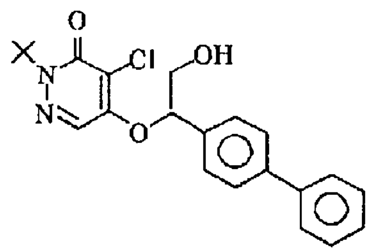

In substantially the same manner as Working Example 7, Compound 82 (68 mg; yield 40%) and Compound 83 (41 mg; yield 24%) were produced from 2-t-butyl-4,5-dichloro-3(2H)-pyridazinone (220 mg), 1-(4-biphenyl)-1,2-ethanediol (214 mg) and anhydrous cesium carbonate.

Compound 82

NMR($CDCl_3$) δ: 1.64 (9H,s), 2.88(1H,d,J = 3Hz), 4.29(1H,dd,J = 10Hz,8Hz), 4.37(1H,dd,J = 10Hz,4Hz), 5.21-(1H,dd,J = 8Hz,4Hz), 7.30-7.65(9H,m), 7.44(1H,s)

Compound 83
NMR($CDCl_3$) δ: 1.60(9H,s), 2.50(1H,brs), 3.87(1H,dd,J = 12Hz,3Hz), 4.06(1H,dd,J = 12Hz,8Hz), 5.48-(1H,dd,J = 8Hz,3Hz), 7.32-7.64(9H,m), 7.65(1H,s)

Working Example 10

Production of 2-t-butyl-4-chloro-5-[2-t-butyldimethylsilyloxy)-1-(p-biphenyl)]ethylthio-3(2H)-pyridazinone (compound 84)

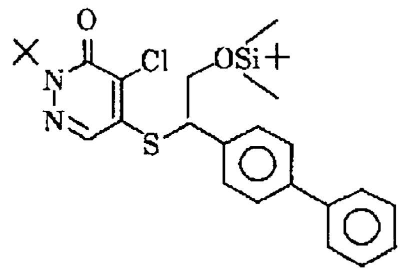

To a solution of 1-(4-biphenyl)-2-(t-butyldimethylsilyloxy)-ethanol(918 mg) and carbon tetrabromide (1.02 g) in THF (15 ml) was added dropwise, under ice-cooling, tributylphosphine (1.36 g), and the mixture was warmed up to room temperature, which was stirred overnight. The reaction mixture was diluted with ethyl acetate, which was then washed with a saturated aqueous solution of sodium chloride, followed by drying over anhydrous sulfate. The solvent was distilled off under reduced pressure, and the residue was refined by means of silica gel column chromatography (developing solvent: ethyl acetate - hexane = 2.98) to give 1-(4-biphenyl)-1-bromo-2-(t-butyldimethyloxy)ethane (810 mg). This product (293 mg) was dissolved in DMF (5 ml). To the solution were added 2-t-butyl-4-chloro-5-mercapto-3(2H)-pyridazinone (109 mg) and sodium carbonate (105 mg), and the mixture was stirred overnight. The reaction mixture was diluted with ethyl acetate, which was then washed with a saturated aqueous solution of sodium chloride, followed by drying over anhydrous sodium sulfate. The solvent was distilled off under reduced pressure. The residue was refined by means of silica gel column chromatography (developing solvent: ethyl acetate - hexane = 5.95) to give Compound 84 (171 mg; yield 65%).
NMR($CDCl_3$) δ: -0.06(6H,s), 0.79(9H,s), 1.55(9H,s), 3.92(1H,dd,J = 11Hz,7Hz), 4.03(1H,dd,J = 11Hz,6Hz), 4.62(1H,dd,J = 7Hz,6Hz), 7.30-7.60(9H,m), 7.62(1H,s)

Working Example 11

Production of 2-t-butyl-4-chloro-5-[2-hydroxy-1-(p-biphenyl)]ethylthio-3(2H)-pyridazinone (Compound 85)

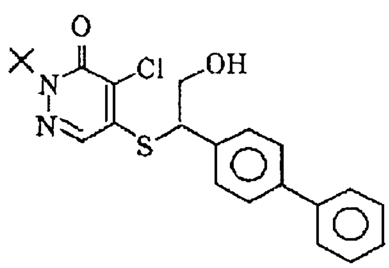

To a solution of Compound 84 (212 mg) in THF (4 ml) was added dropwise, under ice-cooling, tetrabutyl ammonium fluoride (1N THF solution: 0.44 ml). The mixture was stirred for one hour. To the reaction mixture was added water, followed by extraction with ether. The extract solution was washed with 1N HCl or a saturated aqueous solution of sodium chloride, which was dried over anhydrous sodium sulfate followed by distilling off the solvent under reduced pressure. The residue was refined by means of silica gel column chromatography (developing solvent: ether-hexane = 1:1) to give compound 85 (45 mg).
NMR($CDCl_3$) δ: 1.60(9H,s), 2.04(1H,t,J = 6Hz), 4.00(1H,dd,J-12Hz,7Hz), 4.08(1H,dd,J = 12Hz,6Hz), 4.69-

(1H,dd,J = 7Hz,6Hs), 7.30-7.65(10H,m)

Working Example 12

Production of 2-t-butyl-4-chloro-5-[4-[2-(5-t-butoxycarbonylamino)pyridyl]benzylthio]-3(2H)-pyridazinone (Compound 86)

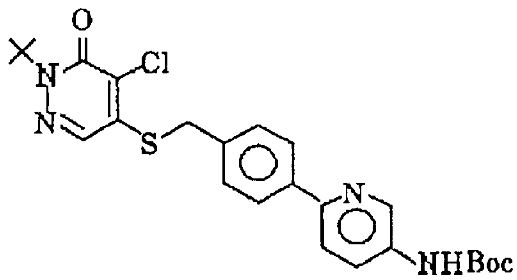

In substantially the same manner as Working Example 1, Compound 86 (28 mg) was produced from 2-t-butyl-4-chloro-5-mercapto-3(2H)-pyridazinone (33 mg), 3-t-butoxycarbonylamino-6-(4-bromomethylphenyl)-pyridine (50 mg) and anhydrous sodium carbonate (22 mg).
NMR($CDCl_3$) δ: 1.47(9H,s), 1.62(9H,s), 4.33(2H,s), 7.53(2H,d,J = 8Hz), 7.61(1H,s), 7.70-7.80(2H,m), 8.00-(2H,d,J = 8Hz), 8.65(1H,m)

Working Example 13

Production of 2-t-butyl-4-chloro-5-[4-[2-(5-t-butoxycarbonylamino)pyridyl]benzyloxy]-3(2H)-pyridazinone (Compound 87)

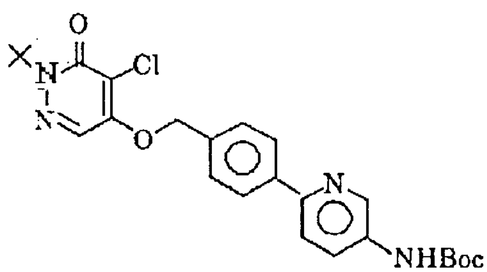

In substantially the same manner as Working Example 4, Compound 87 (37 mg) was produced from 2-t-butyl-4-chloro-5-hydroxy-3(2H)-pyridazinone (31 mg), 3-t-butoxycarbonylamino-6-(4-bromomethylphenyl)-pyridine (50 mg) and anhydrous sodium carbonate (22 mg).
NMR($CDCl_3$) δ: 1.47(9H,s), 1.63(9H,s), 5.39(2H,s), 7.53(2H,d,J = 8Hz), 7.73(1H,s), 7.74-7.85(2H,m), 8.06-(2H,d,J = 8Hz), 8.66(1H,m)

Working Example 14

Production of 5-[4-[2-(5-amino)pyridyl]benzylthio]-2-t-butyl-4-chloro-3(2H)-pyridazinone (Compound 88)

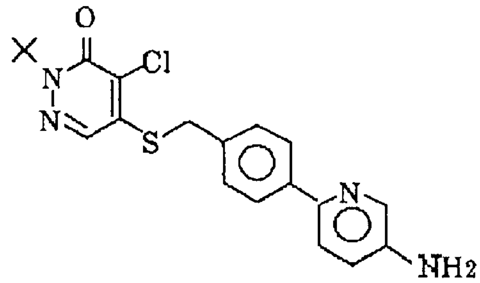

To a solution of Compound 86 (27 mg) in dichloromethane (0.5 ml) was added, under ice-cooling, trifluoroacetic acid (0.1 ml). The mixture was stirred for two hours at room temperature. The solvent was distilled off under reduced pressure. The residue was dissolved in ethyl acetate. The organic layer was washed with a saturated aqueous solution of sodium hydrogencarbonate and a saturated aqeuous solution of sodium chloride, which was dried over anhydrous magnesium sulfate. The solvent was distilled off under reduced pressure. The residue was refined by means of silica gel column chromatography (developing solvent: chloroform-methanol = 98:2) to give Compound 88 (16mg).
NMR($CDCl_3$-$CD_3OD$) δ: 1.63(9H,s), 4.36(2H,s), 7.54(2H,d,J = 8Hz), 7.71(1H,s), 7.73(1H,d,J = 9Hz), 7.91-(2H,d,J = 8Hz), 8.30(1H,dd,J = 9Hz,3Hz), 8.47(1H,d,J = 3Hz)

Working Example 15

Production of 5-[4-[2-(5-amino)pyridyl]benzyloxyl]-2-t-butyl-4-chloro-3(2H)-pyridazinone (Compound 89)

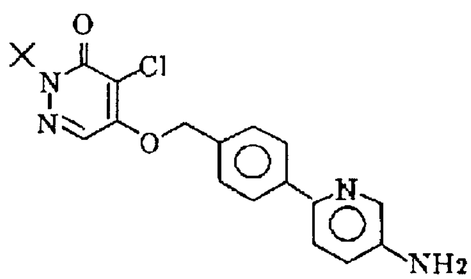

In substantially the same manner as Working Example 14, Compound 87 (33 mg) was processed with trifluoroacetic acid (0.1 mg) to afford Compound 89 (16 mg).
NMR($CDCl_3$-$CD_3OD$) δ: 1.63(9H,s), 5.41(2H,s), 7.53(2H,d,J = 8Hz), 7.74(1H,d,J = 9Hz), 7.82(1H,s), 7.96-(2H,d,J = 8Hz), 8.32(1H,dd,J = 9Hz,2Hz), 8.47(1H,d,J = 2Hz)

Working Example 16

Production of 2-t-butyl-4-chloro-5-[4-[4-(2-t-butoxycarbonylamino)pyridyl]benzylthio]-3(2H)-pyridazinone (Compound 90)

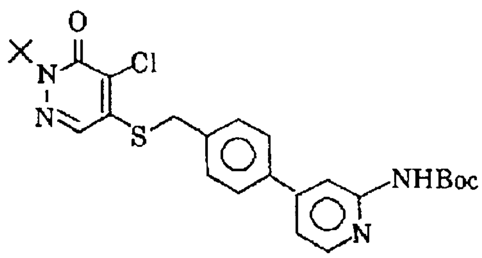

In substantially the same manner as Working Example 1, compound 90 (364 mg) was produced from 2-t-butyl-4-chooro-5-mercapto-3(2H)-pyridazinone (274 mg), 4-t-butoxycarbonylamino-4-(4-bromomethylphenyl)pyridine (429 mg) and anhydrous sodium carbonate (136 mg).
NMR($CDCl_3$) δ: 1.54(9H,s), 1.63(9H,s), 4.31(2H,s), 7.17(1H,dd,J = 4Hz,2Hz), 7.46-7.56(m,3H), 7.60(1H,s), 7.68(2H,d,J = 8Hz), 8.20(1H,s), 8.28(1H,d,J = 5Hz)

Working Example 17

Production of 2-t-butyl-4-chloro-5-[4-(2-t-butoxycarbonylamino)pyridyl]benzyloxy]-3(2H)-pyridazinone (Compound 91)

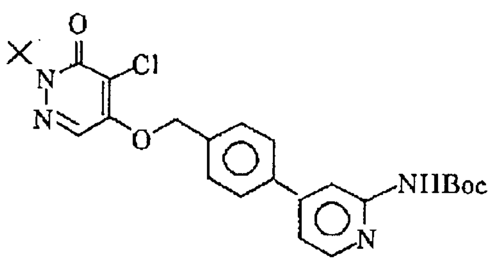

In substantially the same manner as Working Example 4, Compound 91 (629 mg) was produced from 2-t-butyl-4-chloro-5-hydroxyl-3(2H)-pyridazinone (465 mg), 2-t-butoxycarbonylamino-4-(4-bromomethylphenyl)pyridine (797 mg) and anhydrous sodium carbonate (246 mg).
NMR($CDCl_3$) δ: 1.54(9H,s), 1.64(9H,s), 5.37(2H,s), 7.18(1H,dd,J = 5H,2Hz), 7.41(1H,brs), 7.50(2H,d,J = 8Hz), 7.72(1H,s), 7.73(2H,d,J = 8Hz), 8.21(1H,s), 8.28(1H,d,J = 5Hz)

Working Example 18

Production of 5-[4-[4-(2-amino)pyridyl]benzylthio]-2-t-butyl-4-chloro-3(2H)-pyridazinone (Compound 92)

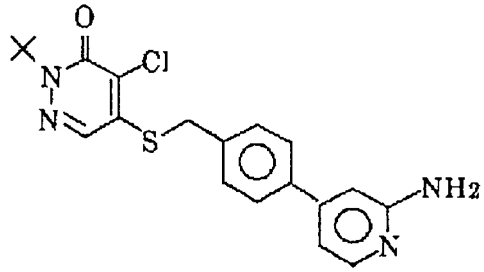

In substantially the same manner as working Example 14, Compound 90 (289 mg) was processed with trifluoroacetic acid (0.5 ml) to give Compound 92 (143 mg).
NMR($CDCl_3$) $\delta$: 1.63(9H,s), 4.31(2H,s), 4.52(2H,brs), 6.68(1H,s), 6.86(1H,dd,J = 5Hz,1Hz), 7.44-7.64(5H,m), 8.12(1H,d,J = 5Hz)

Working Example 19

Production of 5-[4-[4-(2-amino)pyridyl]benzyloxy]-2-t-butyl-4-chloro-3(2H)-pyridazinone (Compound 93)

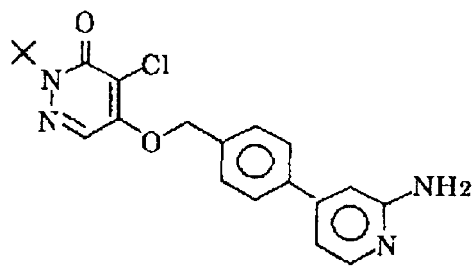

In substantially the same manner as Working Example 14, Compound 91 (511 mg) was processed with trifluoroacetic acid (1 ml) to give Compound 93 (360 mg).
NMR($CDCl_3$) $\delta$: 1.64(9H,s), 4.52(2H,brs), 5.36(2H,s), 6.69(1H,m), 6.88(1H,dd,J = 5Hz,2Hz), 7.50(2H,d,J = 8Hz), 7.64(2H,d,J = 8Hz), 7.74(1H,s), 8.13(1H,d,J = 5Hz)

Working Example 20

Production of 4-chloro-2-cyclohexyl-5-[4-(2-pyridyl)]benzylthio-3(2H)-pyridazinone (Compound 94)

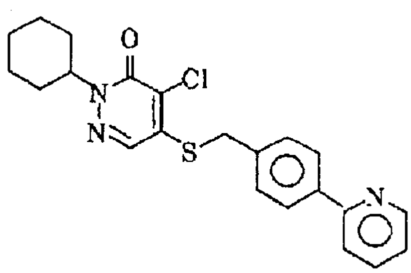

In substantially the same manner as Working Example 1, Compound 94 (16 mg; yield 38%) was produced as colorless crystals (recrystallization solvent: hexane-benzene) from 4-chloro-2-cyclohexyl-5-mercapto-3(2H)-pyridazinone (15 mg), 4-(2-pyridyl)benzylbromide (51 mg) and anhydrous sodium carbonate

(11 mg).
NMR($CDCl_3$) δ: 1.05-1.95(10H,m), 4.33(2H,s), 4.75-4.95(1H,m), 7.25(1H,m), 7.52(2H,d,J = 8Hz), 7.65-7.80-(3H,m), 8.00(2H,d,J = 8Hz), 8.67(1H,m)

| Elemental Analysis for $C_{22}H_{22}N_3OSCl \cdot 0.3H_2O$: | | | |
|---|---|---|---|
| Calcd.:<br>Found : | C:63.31%;<br>C:63.37%; | H:5.46%;<br>H:5.29%; | N:10.07%<br>N:10.14% |

Melting point: 192-193 °C

Working Example 21

Production of 4-chloro-2-cyclopropyl-5-[4-(2-pyridyl)]benzylthio-3(2H)-pyridazinone (Compound 95)

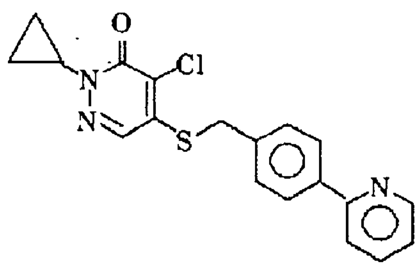

In substantially the same manner as Working Example 1, Compound 95 (12 mg; yield 44%) was produced as colorless crystals (recrystallization solvent: hexane-benzene) from 4-chloro-2-cyclopropyl-5-mercato-3(2H)-pyridazinone (26 mg), 4-(2-pyridyl)benzylbromide (63 mg) and anhydrous sodium carbonate (11 mg).
NMR($CDCl_3$) δ: 0.95-1.15 (4H,m), 4.00-4.15(1H,m), 4.33(2H,s), 7.27(1H,m), 7.52(2H,d,J = 8Hz), 7.60(1H,m), 7.70-7.85(2H,m), 8.01(2H,d,J = 8Hz), 8.70(1H,m)

| Elemental Analysis for $C_{19}H_{16}N_3OSCl$: | | | |
|---|---|---|---|
| Calcd.:<br>Found : | C:61.70%;<br>C:61.56%; | H:4.36%;<br>H:4.37%; | N:11.36%<br>N:11.44% |

Melting point: 169-170 °C

Working Example 22

Production of 2-t-butyl-4-chloro-5-[4-(2-pyrimidyl)]benzylthio-3(2H)-pyridazine (Compound 96)

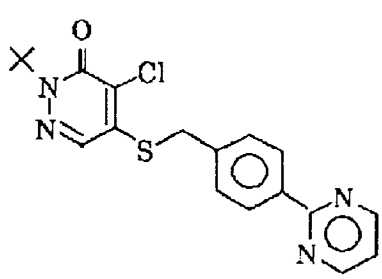

In substantially the same manner as Working Example 1, Compound 96 (194 mg; yield 83%) was produced as a colorless crystalline product (recrystallization solvent: ethanol) from 2-t-butyl-4-chloro-5-mercapto-3(2H)-pyridazinone (132 mg), 4-(2-pyrimidyl)benzyl bromide (150 mg) and anhydrous sodium carbonate (64 mg).
NMR($CDCl_3$) δ: 1.63(9H,s), 4.34(2H,s), 7.21(1H,t,J=5Hz), 7.50(2H,d,J=9Hz), 7.60(1H,s), 8.44(2H,d,J=9Hz), 8.81(2H,d,J=5Hz)

| Elemental Analysis for $C_{19}H_{19}N_4OSCl$: | | | |
|---|---|---|---|
| Calcd.: | C:58.98%; | H:4.95%; | N:14.48% |
| Found : | C:59.14%; | H:4.98%; | N:14.44% |

Melting point: 169-170 °C

Working Example 23

Production of 2-t-butyl-4-chloro-5-[4-(2-pyrimidyl)]benzyloxy-3(2H)-pyridazine (Compound 97)

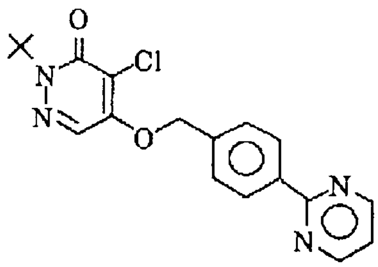

In substantially the same manner as Working Example 4, Compound 97 (124 mg; yield 33%) was produced as a colorless crystalline product (recrystallization solvent: ethanol) from 2-t-butyl-4-chloro-5-hydroxy-2(2H)-pyridazinone (203 mg), 4-(2-pyrimidyl)benzyl bromide (250 mg) and anhydrous sodium carbonate (106 mg).
NMR($CDCl_3$) δ: 1.63(9H,s), 5.40(2H,s), 7.22(1H,t,J=5Hz), 7.54(2H,d,J=8Hz), 7.72(1H,s), 8.49(2H,d,J=8Hz), 8.82(2H,d,J=5Hz)

| Elemental Analysis for $C_{19}H_{19}N_4O_2Cl$: | | | |
|---|---|---|---|
| Calcd.: | C:61.54%; | H:5.16%; | N:15.18% |
| Found : | C:61.26%; | H:5.13%; | N:15.10% |

Melting point: 193-194 °C

Working Example 24

Production of 2-t-butyl-4-chloro-5-[4-[2-(4,6-dimethoxy)pyrimidyl]]benzylthio-3(2H)-pyridazinone (Compound 98)

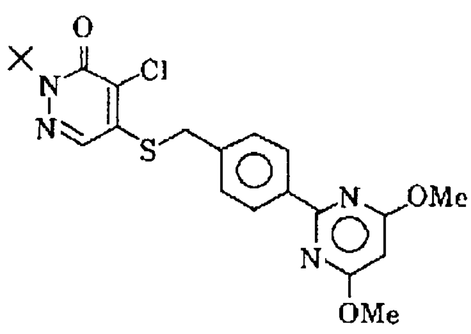

In substantially the same manner as Working Example 1, Compound 98 (107 mg; yield 48%) was produced as a colorless crystalline product (recrystallization solvent: ethyl acetate) from 2-t-butyl-4-chloro-5-mercapto-3(2H)-pyridazinone (109 mg), 4-[2-(4,6-dimethyloxy)pyrimidyl]benzyl bromide (155 mg) and anhydrous sodium carbonate (53 mg).

NMR($CDCl_3$) δ: 1.62(9H,s), 4.04(6H,s), 4.33(2H,s), 5.98(1H,s), 7.51(2H,d,J=8Hz), 7.60(1H,s), 8.45-(2H,d,J=8Hz)

| Elemental Analysis for $C_{21}H_{23}N_4O_3SCl$: | | | |
|---|---|---|---|
| Calcd.: | C:56.43%; | H:5.19%; | N:12.54% |
| Found : | C:56.68%; | H:5.13%; | N:12.36% |

Working Example 25

Production of 2-t-butyl-4-chloro-5-[4-[2-(4,6-dimethoxy)pyrimidyl]]benzyloxy-3(2H)-pyridazinone (Compound 99)

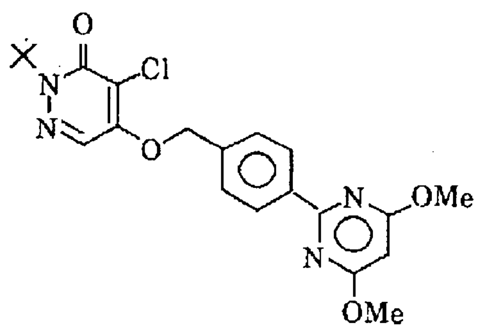

In substantially the same manner as Working Example 4, Compound 99 (227 mg; yield 65%) was produced as a colorless crystalline product (recrystallization solvent: ethyl acetate) from 2-t-butyl-4-chloro-5-hydroxy-3(2H)-pyridazinone (164 mg), 4-[2-(4,6-dimethoxy)pyrimidyl]benzyl bromide (250 mg) and anhydrous sodium carbonate (86 mg).

NMR($CDCl_3$) δ: 1.63(9H,s), 4.05(6H,s), 5.40(2H,s), 5.99(1H,s), 7.50(2H,d,J=8Hz), 7.71(1H,s), 8.50-(2H,d,J=8Hz)

| Elemental Analysis for $C_{21}H_{23}N_4O_4Cl$: | | | |
|---|---|---|---|
| Calcd.: | C:58.54%; | H:5.38%; | N:13.00% |
| Found : | C:58.56%; | H:5.38%; | N:12.99% |

Working Example 26

Production of 2-t-butyl-4-chloro-5-[4-[2-(4,6-dichloro)pyrimidyl]benzylthio-3(2H)-pyridazinone (Compound 100)

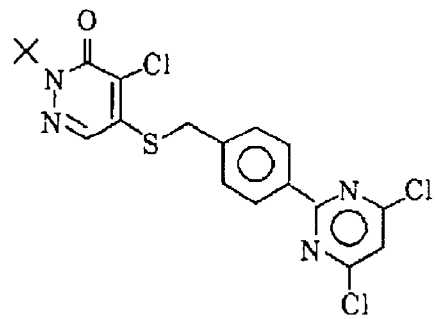

In substantially the same manner as Working Example 1, Compound 100 (75 mg; yield 35%) was produced as a colorless crystalline product (recrystallization solvent: ethanol) from 2-t-butyl-4-chloro-5-mercapto-3(2H)-pyridazinone (103 mg), 4-[2-(4,6-dichloro)pyrimidyl]benzyl bromide (150 mg) and anhydrous sodium carbonate (50 mg).
NMR($CDCl_3$) $\delta$: 1.62(9H,s), 4.33(2H,s), 7.29(1H,s), 7.54(2H,d,J=9Hz), 7.57(1H,s), 8.43(2H,d,J=9Hz)

| Elemental Analysis for $C_{19}H_{17}N_4OSCl_3$: | | | |
|---|---|---|---|
| Calcd.: | C:50.07%; | H:3.76%; | N:12.29% |
| Found : | C:49.77%; | H:3.73%; | N:12.46% |

Working Example 27

Production of 2-t-butyl-4-chloro-5-[4-[2-(4,6-dichloro)pyrimidyl]]benzyloxy-3(2H)-pyridazinone (Compound 101)

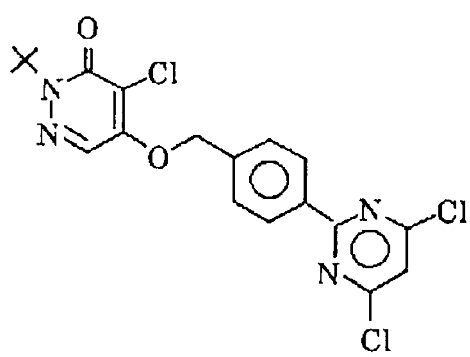

In substantially the same manner as Working Example 4, Compound 101 (242 mg; yield 55%) was produced as a colorless crystalline product (recrystallization solvent: ethanol) from 2-t-butyl-4-chloro-5-hydroxy-3(2H)-pyridazinone (203 mg), 4-[2-(4,6-dichloro)pyrimidyl]benzyl bromide (318 mg) and anhydrous sodium carbonate (106 mg).
NMR($CDCl_3$) $\delta$: 1.63(9H,s), 5.40(2H,s), 7.30(1H,s), 7.54(2H,d,J=9Hz), 7.70(1H,s), 8.48(2H,d,J=9Hz)

| Elemental Analysis for $C_{19}H_{17}N_4O_2Cl_3$: | | | |
|---|---|---|---|
| Calcd.:<br>Found : | C:51.90%;<br>C:51.67%; | H:3.90%;<br>H:3.88%; | N:12.74%<br>N:12.60% |

Working Example 28

Production of 5-[4-[4-(2-aminothiazolyl]]benzylthio-2-t-butyl-4-chloro-3(2H)-pyridazinone (Compound 102)

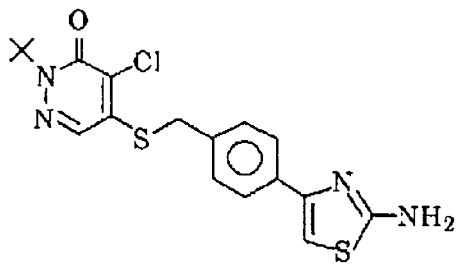

In substantially the same manner as Working Example 1, Compound 102 (40 mg; yield 20%) was produced as a colorless crystalline product (recrystallization solvent: ethanol) from 2-t-butyl-4-chloro-5-mercapto-3(2H)-pyridazinone (109 mg), 2-amino-4-(4-bromomethylphenyl)thiazole•hydrobromide (240 mg) and anhydrous sodium carbonate (80 mg).
NMR(DMSO-$d_6$) δ: 1.56(9H,s), 4.54(2H,brs), 7.02(1H,s), 7.06(2H,brs), 7.45(2H,d,J = 8Hz), 7.79(2H,d,J = 8Hz), 8.05(1H,s)

| Elemental Analysis for $C_{18}H_{19}N_4OS_2Cl$: | | | |
|---|---|---|---|
| Calcd.:<br>Found : | C:53.13%;<br>C:52.97%; | H:4.71%;<br>H:4.53%; | N:13.77%<br>N:13.86% |

Working Example 29

Production of 5-[4-[4-(2-aminothiazolyl]]benzyloxy-2-t-butyl-4-chloro-3(2H)-pyridazinone (Compound 103)

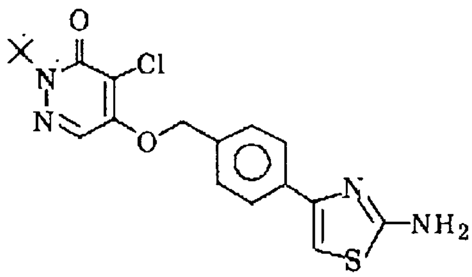

In substantially the same manner as Working Example 4, Compound 103 (83 mg; yield 4%) was produced as a colorless crystalline product (recrystallization solvent: ethanol) from 2-t-butyl-4-chloro-5-hydroxy-3(2H)-pyridazinone (1.01 g), 2-amino-4-(4-bromomethylphenyl)thiazole•hydrobromide (3.85 g) and anhydrous sodium carbonate (1.06 g).
NMR(DMSO-$d_6$) δ: 1.57(9H,s), 5.45(2H,brs), 7.04(1H,s), 7.06(1H,brs), 7.44(2H,d,J = 9Hz), 7.83(2H,d,J = 9Hz), 8.26(1H,s)

| Elemental Analysis for $C_{18}H_{19}N_4O_2SCl \cdot H_2O$: | | | |
|---|---|---|---|
| Calcd.:<br>Found : | C:52.87%;<br>C:52.79%; | H:5.18%;<br>H:4.85%; | N:13.70%<br>N:13.42% |

Working Example 30

Production of 2-t-butyl-4-methyl-5-[4-(2-pyridyl)]benzyloxy-3(2H)-pyridazinone (Compound 104)

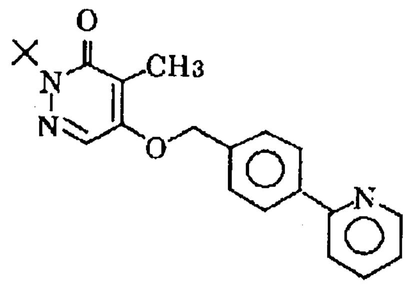

In substantially the same manner as Working Example 1, Compound 104 (140 mg; yield 5%) was produced as a colorless crystalline product (recrystallization solvent: isopropyl ether) from 2-t-butyl-4-methyl-5-hydroxy-3(2H)-pyridazinone (1.61 g), 4-(2-pyridyl)benzyl bromide (3.20 g) and anhydrous sodium carbonate (936 mg).
NMR($CDCl_3$) $\delta$: 1.63(9H,s), 2.09(3H,s), 5.27(2H,s), 7.27(1H,m), 7.50(2H,d,J=9Hz), 7.72(1H,s), 7.68-7.80-(2H,m), 8.03(2H,d,J=9Hz), 8.70(1H,m)

| Elemental Analysis for $C_{21}H_{23}N_3O_2 \cdot H_2O$: | | | |
|---|---|---|---|
| Calcd.:<br>Found : | C:71.45%;<br>C:71.43%; | H:6.68%;<br>H:6.59%; | N:11.90%<br>N:11.93% |

Working Example 31

Production of 2-t-butyl-4-chloro-5-[4-[1-(triphenylmethyl)-1H-1,2,4-triazol-5-yl]benzylthio]-3(2H)-pyridazinone (Compound 105)

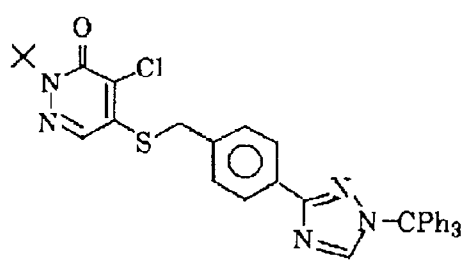

To a solution of 2-t-butyl-chloro-5-mercapto-3(2H)-pyridazinone(792 mg) in DMF (30 ml) were added anhydrous potassium carbonate (751 mg) and N-(triphenylmethyl)-5-(4-bromomethylphenyl)-1,2,4-triazole (1.85 g). The mixture was stirred for 3.5 hours at 100 °C. The reaction mixture was cooled to room temperature, to which was added water (300 ml), followed by extraction with ethyl acetate. The extract solution was washed with a saturated aqueous solution of sodium chloride, which was dried over anhydrous magnesium sulfate. The solvent was distilled off under reduced pressure. The residue was recrystallized

from ethyl acetate to give Compound 105 (1.30 g; yield 58%) as a pale green crystalline product.
NMR(DMSO-$d_6$) δ: 1.56(9H,s), 4.58(2H,s), 7.10-7.17(6H,m), 7.35-7.43(9H,m), 7.55(2H,d,J=8Hz), 7.96-(2H,d,J=8Hz), 8.03(1H,s), 8.19(1H,s)
Melting point: 212-213 °C

| Elemental Analysis for $C_{36}H_{32}ClN_5OS$: | | | |
| --- | --- | --- | --- |
| Calcd.:<br>Found : | C:69.94%;<br>C:69.76%; | H:5.22%;<br>H:5.14%; | N:11.33%<br>N:11.25% |

Working Example 32

Production of 2-t-butyl-4-chloro-5-[4-[1-(triphenylmethyl)-1H-1,2,4-triazol-5-yl]benzyloxy]-3(2H)-pyridazinone (Compound 106)

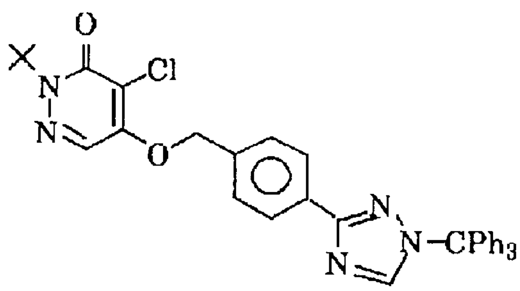

To a solution of 2-t-butyl-4-chloro-5-hydroxy-3(2H)-pyridazinone (792 mg) in DMF (30 ml) were added anhydrous potassium carbonate (751 mg) and N-(triphenylmethyl)-5-(4-bromomethylphenyl)-1,2,4-triazole (1.85 g). The mixture was stirred for 2.5 hours at 100 °C, which was then cooled to room temperature, followed by addition of water. The mixture was subjected to extraction with ethyl acetate. The extraction was washed with a saturated aqueous solution of sodium chloride, which was dried over anhydrous magnesium sulfate. The solvent was distilled off under reduced pressure. The residue was recrystallized from ethyl acetate to give Compound 106 (1.03 g; yield 47%) as a colorless crystalline product.
NMR(DMSO-$d_6$) δ: 1.57(9H,s), 5.50(2H,s), 7.09-7.18(6H,m), 7.29-7.45(9H,m), 7.54(2H,d,J=8Hz), 8.01-(2H,d,J=8Hz), 8.21(1H,s), 8.25(1H,s)
Melting point: 168-169 °C

| Elemental Analysis for $C_{36}H_{32}ClN_5O_2$: | | | |
| --- | --- | --- | --- |
| Calcd.:<br>Found : | C:71.81%;<br>C:71.57%; | H:5.36%;<br>H:5.37%; | N:11.63%<br>N:11.26% |

Working Example 33

Production of 2-t-butyl-4-chloro-5-[4-(1H-1,2,4-triazol-5-yl)benzylthio]-3(2H)-pyridazinone (Compound 107)

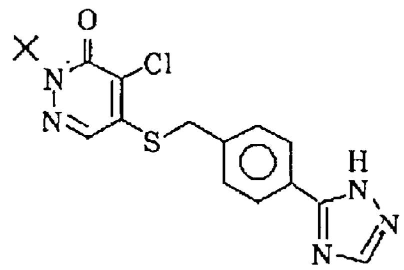

In a mixture of 10% HCl (2 ml) and tetrahydrofuran (4 ml) was dissolved 2-t-butyl-4-chloro-5-[4-[1-(triphenyl)-1H-1,2,4-triazol-5-yl]benzylthio]-3(2H)-pyridazinone (618 mg), and the solution was stirred for 3 hours at 75°C. The solvent was distilled off under reduced pressure. To the residue was added water, which was subjected to extraction with ethyl acetate. The extract solution was dried over anhydrous magnesium sulfate and, then, the solvent was distilled off under reduced pressure. The residue was refined by means of silica gel column chromatography (developing solvent: chloroform-methanol=95:5) to give Compound 107 (199 mg; yield 53%) as a white powdery product.
NMR($CDCl_3$) δ: 1.63(9H,s), 4.32(2H,s), 7.51(2H,d,J=8Hz), 7.62(2H,d,J=8Hz), 8.09(1H,s), 8.26(1H,s)
IR(KBr): 3210, 3100, 2990, 2940, 1620, 1555, 1495 $cm^{-1}$
Melting point: 199-200°C

Working Example 34

Production of 2-t-butyl-4-chloro-5-[4-(1H-1,2,4-triazol-5-yl)benzyloxy]-3(2H)-pyridazinone (Compound 108)

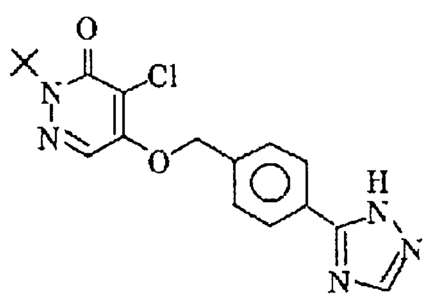

In substantially the same manner as Working Example 33, Compound 108 (114 mg; yield 32%) was produced as a white powdery product from 2-t-butyl-4-chloro-5-[4-[1-(triphenylmethyl)-1H-1,2,4-triazol-5-yl]-benzyloxo]-3(2H)-pyridazinone (602 mg).
NMR(DMSO-$d_6$) δ: 1.58(9H,s), 5.51(2H,s), 7.57(2H,d,J=8Hz), 8.07(2H,d,J=8Hz), 8.27(1H,s), 8.46(1H,s)
IR(KBr): 3155, 3100, 3000, 2840, 1625, 1595, 1555, 1500 $cm^{-1}$
Melting point: 220-221°C

| Elemental Analysis for $C_{17}H_{18}ClN_5O_2$: | | | |
|---|---|---|---|
| Calcd.: | C:56.75%; | H:5.04%; | N:19.46% |
| Found : | C:56.54%; | H:4.95%; | N:19.49% |

Working Example 35

Production of 2-t-butyl-4-chloro-5-[4-[1-(triphenylmethyl)pyrazol-3-yl]benzylthio]-3(2H)-pyridazinone (Compound 109)

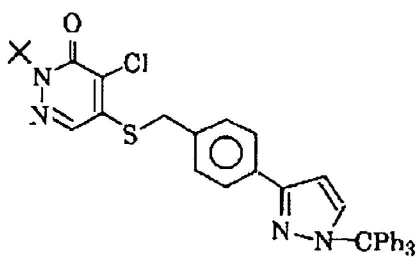

In substantially the same manner as Working Example 31, Compound 109 (3.41 g; yield 72%) was produced as a colorless crystalline product from 2-t-butyl-4-chloro-5-mercapto-3(2H)-pyridazinone (1.69 g) and N-(triphenylmethyl)-3-(4-bromomethylphenyl)pyrazole (4.08 g).
NMR(DMSO-$d_6$) δ: 1.56(9H,s), 4.56(2H,s), 6.80(1H,d,J=2Hz), 7.08-7.16(6H,m), 7.34-7.46(10H,m), 7.49-(2H,d,J=8Hz), 7.76(2H,d,J=8Hz), 8.05(1H,s)
Melting point: 187-188 °C

Working Example 36

Production of 2-t-butyl-4-chloro-5-[4-[1-(triphenylmethyl)pyrazol-3-yl]benzyloxy]-3(2H)-pyridazinone (Compound 110)

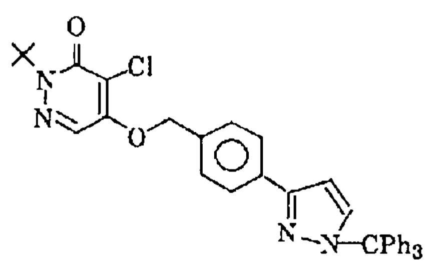

In substantially the same manner as Working Example 32, Compound 110 (2.24 g; yield 52%) was produced as a colorless crystalline product from 2-t-butyl-4-chloro-5-hydroxy-3(2H)-pyridazinone (1.44 g) and N-(triphenylmethyl)-3-(4-bromomethylphenyl)pyrazole (4.08 g).
NMR(DMSO-$d_6$) δ: 1.57(9H,s), 5.47(2H,s), 6.82(1H,d,J=2.6Hz), 7.08-7.17(6H,m), 7.33-7.45(10H,m), 7.48-(2H,d,J=8Hz), 7.81(2H,d,J=8Hz), 8.26(1H,s)
Melting point: 89-91 °C

Working Example 37

Production of 2-t-butyl-4-chloro-5-[4-(1H-pyrazol-3-yl)benzylthio]-3(2H)-pyridazinone (Compound 111)

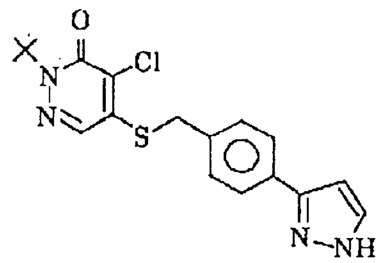

In substantially the same manner as Working Example 33, Compound 111 (139 mg; yield 60%) was produced as a white powdery product from 2-t-butyl-4-chloro-5-[4-[1-(triphenylmethyl)pyrazolo-3-yl]-benzylthio]-3(2H)-pyridazinone (379 mg).
NMR($CDCl_3$) δ: 1.63(9H,s), 4.31(2H,s), 6.65(1H,d,J = 2Hz), 7.47(2H,d,J = 8Hz), 7.63(1H,s), 7.64(1H,d,J = 2Hz), 7.79(2H,d,J = 8Hz)
IR(KBr): 3250, 3000, 2970, 2940, 2890, 1630, 1560, 1520 $cm^{-1}$
Melting point: 161-163 ° C

| Elemental Analysis for $C_{18}H_{19}ClN_4OS$: | | | |
|---|---|---|---|
| Calcd.:<br>Found : | C:57.67%;<br>C:57.66%; | H:5.11%;<br>H:4.90%; | N:14.94%<br>N:15.01% |

Working Example 38

Production of 2-t-butyl-4-chloro-5-[4-(1H-pyrazol-3-yl)benzyloxy]-3(2H)-pyridazinone (Compound 112)

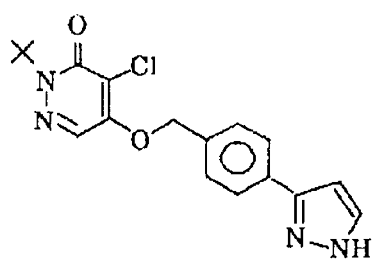

In substantially the same manner as Working Example 33, Compound 112 (311 mg; yield 87%) was produced as a white powdery product from 2-t-butyl-4-chloro-5-[4-[1-(triphenylmethyl)pyrazolo-3-yl]-benzyloxy]-3(2H)-pyridazinone (601 mg).
NMR($CDCl_3$) δ: 1.63(9H,s), 5.35(2H,s), 6.66(1H,d,J = 2Hz), 7.47(2H,d,J = 8Hz), 7.64(1H,d,J = 2Hz), 7.74(1H,s), 7.85(2H,d,J = 8Hz)
IR(KBr): 3000, 2960, 2940, 2860, 1620, 1600, 1515 $cm^{-1}$
Melting point: 198-200 ° C

| Elemental Analysis for $C_{18}H_{19}ClN_4O_2$: | | | |
|---|---|---|---|
| Calcd.:<br>Found : | C:60.25%;<br>C:59.91%; | H:5.34%;<br>H:5.24%; | N:15.61%<br>N:15.78% |

Working Example 39

Production of 2-t-butyl-4-chloro-5-[4-(1,2-oxazol-5-yl)benzylthio]-3(2H)-pyridazinone (Compound 113)

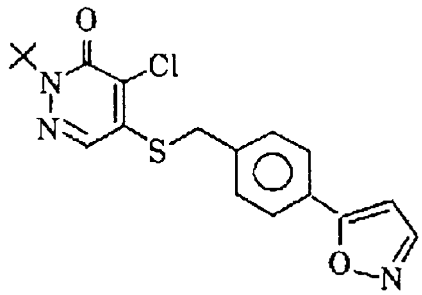

In substantially the same manner as Working Example 31, Compound 113 (27 mg; yield 4%) was produced as a white powdery product from 2-t-butyl-4-chloro-5-mercapto-3(2H)-pyridazinone (350 mg) and 5-(4-bromomethylphenyl)-1,2-oxazole (419 mg).
NMR($CDCl_3$) δ: 1.62(9H,s), 4.31(2H,s), 6.54(1H,d,J = 2Hz), 7.53(2H,d,J = 8Hz), 7.59(1H,s), 7.81(2H,d,J = 8Hz), 8.30(1H,d,J = 2Hz)
IR(KBr): 3080, 2950, 2920, 1640, 1605, 1590, 1560, 1500 $cm^{-1}$
Melting point: 147-149 °C

| Elemental Analysis for $C_{18}H_{18}ClN_3O_2$: | | | |
|---|---|---|---|
| Calcd.: | C:57.52%; | H:4.83%; | N:11.18% |
| Found : | C:57.48%; | H:4.89%; | N:11.04% |

Working Example 40

Production of 2-t-butyl-4-chloro-5-[4-(1,2-oxazol-5-yl)benzyloxy]-3(2H)-pyridazinone (Compound 114)

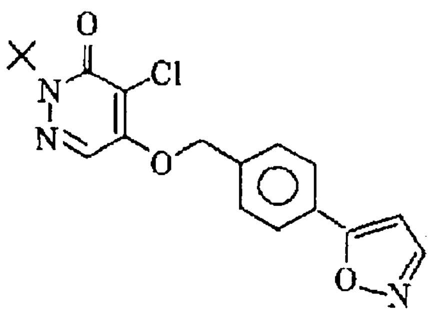

In substantially the same manner as Working Example 32, Compound 114 (47 mg; yield 4%) was produced as a pale yellow powdery product from 2-t-butyl-4-chloro-5-hydroxy-3(2H)-pyridazinone (608 mg) and 5-(4-bromomethylphenyl)-1,2-oxazole (786 mg).
NMR($CDCl_3$) δ: 1.64(9H,s), 5.37(2H,s), 6.57(1H,d,J = 2Hz), 7.54(2H,d,J = 8Hz), 7.73(1H,s), 7.87(2H,d,J = 8Hz), 8.32(1H,d,J = 2Hz)
IR(KBr): 3110, 2990, 2930, 1640, 1600, 1555, 1500 $cm^{-1}$
Melting point: 194-195 °C

| Elemental Analysis for $C_{18}H_{18}ClN_3O_3$: | | | |
|---|---|---|---|
| Calcd.:<br>Found : | C:60.09%;<br>C:59.70%; | H:5.04%;<br>H:5.00%; | N:11.68%<br>N:11.42% |

Working Example 41

Production of 2-t-butyl-4-chloro-5-[4-(1,2,3-thiadiazol-4-yl)benzylthio]-3(2H)-pyridazinone (Compound 115)

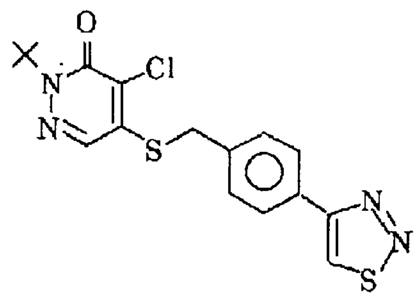

In substantially the same manner as Working Example 31, Compound 115 (470 mg; yield 60%) was produced as a pale yellow crystalline product by allowing 2-t-butyl-4-chloro-5-mercapto-3(2H)-pyridazinone (437 mg) to react with 4-bromomethylphenyl)-1,2,3-thiadiazole (561 mg), followed by recrystallization from chloroform/ether.
NMR($CDCl_3$) δ: 1.63(9H,s), 4.34(2H,s), 7.55(1H,s), 7.61(2H,d,J = 8Hz), 8.07(2H,d,J = 8Hz), 8.68(1H,s)
IR(KBr): 3050, 2980, 2955, 1640, 1560 $cm^{-1}$
Melting point: 175-177 °C

Working Example 42

Production of 2-t-butyl-4-chloro-5-[4-(1,2,3-thiadiazol-4-yl)benzyloxy]-3(2H)-pyridazinone (Compound 116)

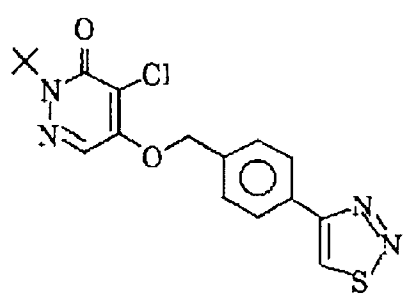

In substantially the same manner as Working Example 32, 2-t-butyl-4-chloro-5-hydroxy-3(2H)-pyridazinone (608 mg) was reacted with 4-(4-bromomethylphenyl)-1,2,3-thiadiazole (842 mg), and, then the reaction product was refined by means of silica gel column chromatography (developing solvent: hexane - ethyl acetate = 3:1) to afford Compound 116 (40 mg; yield 4%) as a yellow powdery product.
NMR(DMSO-$d_6$) δ: 1.58(9H,s), 5.54(2H,s), 7.64(2H,d,J = 8Hz), 8.21(2H,d,J = 8Hz), 8.29(1H,s), 9.66(1H,s)
IR(KBr): 3050, 2990, 2940, 1635, 1600 $cm^{-1}$
Melting point: 240-242 °C

Working Example 43

Production of 2-t-butyl-4-chloro-5-[4-(1,2,4-thiadiazol-5-yl)benzylthio]-3(2H)-pyridazinone (Compound 117)

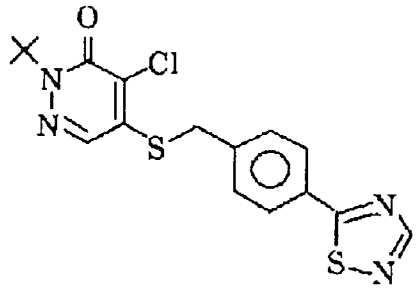

In substantially the same manner as Working Example 31, 2-t-butyl-4-chloro-5-mercapto-3(2H)-pyridazinone (437 mg) was reacted with 5-(4-bromomethylphenyl)-1,2,4-thiadiazole (561 mg), and, then the reaction product was refined by means of silica gel column chromatography (developing solvent: hexane - ethyl acetate = 8:1 - 6:1) to give Compound 117 (330 mg; yield 42%) as a colorless powdery product.
NMR($CDCl_3$) $\delta$: 1.62(9H,s), 4.33(2H,s), 7.57(2H,d,J = 8Hz), 7.58(1H,s), 8.00(2H,d,J = 8Hz), 8.72(1H,s)
IR(KBr): 2990, 2960, 2940, 1640, 1560 $cm^{-1}$
Melting point: 93-95 °C

Working Example 44

Production of 2-t-butyl-4-chloro-5-[4-(1,2,4-thiadiazol-5-yl)benzyloxy-3(2H)-pyridazinone (Compound 118)

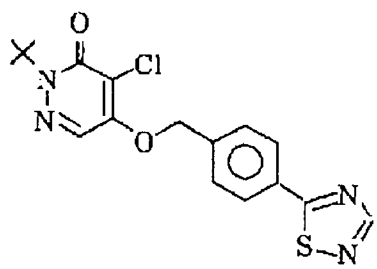

In substantially the same manner as Working Example 32, 2-t-butyl-4-chloro-5-hydroxy-3(2H)-pyridazinone (709 mg) was reacted with 5-(4-bromomethylphenyl)-1,2,4-thiadiazole (982 mg), and, then the reaction product was recrystallized from methanol to give Compound 118 (887 mg; yield 67%) as a colorless crystalline product.
NMR(DMSO-$d_3$) $\delta$: 1.58(9H,s), 5.58(2H,s), 7.68(2H,d,J = 8Hz), 8.14(2H,d,J = 8Hz), 8.28(1H,s), 9.00(1H,s)
IR(KBr): 3050, 3000, 2960, 2925, 1635, 1600, 1500 $cm^{-1}$
Melting point: 197-200 °C

| Elemental Analysis for $C_{17}H_{17}ClN_4O_2S$: | | | |
|---|---|---|---|
| Calcd.: | C:54.18%; | H:4.55%; | N:14.87% |
| Found : | C:53.92%; | H:4.44%; | N:14.93% |

Working Example 45

Production of 2-t-butyl-4-chloro-5-[4-(1,3-oxazol-5-yl)benzylthio]-3(2H)-pyridazinone (Compound 119)

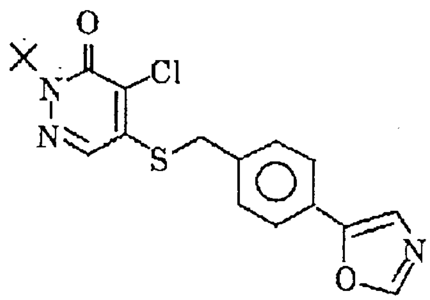

In substantially the same manner as Working Example 31, 2-t-butyl-4-chloro-5-mercapto-3(2H)-pyridazinone (437 mg) was reacted with 5-(4-bromomethylphenyl)-1,3-oxazole (524 mg), and, then the reaction product was refined by means of silica gel column chromatography (developing solvent: hexane - ethyl acetate = 1:1) to give Compound 119 (658 mg; yield 88%) as a colorless powdery product.
NMR($CDCl_3$) δ: 1.63(9H,s), 4.30(2H,s), 7.39(1H,s), 7.49(2H,d,J = 8Hz), 7.60(1H,s), 7.68(2H,d,J = 8Hz), 7.94-(1H,s)
IR(KBr): 3140, 3120, 2995, 2955, 1640, 1560, 1505, 1485 $cm^{-1}$
Melting point: 98-99 ° C

| Elemental Analysis for $C_{18}H_{18}ClN_3O_2S$: | | | |
|---|---|---|---|
| Calcd.: | C:57.52%; | H:4.83%; | N:11.18% |
| Found : | C:57.80%; | H:4.88%; | N:11.14% |

Working Example 46

Production of 2-t-butyl-4-chloro-5-[4-(1,3-oxazol-5-yl)benzyloxy]-3(2H)-pyridazinone (Compound 120)

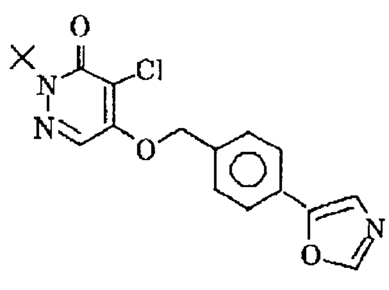

In substantially the same manner as Working Example 32, 2-t-butyl-4-chloro-5-hydroxy-3(2H)-pyridazinone (608 mg) was reacted with 5-(4-bromomethylphenyl)-1,3-oxazole (786 mg), and, then the reaction product was recrystallized from methanol to give Compound 120 (733 mg; yield 68%) as a pale yellow crystalline product.
NMR($CDCl_3$) δ: 1.64(9H,s), 5.34(2H,s), 7.40(1H,s), 7.49(2H,d,J = 8Hz), 7.72(2H,d,J = 8Hz), 7.73(1H,s), 7.94-(1H,s)
IR(KBr): 3050, 3000, 29550, 2930, 1630, 1600, 1515, 1490 $cm^{-1}$
Melting point: 182-183 ° C

| Elemental Analysis for $C_{18}H_{18}ClN_3O_3$: | | | |
|---|---|---|---|
| Calcd.:<br>Found : | C:60.09%;<br>C:59.81%; | H:5.04%;<br>H:5.06%; | N:11.68%<br>N:11.86% |

Working Example 47

| | |
|---|---|
| (1) Compound 1 | 10.0 mg |
| (2) lactose | 60.0 mg |
| (3) corn starch | 35.0 mg |
| (4) gelatine | 3.0 mg |
| (5) magnesium stearate | 2.0 mg |

A mixture of Compound 1 (10.0 mg), lactose (60.0 mg) and corn starch (35.0 mg) was granulated through a 1 mesh screen using a 10% aqueous solution of gelatin (0.03 ml: 3.0 mg in terms of gelatin). The granule was dried at 40°C and screened again. The thus-obtained granule was mixed with magnesium stearate (2.0 g), and the mixture was compressed to form a core tablet. The core tablet was sugar-coated with an aqueous solution of sucrose, titanium dioxide, talc and gum arabic. The coated tablet was polished with bees wax.

Working Example 48

| | |
|---|---|
| (1) Compound 1 | 10.0 mg |
| (2) lactose | 70.0 mg |
| (3) corn starch | 50.0 mg |
| (4) soluble starch | 7.0 mg |
| (5) magnesium stearate | 3.0 mg |

Compound 1 (10.0 mg) and lactose (70.0 mg) were granulated with an aqueous solution of soluble starch (0.07 ml: 7.0 mg in terms of soluble starch). The granule was dried and mixed with magnesium stearate (3.0 mg) and corn starch (50.0 mg). The mixture was compressed into a tablet.

Working Example 49

| | |
|---|---|
| (1) Compound 1 | 20.0 mg |
| (2) lactose | 70.0 mg |
| (3) magnesium stearate | 3.0 mg |

The above (1), (2) and (3) were sufficiently blended and filled in a capsule (size No.3).

Working Example 50

| | |
|---|---|
| (1) Compound 2 | 20.0 mg |
| (2) lactose | 60.0 mg |
| (3) corn starch | 35.0 mg |
| (4) hydroxypropylcellulose | 3.0 mg |
| (5) magnesium stearate | 2.0 mg |

A mixture of Compound 2 (20.0 mg), lactose (60.0 mg) and corn starch (35.0 mg) was screened through a 1 mm mesh screen, which was granulated. The granule was dried at 40 °C and passed through the screen again. The granule was blended with magnesium stearate (2.0 mg), and the mixture was compressed to form a core tablet. The core tablet was sugar-coated with an aqueous solution of sugar, titanium dioxide, talc and gum arabic. The coated tablet was polished with bees wax.

Working Example 51

| | |
|---|---|
| (1) Compound 19 | 10.0 mg |
| (2) lactose | 70.0 mg |
| (3) corn starch | 50.0 mg |
| (4) soluble starch | 7.0 mg |
| (5) magnesium stearate | 3.0 mg |

Compound 19 (10.0 mg) and lactose (70.0 mg) were granulated with an aqueous solution of soluble starch (0.07 ml: 7.0 mg in terms of soluble starch), which was dried, followed by blending with magnesium stearate (3.0 mg) and corn starch (50.0 mg). The mixture was compressed into a tablet.

Working Example 52

| | |
|---|---|
| (1) Compound 3 | 20.0 mg |
| (2) lactose | 79.0 mg |
| (3) magnesium stearate | 3.0 mg |

The above ingredients were blended sufficiently, and filled in a capsule (size No.3).

Working Example 53

| | |
|---|---|
| (1) Compound 4 | 10.0 mg |
| (2) lactose | 60.0 mg |
| (3) corn starch | 35.0 mg |
| (4) hydroxypropylcellulose | 3.0 mg |
| (5) magnesium stearate | 2.0 mg |

A mixture of Compound 4 (10.0 mg), lactose (60.0 mg) and corn starch (35.0 mg) was granulated through a 1 mm mesh using a 10% aqueous solution of hydroxypropylcellulose (0.03 ml: 3.0 mg in terms of hydroxypropylcellulose). The granule was dried at 40 °C and screened again. The granule thus obtained was blended with magnesium stearate (2.0 mg), and the mixture was compressed to form a core tablet. The core tablet was sugar-coated with an aqueous solution of sucrose, titanium dioxide, talc and gum arabic. The coated tablet was polished with bees wax.

Working Example 54

| | |
|---|---|
| (1) Compound 6 | 20.0 mg |
| (2) lactose | 70.0 mg |
| (3) corn starch | 50.0 mg |
| (4) soluble starch | 7.0 mg |
| (5) magnesium stearate | 3.0 mg |

Compound 6 (20.0 mg) and lactose (70.0 mg) were granulated using an aqueous solution of soluble starch (0.07 ml: 7.0 mg in terms of soluble starch), which was dried, followed by blending with magnesium stearate (3.0 mg) and corn starch (50.0 mg). The mixture was compressed into a tablet.

Working Example 55

| | |
|---|---|
| (1) Compound 12 | 10.0 mg |
| (2) lactose | 70.0 mg |
| (3) magnesium stearate | 3.0 mg |

The above three components were sufficiently blended and filled in a capsule (size No.3).

Working Example 56

| | |
|---|---|
| (1) Compound 12 | 7.2 mg |
| (2) 1N HCl | 0.02 ml |
| (3) mannitol | 90.0 mg |
| (4) distilled water to make the whole volume 3 ml | |

Compound 12 (7.2 mg), 1N HCl (0.02 ml) and mannitol (90.0 mg) were dissolved in distilled water to make the whole volume 3.0 ml. The solution was subjected to filtration with a millipore filter. The filtrate was filled, under sterile conditions, in an ampoule of 3ml capacity. The ampoule was sterilized and sealed to give an injection preparation.

Working Example 57

| | |
|---|---|
| (1) Compound 17 | 10.0 mg |
| (2) lactose | 60.0 mg |
| (3) corn starch | 35.0 mg |
| (4) gelatin | 3.0 mg |
| (5) magnesium stearate | 2.0 mg |

A mixture of Compound 17 (10.0 mg), lactose (60.0 mg) and corn starch (35.0 mg) was granulated through a 1mm-screen, using a 10% aqueous solution of gelatin (0.04 ml: 3.0 mg in terms of gelatin). The granule was dried at 40 °C and screened again. The granule thus obtained was blended with magnesium stearate (2.0 mg), and the mixture was compressed to form a core tablet. The core tablet was sugar-coated with an aqueous solution of sucrose, titanium dioxide, talc and gum arabic. The coated tablet was polished with bees wax.

Working Example 58

| | |
|---|---|
| (1) Compound 39 | 10.0 mg |
| (2) lactose | 70.0 mg |
| (3) corn starch | 50.0 mg |
| (4) soluble starch | 7.0 mg |
| (5) magnesium stearate | 3.0 mg |

Compound 39 (10.0 mg) and lactose (70.0 mg) was granulated using an aqueous solution of soluble starch (0.07 ml:7.0 mg in terms of soluble starch. The granule was dried and blended with magnesium stearate (3.0 mg) and corn starch (50.0 mg). The mixture was compressed into a tablet.

Working Example 59

| | |
|---|---|
| (1) Compound 18 | 20.0 mg |
| (2) lactose | 79.0 mg |
| (3) magnesium stearate | 3.0 mg |

The above three components were blended sufficiently, and filled into a capsule of size No.3.

Working Example 60

| | |
|---|---|
| (1) Compound 20 | 20.0 mg |
| (2) lactose | 60.0 mg |
| (3) corn starch | 35.0 mg |
| (4) hydroxypropylcellulose | 3.0 mg |
| (5) magnesium stearate | 2.0 mg |

A mixture of Compound 20 (20.0 mg), lactose (60.0 mg) and corn starch (35.0 mg) was granulated through a 1-mesh screen, using a 10% aqueous solution of hydroxypropylcellulose (0.03 ml:3.0 mg in terms of hydroxypropylcellulose). The granule was dried and screened again. The granule thus obtained was blended with magnesium stearate (2.0 mg), and the mixture was compressed to form a core tablet. The core tablet was subjected to sugar-coating with an aqueous solution of sugar, titanium dioxide, talc and gum arabic. The coated tablet was polished with bees wax.

Working Example 61

| | |
|---|---|
| (1) Compound 42 | 10.0 mg |
| (2) lactose | 70.0 mg |
| (3) corn starch | 50.0 mg |
| (4) soluble starch | 7.0 mg |
| (5) magnesium stearate | 3.0 mg |

A mixture of Compound 42 (10.0 mg) and lactose was granulated using an aqueous solution of soluble starch (0.07 ml:7.0 mg in terms of soluble starch). The granule was dried and blended with magnesium stearate (3.0 mg) and corn starch (50.0 mg). The mixture was compressed into a tablet.

Working Example 62

| | |
|---|---|
| (1) Compound 27 | 10.0 mg |
| (2) lactose | 70.0 mg |
| (3) magnesium stearate | 3.0 mg |

The above three ingredients were blended sufficiently and filled into a capsule of size No. 3.

Working Example 63

| | |
|---|---|
| (1) Compound 31 | 10.0 mg |
| (2) lactose | 60.0 mg |
| (3) corn starch | 35.0 mg |
| (4) hydroxypropylcellulose | 3.0 mg |
| (5) magnesium stearate | 2.0 mg |

A mixture of Compound 31 (10.0 mg), lactose (60.0 mg) and corn starch (35.0 mg) was granulated through a 1-mesh screen, using a 10% aqueous solution of hydroxypropylcellulose (0.03 ml:3.0 mg in terms of hydroxypropylcellulose). The granule was dried at 40 °C and screened again. The granule thus obtained was blended with magnesium stearate (2.0 mg) and compressed to form a core core tablet. The core tablet was sugar-coated with an aqueous solution of sugar, titanium dioxide, talc and gum arabic. The coated tablet was polished with bees wax.

Working Example 64

| | |
|---|---|
| (1) Compound 48 | 10.0 mg |
| (2) lactose | 70.0 mg |
| (3) corn starch | 50.0 mg |
| (4) soluble starch | 7.0 mg |
| (5) magnesium stearate | 3.0 mg |

A mixture of Compound 48 (10.0 mg) and lactose (70.0 mg) was granulated using an aqueous solution of soluble starch (0.07 ml:7.0 mg in terms of soluble starch). The granule was dried, which was blended with magnesium stearate (3.0 mg) and corn starch (50.0 mg). The mixture was compressed into a tablet.

Test Example 1

Action of suppressing proliferation of pancreatic carcinoma and prostatic cancer cells in vitro

(Method)

A cell suspension of pancreatic cancer cell AsPC-1 (20000/ml) in RPMI 1640 medium (Flow Laboratories) supplemented with 10% of inactivated fetal calf serum, 1 mM of sodium pyruvate and 25 mM of HEPES [2-{4-(2-hydroxyethyl)-1-piperazinyl}ethanesulfonic acid] was added to 96-well micro-culture plates (50ul/well). The culture plates were incubated at 37 °C in a 5% $CO_2$ incubator. On the following day, the solution of each test compound dissolved in dimethylformamide and diluted (3-fold steps) was added (50 ul/well), which was incubated for further 3 days. The culture broth containing the test compounds was eliminated, and the cells were washed, to which was added a solution of MTT pigment, and the number of living cells was calculated [Tada et al. J. Immunol. Methods, 93 p.157 (1986)]. The number of cells in the control group containing no test compound was assumed to be 100%, and the respective ratios of cell numbers in groups containing the respective test compounds were calculated to thereby determine the concentrations ($IC_{50}$ value) of the respective test compounds necessary to suppress the number of of living cells to 50% of that of the control group.

For the test of pancreatic cancer cell strain PC-3, the same method in the case of AcPC-1 was employed excepting the use of F-12K medium containing 10% inactivated fetal calf serum (Flow Laboratories) and change of the incubation period after adding the test compounds to 4 days, to thereby determine the $IC_{50}$ value (nm).

(Results)

The results are shown in Table 2. All the test compounds showed remarkable actions of suppressing the proliferation of pancreatic cancer cells and of prostatic cancer cells. Thus, it is clear that the antitumor

agent of this invention has excellent activity to suppress the proliferation of tumor cells.

[Table 2]

| Compound No. | $Ic_{50}$ (nM) | |
|---|---|---|
| | AsPC-1 | PC-3 |
| 1 | 16 | <1.52 |
| 2 | 440 | 120 |
| 3 | <1.52 | 2 |
| 4 | 15 | <1.52 |
| 6 | 290 | 53 |
| 12 | 74 | 42 |
| 13 | <1.52 | <1.52 |
| 17 | 59 | 56 |
| 18 | <1.52 | <1.52 |
| 19 | 180 | <1.52 |
| 20 | 440 | 5 |
| 27 | 9 | <1.52 |
| 31 | 84 | 25 |
| 39 | <1.52 | 4 |
| 42 | 5 | 2 |
| 48 | 67 | 45 |
| 49 | 120 | <1.52 |
| 50 | 11 | <1.52 |
| 72 | <1.52 | <1.52 |
| 73 | 8 | <1.52 |
| 74 | 29 | <1.52 |
| 82 | 480 | 27 |
| 83 | 73 | 2 |
| 85 | 12 | <1.52 |
| 90 | 270 | 64 |

| Compound No. | $Ic_{50}$ (nM) | |
|---|---|---|
| | AsPC-1 | PC-3 |
| 91 | 560 | 92 |
| 92 | 28 | <1.52 |
| 93 | 770 | 110 |
| 96 | 16 | <1.52 |
| 97 | 280 | <1.52 |
| 98 | 54 | 6 |
| 99 | 230 | 52 |
| 102 | 33 | 2 |
| 103 | 250 | 210 |
| 104 | 85 | <1.52 |
| 111 | 550 | 3 |
| 113 | 120 | <1.52 |
| 115 | 49 | <1.52 |
| 116 | 370 | 14 |
| 117 | 100 | 2 |
| 119 | 370 | <1.52 |

Test Example 2

Action of suppressing proliferation of pancreatic carcinoma in vivo

(Method)

The human pancreatic carcinoma cells AsPC-1 ($3x10^6$ cells) were transplanted subcutaneously into Balb/c nude mice (7 week old) at the flank. On 35th day after the transplantation, the tumor size was measured. Five mice (per group) with almost the same tumor size were employed for the experiment. These groups were respectively administered intraperitoneally with No. 1, 2 and 19 of the compounds of this invention dissolved in corn oil, once for two days during 14 days. On the day following the completion of administration, the tumor size was measured, and the tumor volume was calculated by the formula: Tumor volume = major axis x minor axis x minor axis x (1/2). The ratio of the tumor volume in the treated group to that in a control group to which corn oil alone was administered was calculated as a proliferation ratio.

(Results)

The results are shown in [Table 3]. All the test compounds suppressed the growth of human pancreatic cancer cell strain AsPC-1 transplanted into nude mice.

[Table 3]

| Compound No. | Dosage (mg/kg) | Proliferation ratio (%) |
|---|---|---|
| Control | - | 100 |
| 1 | 6.7 | 56 |
| 2 | 16.7 | 48 |
| 19 | 16.7 | 29 |

Since the antitumor agent of this invention exhibits activity for suppressing the proliferation of tumor cells, it is useful for the therapy of intractable solid tumors including, for example, pancreatic carcinoma and prostatic cancer.

## Claims

1. Use of a 4,5-disubstituted 3(2H)-pyridazinone, the substituent at the 5-position being -X-$Y^0$-$Q^0$ wherein X stands for O or S, $Y^0$ stands for an optionally substituted divalent aliphatic hydrocarbon residue and $Q^0$ stands for an optionally substituted cyclic group, or its pharmaceutically acceptable salt thereof, as a component in the preparation of an antitumor composition.

2. Use as claimed in claim 1, wherein the substituent at the 4-position of the 3(2H)-pyridazinone stands for a group having 1 to 150 atoms.

3. Use as claimed in claim 1, wherein the 3(2H)-pyridazinone may be substituted with an optionally substituted hydrocarbon residue at the 2-position.

4. Use as claimed in claim 1, wherein the divalent aliphatic hydrocarbon residue has 1 to 3 carbon atoms.

5. Use as claimed in claim 4, wherein the substituent of the divalent aliphatic hydrocarbon residue is hydroxyl group.

6. Use as claimed in claim 1, wherein the cyclic group stands for an optionally substitutted aromatic cyclic group.

7. Use as claimed in claim 1, wherein the cyclic group Q stands for an optionally substituted phenyl group.

8. Use as claimed in claim 7, wherein the phenyl group stands for

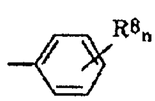

wherein $R^8$ stands for a halogen, a lower alkyl, a lower cycloalkyl, a lower alkoxy, a halo-lower alkyl, a halo-lower alkoxy, a cyano, a trimethylsilyl, a nitro,

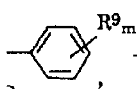 , 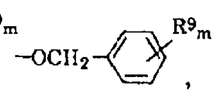 , 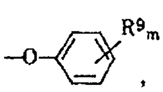 , 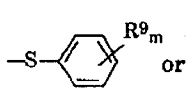 or 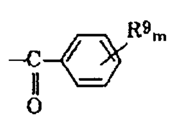

wherein $R^9$ stands for a halogen, a lower alkyl, a lower cycloalkyl, a lower alkoxy, a halo-lower alkyl, a halo-lower alkoxy, a cyano or a nitro group, m denotes an integer 0 to 5 and $R^9$ may be different in each of the m repeating units, n denotes an integer of 0 to 5 and $R^8$ may be different in each of the n repeating units.

9. Use as claimed in claim 8, wherein $R^8$ stands for

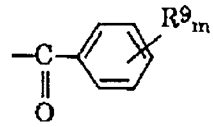

wherein $R^9$ stands for a halogen, a lower alkyl, a lower cycloalkyl, a lower alkoxy, a halo-lower alkyl, a halo-lower alkoxy, a cyano or a nitro group, m denotes an integer 0 to 5 and $R^9$ may be different in each of the m repeating units.

**10.** Use as claimed in claim 9, wherein $R^9$ stands for a halogen atom.

**11.** Use as claimed in claim 1, wherein the 3(2H)-pyridazinone stands for a compound of the formula:

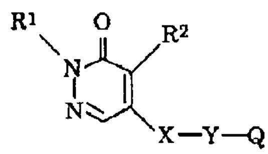

(I)

wherein $R^1$ stands for a lower alkyl group, cycloalkyl group or an optionally substituted phenyl group, $R^2$ stands for a halogen, an optionally substituted lower alkoxy group, an optionally substituted lower alkylthio group or an optionally substituted aliphatic hydrocarbon group, X stands for O or S, Y stands for an optionally substituted alkylene group or an optionally substituted alkenylene group and Q stands for an optionally substituted aryl group or an optionally substituted aromatic heterocyclic group, or its salt.

**12.** Use as claimed in claim 11, wherein $R^1$ stands for a branched lower alkyl group.

**13.** Use as claimed in claim 11, wherein $R^2$ stands for a halogen, a lower alkoxy group or a lower alkyl group.

**14.** Use as claimed in claim 11, wherein Y stands for

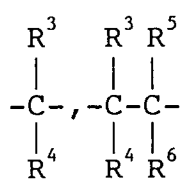

or

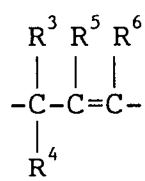

wherein $R^3$, $R^4$, $R^5$ and $R^6$ independently stand for a hydrogen, a hydroxy group or an optionally substituted a lower alkyl group.

**15.** A 4,5-disubstituted 3(2H)-pyridazinone, the substituents at the 5-position being

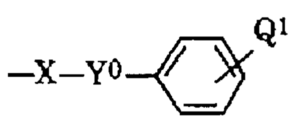

wherein X stands for O or S, Y stands for an optionally substituted divalent aliphatic hydrocarbon residue and $Q^1$ stands for an optionally substituted N-containing aromatic heterocyclic group, or its salt.

16. A 4,5-disubstituted 3(2H)-pyryridazinone, the substitutent at the 5-position being -X-$Y^1$-$Q^0$ wherein X stands for sO or S, $Y^1$ stands for a divalent aliphatic hydrocarbon residue substituted with a hydroxy group, and $Q^0$ stands for an optionally substituted cyclic group, or its salt.

17. A compound as claimed in claim 15, which is 2-tert-Butyl-4-chloro-5-[4-(1,2,4-thiadiazol-5-yl)benzylthio]-3-(2H)-pyridazinone.

18. A compound of the formula:

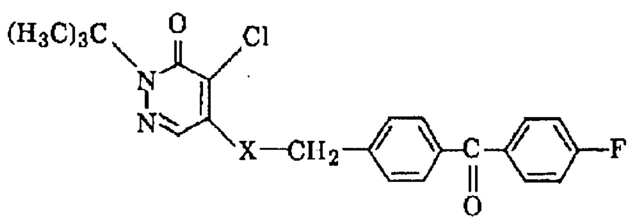

wherein X stands for O or S, or its salt.

19. A compound of the formula:

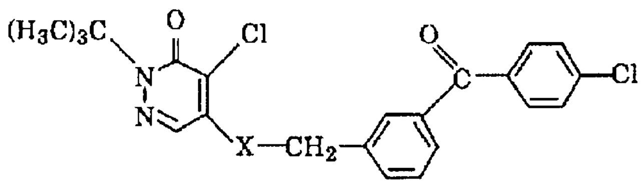

wherein X stands for O or S, or its salts.

20. A process for producing a compound as claimed in Claim 15, which comprises reacting a 4,5-disubstituted 3(2H)-pyridazinone, the substitutent at the 5-position being -X-H wherein X stands for O or S, with a compound of the formula:

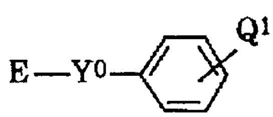

wherein E stands for a leaving group, $Y^0$ stands for an optionally substituted divalent aliphatic hydrocarbon residue and $Q^1$ stands for an optionally substituted N-containing aromatic heterocyclic group or its salt.

21. A process for producing a compound as claimed in Claim 16, which comprises reacting a 4,5-disubstituted 3(2H)-pyridazinone, the substituent at the 5-position being -X-H wherein X stands for O or S, with a compound of the formula: E-$Y^1$-$Q^0$
wherein E stands for a leaving group, $Y^1$ stands for a divalent aliphatic hydrocarbon residue substituted with a hydroxyl group and $Q^0$ stands for an optionally substituted cyclic group.

22. An antitumor composition which comprises a compound as claimed in claim 17, 18 or 19, together with a pharmaceutically acceptable carrier or diluent.

European Patent Office

# EUROPEAN SEARCH REPORT

Application Number
EP 95 10 1077

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X,Y | JOURNAL OF MEDICINAL CHEMISTRY, vol. 25,no. 7, 1982 pages 813-821, D.J. KATZ ET AL. 'Synthesis of Pyridazine Analogues of the Naturally Occurring Nucleosides Cytidine, Uridine, Deoxycytidine, and Deoxyuridine' * page 815, compound no. 13; page 817, compounds nos. 26 and 31; page 818, paragraph "Biology" * --- | 1 | C07D237/16<br>C07D237/18<br>A61K31/50 |
| Y | EP-A-0 521 381 (NISSAN CHEMICAL INDUSTRIES LTD. / ZERIA PHARMACEUTICAL CO., LTD.) * page 2, lines 1-14, 44-48 * | 1 | |
| D | & JP-A-05 017 357 --- | | |
| A,D | DATABASE WPI Week 7737 Derwent Publications Ltd., London, GB; AN 77-65668y & JP-A-52 091 883 (MORISHITA SEIYAKU K) , 2 August 1977 * abstract * --- | 1 | |
| | | | TECHNICAL FIELDS SEARCHED (Int.Cl.6) |
| | | | C07D<br>A61K |
| A | PATENT ABSTRACTS OF JAPAN vol. 5 no. 45 (C-48) [717] ,25 March 1981 & JP-A-55 167221 (SHIGEYUKI YASUDA) 26 December 1980, * abstract * --- | 1 | |
| X | EP-A-0 302 346 (NISSAN CHEMICAL INDUSTRIES LTD.) * pages 83-98, 105-109, 122-124 * --- | 15 | |
| | -/-- | | |

The present search report has been drawn up for all claims

| Place of search | Date of completion of the search | Examiner |
|---|---|---|
| BERLIN | 22 May 1995 | Hass, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 95 10 1077

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP-A-0 183 212 (NISSAN CHEMICAL INDUSTRIES LTD.)<br>* page 25, compound no. 106 * | 15 | |
| A | * page 21, compounds no. 67, 68 *<br>--- | 18,19 | |
| A | EP-A-0 193 853 (NISSAN CHEMICAL INDUSTRIES LTD.)<br>* examples 3, 11; table 8 *<br>--- | 15,16 | |
| A | EP-A-0 134 439 (NISSAN CHEMICAL INDUSTRIES LTD.)<br>* table 3 * | 15,16 | |
| D | & JP-A-60 004 173<br>--- | | |
| A | EP-A-0 232 825 (NISSAN CHEMICAL INDUSTRIES LTD.)<br>* page 27, compound no. 460; page 33, compound no. 640 *<br>--- | 18,19 | |
| A,D | PATENT ABSTRACTS OF JAPAN<br>vol. 9 no. 184 (C-294) ,30 July 1985<br>& JP-A-60 054370 (NISSAN KAGAKU KOGYO KK) 28 March 1985,<br>* abstract *<br>----- | 18,19 | TECHNICAL FIELDS SEARCHED (Int.Cl.6) |

The present search report has been drawn up for all claims

| Place of search | Date of completion of the search | Examiner |
|---|---|---|
| BERLIN | 22 May 1995 | Hass, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)